(19) Europäisches Patentamt
European Patent Office
Office européen des brevets

(11) EP 2 441 837 A1

(12) # EUROPEAN PATENT APPLICATION

(43) Date of publication:
**18.04.2012 Bulletin 2012/16**

(51) Int Cl.:
***C12N 15/113*** *(2010.01)*

(21) Application number: **11009592.4**

(22) Date of filing: **25.03.2008**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **26.03.2007 US 908098 P**

(62) Document number(s) of the earlier application(s) in accordance with Art. 76 EPC:
**08718165.7 / 2 137 313**

(71) Applicant: **Alnylam Pharmaceuticals Inc.**
**Cambridge MA 02142 (US)**

(72) Inventors:
- **Benson, John**
**West Roxbury, MA 02132 (US)**
- **Fitzgerald, Kevin**
**Brookline, MA 02446 (US)**
- **Bramlage, Birgit**
**95326 Kulmbach (DE)**
- **Tan, Pamela**
**95326 Kulmbach (DE)**
- **Vornlocher, Hans-Peter**
**95448 Bayreuth (DE)**

(74) Representative: **Vossius & Partner**
**Siebertstrasse 4**
**81675 München (DE)**

Remarks:
This application was filed on 05-12-2011 as a divisional application to the application mentioned under INID code 62.

(54) **dsRNA compositions and methods for treating HPV infections**

(57) This invention relates to a double-stranded ribonucleic acid (dsRNA), wherein said dsRNA comprises at least two sequences that are complementary to each other and wherein a sense strand comprises a first sequence and an antisense strand comprises a second sequence comprising a region of complementarity which is substantially complementary to at least a part of a mRNA encoding E6AP, and wherein said region of complementarity is less than 30 nucleotides in length and wherein said dsRNA, upon contact with a cell expressing said E6AP, inhibits expression of said E6AP gene by at least 40%.

EP 2 441 837 A1

## Description

### Field of the Invention

This invention relates to double-stranded ribonucleic acid (dsRNA), and its use in mediating RNA interference to treat pathological processes mediated by human papillomavirus (HPV) infection, such as cervical cancer, anal cancer, HPV associated precancerous lesions, and genital warts.

### Background of the Invention

Papillomaviruses (PV) are non-enveloped DNA viruses that induce hyperproliferative lesions of the epithelia. The papillomaviruses are widespread in nature and have been recognized in higher vertebrates. Viruses have been characterized, amongst others, from humans, cattle, rabbits, horses, and dogs. The first papillomavirus was described in 1933 as cottontail rabbit papillomavirus (CRPV). Since then, the cottontail rabbit as well as bovine papillomavirus type 1 (BPV-1) have served as experimental prototypes for studies on papillomaviruses. Most animal papillomaviruses are associated with purely epithelial proliferative lesions, and most lesions in animals are cutaneous. In the human more than 100 types of papillomavirus (HPV) have been identified and they have been catalogued by site of infection: cutaneous epithelium and mucosal epithelium (oral and genital mucosa). The cutaneous-related diseases include flat warts, plantar warts, etc. The mucosal-related diseases include laryngeal papillomas and anogenital diseases comprising cervical carcinomas (Fields, 1996, Virology, 3rd ed. Lippincott--Raven Pub., Philadelphia, N.Y.; Bernard, H-U-, 2005. J. Clin. Virol. 328: S1-S6).

Human papillomavirus (HPV) is one of the most prevalent sexually transmitted infections in the world. The majority of HPV infections are harmless. Some types of HPV cause genital warts, which appear as single or multiple bumps in the genital areas of men and women including the vagina, cervix, vulva (area outside of the vagina), penis, and rectum. Many people infected with HPV have no symptoms.

White most HPV subtypes result in benign lesions, certain subtypes are considered high-risk and can lead to more serious lesions, such as cervical and anal dysplasia. Fifteen HPV types were recently classified as high-risk types (Munoz, N. et al. 2003. N. Engl. J. Med. 348(6):518-27.) These high-risk subtypes are genetically diverse, demonstrating >10% sequence divergence at the L1 gene, a major virus capsid protein. (Bernard, H-U., 2005. J. Clin. Virol. 328: S1-S6).

Women having HPV infection are often asymptomatic and may only discover their lesion after cervical screening. Cervical screening is widely performed using the Pap test. A Pap test is a histological evaluation of cervical tissue which is used to identify abnormal cervical cells. As part of a Pap test, the presence of HPV infection and the specific subtype may be determined with the use of nucleic acid based assays such as PCR or the commercial Hybrid Capture II technique (HCII) (Digene, Gaithersburg, Maryland, U.S.A).

Abnormal cervical cells, if identified, are graded as LSIL (low-grade squamous intraepithelial lesions) having a low risk of progressing to cancer (including CIN-1 designated cells ("cervical intraepithelial neoplasia-1")); or HSIL (High-grade squamous intraepithelial lesions), including CIN-2 and CIN-3 designated cells, having a higher likelihood of progressing to cancer.

About 85% of low-grade lesions spontaneously regress, and the remainder either stay unchanged, or progress to high-grade lesions. About 10% of high-grade lesions, if left untreated, are expected to transform into cancerous tissues. HPV-16 and HPV-18 are most often associated with dysplasias, although several other transforming HPV subtypes are also associated with dysplasias.

Recent studies indicate that up to 89% of HTV positive homosexual males may be infected with these high-risk subtypes of HPV. HIV positive patients are also more likely to be infected with multiple subtypes of HPV at the same time, which is associated with a higher risk of dysplasia progression.

Evidence over the last two decades has led to a broad acceptance that HPV infection is necessary, though not sufficient, for the development of cervical cancer. The presence of HPV in cervical cancer is estimated at 99.7%. Anal cancer is thought to have a similar association between HPV infection and the development of anal dysplasia and anal cancer as is the case with cervical cancer. In one study of HIV negative patients with anal cancer, HPV infection was found in 88% of anal cancers. In the US in 2003, 12,200 new cases of cervical cancer and 4,100 cervical-cancer deaths were predicted along with 4,000 new cases of anal cancer and 500 anal-cancer deaths. While the incidence of cervical cancer has decreased in the last four decades due to widespread preventive screening, the incidence of anal cancer is increasing. The increase in anal cancer incidence may be attributed in part to HIV infection since HIV positive patients have a higher incidence of anal cancer than the general population. While anal cancer has an incidence of 0.9 cases per 100,000 in the general population, anal cancer has an incidence of 35 cases per 100,000 in the homosexual male population and 70-100 cases per 100,000 in the HIV positive homosexual male population. In fact, due to the high prevalence of anal dysplasia among HIV-infected patients and a growing trend of anal cancers, the 2003 USPHA/IDSA Guidelines for the Treatment of Opportunistic Infections in HIV Positive Patients will include treatment guidelines for

patients diagnosed with anal dysplasia.

There is no known cure for HPV infection. There are treatments for genital warts, although they often disappear even without treatment. The method of treatment depends on factors such as the size and location of the genital warts. Among the treatments used are Imiquimod cream, 20 percent podophyllin antimitotic solution, 0.5 percent podofilox solution, 5 percent 5-fluorouracil cream, and Trichloroacetic acid. The use of podophyllin or podofilox is not recommended for pregnant women because they are absorbed by the skin and may cause birth defects. The use of 5-fluorouracil cream is also not recommended for pregnant women. Small genital warts can be physically removed by freezing (cryosurgery), burning (electrocautery) or laser treatment. Large warts that do not respond to other treatment may have to be removed by surgery. Genital warts have been known to return following physical removal; in these instances α-interferon has been directly injected into these warts. However, α -interferon is expensive, and its use does not reduce the rate of return of the genital warts.

As such there exists an unmet need for effective HPV treatment. Surprisingly, compounds have been discovered that meet this need, and provide other benefits as well.

Recently, double-stranded RNA molecules (dsRNA) have been shown to block gene expression in a highly conserved regulatory mechanism known as RNA interference (RNAi). WO 99/32619 (Fire et al.) discloses the use of a dsRNA of at least 25 nucleotides in length to inhibit the expression of genes in C. *elegans.* dsRNA has also been shown to degrade target RNA in other organisms, including plants (see, e.g., WO 99/53050, Waterhouse et al.; and WO 99/61631, Heifetz et al.), *Drosophila* (see, e.g., Yang, D., et al., Curr. Biol. (2000) 10:1191-1200), and mammals (see WO 00/44895, Limmer; and DE 101 00 586.5, Kreutzer et al.). This natural mechanism has now become the focus for the development of a new class of pharmaceutical agents for treating disorders that are caused by the aberrant or unwanted regulation of a gene.

PCT Publication WO 03/008573 discloses a previous effort to develop a nucleic acid based medicament for the treatment of disease caused by HPV infection. This publication reports the use of two siRNAs directed to HPV mRNA to inhibit HPV replication in a cell based system; a related publication is found at Jiang, M. et al. 2005. N. A. R. 33(18): e151.

Despite significant advances in the field of RNAi and advances in the treatment of pathological processes mediated by HPV infection, there remains a need for agents that can inhibit the progression of HPV infection and that can treat diseases associated with HPV infection. The challenge is exacerbated because such agents must be designed to inhibit all the high-risk HPV subtypes, which together display a wide degree of genotypic diversity.

## Summary of the Invention

The invention provides a solution to the problem of treating diseases associated with HPV infection, by using double-stranded ribonucleic acid (dsRNA) to silence gene expression essential for HPV propagation. E6AP is a conserved gene of the human host species required by HPV for proliferation.

The invention provides double-stranded ribonucleic acid (dsRNA), as well as compositions and methods for inhibiting the expression of the E6AP gene in a cell or mammal using such dsRNA. The invention also provides compositions and methods for treating pathological conditions and diseases caused by the expression of the E6AP gene in connection with HPV infection, such as in cervical cancer and gential warts. The dsRNA of the invention comprises an RNA strand (the antisense strand) having a region which is less than 30 nucleotides in length, generally 19-24 nucleotides in length, and is substantially complementary to at least part of an mRNA transcript of the E6AP gene.

In one embodiment, the invention provides double-stranded ribonucleic acid (dsRNA) molecules for inhibiting the expression of the E6AP gene. The dsRNA comprises at least two sequences that are complementary to each other. The dsRNA comprises a sense strand comprising a first sequence and an antisense strand comprising a second sequence. The antisense strand comprises a nucleotide sequence which is substantially complementary to at least part of an mRNA encoding E6AP, and the region of complementarity is less than 30 nucleotides in length, generally 19-24 nucleotides in length. The dsRNA, upon contacting with a cell expressing the E6AP, inhibits the expression of the E6AP gene by at least 40%.

For example, the dsRNA molecules of the invention can be comprised of a first sequence of the dsRNA that is selected from the group consisting of the sense sequences of Table 1 and the second sequence is selected from the group consisting of the antisense sequences of Table 1. The dsRNA molecules of the invention can be comprised of naturally occurring nucleotides or can be comprised of at least one modified nucleotide, such as a 2'-O-methyl modified nucleotide, a nucleotide comprising a 5'-phosphorothioate group, and a terminal nucleotide linked to a cholesteryl derivative. Alternatively, the modified nucleotide may be chosen from the group of: a 2'-deoxy-2'-fluoro modified nucleotide, a 2'-deoxy-modified nucleotide, a locked nucleotide, an abasic nucleotide, 2'-amino-modified nucleotide, 2'-alkyl-modified nucleotide, morpholino nucleotide, a phosphoramidate, and a non-natural base comprising nucleotide. Generally, such modified sequence will be based on a first sequence of said dsRNA selected from the group consisting of the sense sequences of Table 1 and a second sequence selected from the group consisting of the antisense sequences of Table 1.

In another embodiment, the invention provides a cell comprising one of the dsRNAs of the invention. The cell

is generally a mammalian cell, such as a human cell.

In another embodiment, the invention provides a pharmaceutical composition for inhibiting the expression of the E6AP gene in an organism, generally a human subject, comprising one or more of the dsRNA of the invention and a pharmaceutically acceptable carrier or delivery vehicle.

In another embodiment, the invention provides a method for inhibiting the expression of the E6AP gene in a cell, comprising the following steps:

(a) introducing into the cell a double-stranded ribonucleic acid (dsRNA), wherein the dsRNA comprises at least two sequences that are complementary to each other. The dsRNA comprises a sense strand comprising a first sequence and an antisense strand comprising a second sequence. The antisense strand comprises a region of complementarity which is substantially complementary to at least a part of a mRNA encoding E6AP, and wherein the region of complementarity is less than 30 nucleotides in length, generally 19-24 nucleotides in length, and wherein the dsRNA, upon contact with a cell expressing the E6AP, inhibits expression of the E6AP gene by at least 40%; and

(b) maintaining the cell produced in step (a) for a time sufficient to obtain degradation of the mRNA transcript of the E6AP gene, thereby inhibiting expression of the E6AP gene in the cell.

In another embodiment, the invention provides methods for treating, preventing or managing pathological processes mediated by HPV infection, e.g. cancer or gential warts, comprising administering to a patient in need of such treatment, prevention or management a therapeutically or prophylactically effective amount of one or more of the dsRNAs of the invention.

In another embodiment, the invention provides vectors for inhibiting the expression of the E6AP gene in a cell, comprising a regulatory sequence operably linked to a nucleotide sequence that encodes at least one strand of one of the dsRNA of the invention.

In another embodiment, the invention provides a cell comprising a vector for inhibiting the expression of the E6AP gene in a cell. The vector comprises a regulatory sequence operably linked to a nucleotide sequence that encodes at least one strand of one of the dsRNA of the invention.

**Brief Description of the Figures**

No Figures are presented

**Detailed Description of the Intention**

The invention provides a solution to the problem of treating diseases associated with HPV infection, by using double-stranded ribonucleic acid (dsRNA) to silence expression of genes essential for HPV proliferation. In particular, the dsRNA of the invention silence the human E6AP gene, a conserved gene of the human host species required by HPV for proliferation. Herein, these genes are sometimes collectively called the HPV Target genes.

The invention provides double-stranded ribonucleic acid (dsRNA), as well as compositions and methods for inhibiting the expression of the E6AP gene in a cell or mammal using the dsRNA. The invention also provides compositions and methods for treating pathological conditions and diseases in a mammal caused by the expression of the E6AP gene in association with HPV infection using dsRNA. dsRNA directs the sequence-specific degradation of mRNA through a process known as RNA interference (RNAi).

The dsRNA of the invention comprises an RNA strand (the antisense strand) having a region which is less than 30 nucleotides in length, generally 19-24 nucleotides in length, and is substantially complementary to at least part of the HPV Target mRNA transcript. The use of these dsRNAs enables the targeted degradation of mRNAs of genes that are implicated in replication and/or maintenance of an HPV in mammals. Using cell-based and animal-based assays, the present inventors have demonstrated that very low dosages of these dsRNA can specifically and efficiently mediate RNAi, resulting in significant inhibition of expression of the E6AP gene. Thus, the methods and compositions of the invention comprising these dsRNAs are useful for treating pathological processes mediated by HPV infection by targeting a host factor gene involved in the HPV life cycle.

Description of the HPV Targets: HPV E1 and E6 and human E6AP

The cellular ubiquitin ligase E6AP of the human host is implicated in the replication of HPV, particularly integrated (non-episomal) forms of HPV, through its complex with the E6 protein of the virus. E6 binds to many proteins regulating cell proliferation pathways and often provokes their degradation (Chakrabarti, O. and Krishna, S. 2003. J. Biosci. 28: 337-348). E6 complexes with E6AP to target the tumor suppressor p53 for degradation (Scheffner, M. et al., 1990. Cell.

63:1129-1136; and Scheffner, M. et al., 1993. Cell 75:495-505). By inactivating p53, the virus not only prevents p53-mediated apoptosis of the infected cells (Chakrabarti and Krishna, 2003) and facilitates the replication of its DNA that would otherwise be blocked by p53 (Lepik, D. et al. 1998. J. Virol. 72:6822-6831), but it also favors oncogenesis by decreasing p53-mediated control on genomic integrity (Thomas, M. et al. 1999. Oncogene. 18:7690-7700).

E1 and E6 are both described in considerable detail in "Papillomaviridae: The Viruses and Their Replication" by Peter M. Howley, pp. 947-978, in: Fundamental Virology, 3rd ed. Bernard N. Fields, David M. Knipe, and Peter M. Howley, eds. Lippincott-Raven Publishers, Philadelphia, 1996. The E1 ORF encodes a 68-76 kD protein essential for plasmid DNA replication. The full-length E1 product is a phosphorylated nuclear protein that binds to the origin of replication in the LCR of BPV1. E1 has also been shown to bind ATP and to bind in vitro to the full length E2 protein called the E2 transcription transactivator (E2TA), thereby enhancing viral transcription. Binding to E2 also strengthens the affinity of E1 for the origin of DNA replication. In HPV-16, E1 has indirect effects on immortalization.

E6 is a small basic cell-transforming protein (e.g., the HPV 16 E6 comprises 151 amino acids), about 16-19 kD, which is localized to the nuclear matrix and non-nuclear membrane fraction. The E6 gene product contains four Cys-X-X-Cys motifs, indicating a potential for zinc binding; it may also act as a nucleic acid binding protein. In high-risk HPVs such as HPV-16, E6 and E7 proteins are necessary and sufficient to immortalize their hosts-squamous epithelial cells. The E6 gene products of high-risk HPVs have been shown to complex with p53, and to promote its degradation.

The following detailed description discloses how to make and use the dsRNA and compositions containing dsRNA to inhibit the expression of the HPV Target genes, as well as compositions and methods for treating diseases and disorders caused by HPV infection, e.g. cervical cancer and genital warts. The pharmaceutical compositions of the invention comprise a dsRNA having an antisense strand comprising a region of complementarity which is less than 30 nucleotides in length, generally 19-24 nucleotides in length, and is substantially complementary to at least part of an RNA transcript of an HPV Target gene, together with a pharmaceutically acceptable carrier. An embodiment of the invention is the employment of more than one dsRNA, optionally targeting different HPV Target genes, in combination in a pharmaceutical formulation.

Accordingly, certain aspects of the invention provide pharmaceutical compositions comprising the dsRNA of the invention together with a pharmaceutically acceptable carrier, methods of using the compositions to inhibit expression of one or more HPV Target genes, and methods of using the pharmaceutical compositions to treat diseases caused by HPV infection.

I. Definitions

For convenience, the meaning of certain terms and phrases used in the specification, examples, and appended claims, are provided below. If there is an apparent discrepancy between the usage of a term in other parts of this specification and its definition provided in this section, the definition in this section shall prevail.

"G," "C," "A" and "U" each generally stand for a nucleotide that contains guanine, cytosine, adenine, and uracil as a base, respectively. However, it will be understood that the term "ribonucleotide" or "nucleotide" can also refer to a modified nucleotide, as further detailed below, or a surrogate replacement moiety. The skilled person is well aware that guanine, cytosine, adenine, and uracil may be replaced by other moieties without substantially altering the base pairing properties of an oligonucleotide comprising a nucleotide bearing such replacement moiety. For example, without limitation, a nucleotide comprising inosine as its base may base pair with nucleotides containing adenine, cytosine, or uracil. Hence, nucleotides containing uracil, guanine, or adenine may be replaced in the nucleotide sequences of the invention by a nucleotide containing, for example, inosine. Sequences comprising such replacement moieties are embodiments of the invention.

As used herein, "E6AP" refers to the ubiquitin protein ligase E3A (ube3A, also referred to as E6-associated protein or E6AP) gene or protein. Human mRNA sequences to E6AP representing different isoforms are provided as GenBank Accession numbers NM_130838.1, NM_130839.1, and NM_000462.2.

As used herein, "E1" refers to the human papillomavirus type 16 (HPV16) E1 gene (GenBank accession number NC_001526, nucleotides 865 to 2813). As used herein, "E6" refers to the human papillomavirus type 16 (HPV 16) E6 gene (GenBank accession number NC_001526, nucleotides 65 to 559). Many variants of the E1 and E6 genes have also been publicly disclosed. These and future published E1 and E6 gene variants are intended to be covered herein by the use of "E1" and "E6", unless specifically excluded by the context.

As used herein, "target sequence" refers to a contiguous portion of the nucleotide sequence of an mRNA molecule formed during the transcription of one of the HPV Target genes, including mRNA that is a product of RNA processing of a primary transcription product.

As used herein, the term "strand comprising a sequence" refers to an oligonucleotide comprising a chain of nucleotides that is described by the sequence referred to using the standard nucleotide nomenclature.

As used herein, and unless otherwise indicated, the term "complementary," when used to describe a first nucleotide sequence in relation to a second nucleotide sequence, refers to the ability of an oligonucleotide or polynucleotide comprising the first nucleotide sequence to hybridize and form a duplex structure under certain conditions with an oligonucleotide or polynucleotide comprising the second nucleotide sequence, as will be understood by the skilled person. Such conditions can, for example, be stringent conditions, where stringent conditions may include: 400 mM NaCl, 40 mM PIPES pH 6.4, 1 mM EDTA, 50°C or 70°C for 12-16 hours followed by washing. Other conditions, such as physiologically relevant conditions as may be encountered inside an organism, can apply. The skilled person will be able to determine the set of conditions most appropriate for a test of complementarity of two sequences in accordance with the ultimate application of the hybridized nucleotides.

This includes base-pairing of the oligonucleotide or polynucleotide comprising the first nucleotide sequence to the oligonucleotide or polynucleotide comprising the second nucleotide sequence over the entire length of the first and second nucleotide sequence. Such sequences can be referred to as "fully complementary" with respect to each other herein. However, where a first sequence is referred to as "substantially complementary" with respect to a second sequence herein, the two sequences can be fully complementary, or they may form one or more, but generally not more than 4, 3 or 2 mismatched base pairs upon hybridization, while retaining the ability to hybridize under the conditions most relevant to their ultimate application. However, where two oligonucleotides are designed to form, upon hybridization, one or more single stranded overhangs, such overhangs shall not be regarded as mismatches with regard to the determination of complementarity. For example, a dsRNA comprising one oligonucleotide 21 nucleotides in length and another oligonucleotide 23 nucleotides in length, wherein the longer oligonucleotide comprises a sequence of 21 nucleotides that is fully complementary to the shorter oligonucleotide, may yet be referred to as "fully complementary" for the purposes of the invention.

"Complementary" sequences, as used herein, may also include, or be formed entirely from, non-Watson-Crick base pairs and/or base pairs formed from non-natural and modified nucleotides, in as far as the above requirements with respect to their ability to hybridize are fulfilled.

The terms "complementary", "fully complementary" and "substantially complementary" herein may be used with respect to the base matching between the sense strand and the antisense strand of a dsRNA, or between the antisense strand of a dsRNA and a target sequence, as will be understood from the context of their use.

As used herein, a polynucleotide which is "substantially complementary to at least part of" a messenger RNA (mRNA) refers to a polynucleotide which is substantially complementary to a contiguous portion of the mRNA of interest (e.g., encoding E6AP). For example, a polynucleotide is complementary to at least a part of a E6AP mRNA if the sequence is substantially complementary to a non-interrupted portion of a mRNA encoding E6AP.

The term "double-stranded RNA" or "dsRNA", as used herein, refers to a complex of ribonucleic acid molecules, having a duplex structure comprising two anti-parallel and substantially complementary, as defined above, nucleic acid strands. The two strands forming the duplex structure may be different portions of one larger RNA molecule, or they may be separate RNA molecules. Where separate RNA molecules, such dsRNA are often referred to in the literature as siRNA ("short interfering RNA"). Where the two strands are part of one larger molecule, and therefore are connected by an uninterrupted chain of nucleotides between the 3'-end of one strand and the 5'end of the respective other strand forming the duplex structure, the connecting RNA chain is referred to as a "hairpin loop", "short hairpin RNA" or "shRNA". Where the two strands are connected covalently by means other than an uninterrupted chain of nucleotides between the 3'-end of one strand and the 5'end of the respective other strand forming the duplex structure, the connecting structure is referred to as a "linker". The RNA strands may have the same or a different number of nucleotides. The maximum number of base pairs is the number of nucleotides in the shortest strand of the dsRNA minus any overhangs that are present in the duplex. In addition to the duplex structure, a dsRNA may comprise one or more nucleotide overhangs. In addition, as used in this specification, "dsRNA" may include chemical modifications to ribonucleotides, internucleoside linkages, end-groups, caps, and conjugated moieties, including substantial modifications at multiple nucleotides and including all types of modifications disclosed herein or known in the art. Any such modifications, as used in an siRNA type molecule, are encompassed by "dsRNA" for the purposes of this specification and claims.

As used herein, a "nucleotide overhang" refers to the unpaired nucleotide or nucleotides that protrude from the duplex structure of a dsRNA when a 3'-end of one strand of the dsRNA extends beyond the 5'-end of the other strand, or vice versa. "Blunt" or "blunt end" means that there are no unpaired nucleotides at that end of the dsRNA, i.e., no nucleotide overhang. A "blunt ended" dsRNA is a dsRNA that is double-stranded over its entire length, i.e., no nucleotide overhang at either end of the molecule. For clarity, chemical caps or non-nucleotide chemical moieties conjugated to the 3' end or 5' end of an siRNA are not considered in determining whether an siRNA has an overhang or is blunt ended.

The term "antisense strand" refers to the strand of a dsRNA which includes a region that is substantially complementary to a target sequence. As used herein, the term "region of complementarity" refers to the region on the antisense strand that is substantially complementary to a sequence, for example a target sequence, as defined herein. Where the region of complementarity is not fully complementary to the target sequence, the mismatches are most tolerated in the terminal regions and, if present, are generally in a terminal region or regions, e.g., within 6, 5, 4, 3, or 2 nucleotides of the 5' and/or 3' terminus.

The term "sense strand," as used herein, refers to the strand of a dsRNA that includes a region that is substantially

complementary to a region of the antisense strand.

"Introducing into a cell", when referring to a dsRNA, means facilitating uptake or absorption into the cell, as is understood by those skilled in the art. Absorption or uptake of dsRNA can occur through unaided diffusive or active cellular processes, or by auxiliary agents or devices. The meaning of this term is not limited to cells in vitro; a dsRNA may also be "introduced into a cell", wherein the cell is part of a living organism. In such instance, introduction into the cell will include the delivery to the organism. For example, for in vivo delivery, dsRNA can be injected into a tissue site or administered systemically. In vitro introduction into a cell includes methods known in the art such as electroporation and lipofection.

The terms "silence" and "inhibit the expression of', in as far as they refer to the HPV Target gene, herein refer to the at least partial suppression of the expression of the HPV Target gene, as manifested by a reduction of the amount of mRNA transcribed from the HPV Target gene which may be isolated from a first cell or group of cells in which the HPV Target gene is transcribed and which has or have been treated such that the expression of the HPV Target gene is inhibited, as compared to a second cell or group of cells substantially identical to the first cell or group of cells but which has or have not been so treated (control cells). The degree of inhibition is usually expressed in terms of

$$\frac{(\text{mRNA in control cells}) - (\text{mRNA in treated cells})}{(\text{mRNA in control cells})} \bullet 100\%$$

Alternatively, the degree of inhibition may be given in terms of a reduction of a parameter that is functionally linked to the HPV Target gene transcription, e.g. the amount of protein encoded by the HPV Target gene which is secreted by a cell, or the number of cells displaying a certain phenotype, e.g., apoptosis. In principle, HPV Target gene silencing may be determined in any cell expressing the target, either constitutively or by genomic engineering, and by any appropriate assay. However, when a reference is needed in order to determine whether a given dsRNA inhibits the expression of the HPV Target gene by a certain degree and therefore is encompassed by the instant invention, the assay provided in the Examples below shall serve as such reference.

For example, in certain instances, expression of the E6AP gene is suppressed by at least about 20%, 25%, 35%, or 50% by administration of the double-stranded oligonucleotide of the invention. In some embodiments, the E6AP gene is suppressed by at least about 60%, 70%, or 80% by administration of the double-stranded oligonucleotide of the invention. In some embodiments, the E6AP gene is suppressed by at least about 85%, 90%, or 95% by administration of the double-stranded oligonucleotide of the invention. Table 2 provides a wide range of values for inhibition of transcription obtained in an in vitro assay using various E6AP dsRNA molecules at various concentrations.

As used herein in the context of HPV infection, the terms "treat", "treatment", and the like, refer to relief from or alleviation of pathological processes mediated by HPV infection. Such description includes use of the therapeutic agents of the invention for prophylaxis or prevention of HPV infection, and relief from symptoms or pathologies caused by HPV infection. In the context of the present invention insofar as it relates to any of the other conditions recited herein below (other than pathological processes mediated by HPV infection), the terms "treat", "treatment", and the like mean to relieve or alleviate at least one symptom associated with such condition, or to slow or reverse the progression of such condition.

As used herein, the phrases "therapeutically effective amount" and "prophylactically effective amount" refer to an amount that provides a therapeutic benefit in the treatment, prevention, or management of pathological processes mediated by HPV infection or an overt symptom of pathological processes mediated by HPV infection. The specific amount that is therapeutically effective can be readily determined by ordinary medical practitioner, and may vary depending on factors known in the art, such as, e.g. the type of pathological processes mediated by HPV infection, the patient's history and age, the stage of pathological processes mediated by HPV infection, and the administration of other anti-pathological agents.

As used herein, a "pharmaceutical composition" comprises a pharmacologically effective amount of a dsRNA and a pharmaceutically acceptable carrier. As used herein, "pharmacologically effective amount," "therapeutically effective amount" or simply "effective amount" refers to that amount of a dsRNA effective to produce the intended pharmacological, therapeutic or preventive result. For example, if a given clinical treatment is considered effective when there is at least a 25% reduction in a measurable parameter associated with a disease or disorder, a therapeutically effective amount of a drug for the treatment of that disease or disorder is the amount necessary to effect at least a 25% reduction in that parameter.

The term "pharmaceutically acceptable carrier" refers to a carrier for administration of a therapeutic agent. Such carriers include, but are not limited to, saline, buffered saline, dextrose, water, glycerol, ethanol, and combinations thereof. The term specifically excludes cell culture medium. For drugs administered orally, pharmaceutically acceptable carriers include, but are not limited to pharmaceutically acceptable excipients such as inert diluents, disintegrating agents,

binding agents, lubricating agents, sweetening agents, flavoring agents, coloring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

As used herein, a "transformed cell" is a cell into which a vector has been introduced from which a dsRNA molecule may be expressed.

II. Double-stranded ribonucleic acid (dsRNA)

In one embodiment, the invention provides double-stranded ribonucleic acid (dsRNA) molecules for inhibiting the expression of the HPV Target gene in a cell or mammal, wherein the dsRNA comprises an antisense strand comprising a region of complementarity which is complementary to at least a part of an mRNA formed in the expression of the HPV Target gene, and wherein the region of complementarity is less than 30 nucleotides in length, generally 19-24 nucleotides in length, and wherein said dsRNA, upon contact with a cell expressing said HPV Target gene, inhibits the expression of said HPV Target gene by at least 10%, 25%, or 40%.

The dsRNA comprises two RNA strands that are sufficiently complementary to hybridize to form a duplex structure. One strand of the dsRNA (the antisense strand) comprises a region of complementarity that is substantially complementary, and generally fully complementary, to a target sequence, derived from the sequence of an mRNA formed during the expression of the HPV Target gene, the other strand (the sense strand) comprises a region which is complementary to the antisense strand, such that the two strands hybridize and form a duplex structure when combined under suitable conditions. Generally, the duplex structure is between 15 and 30, more generally between 18 and 25, yet more generally between 19 and 24, and most generally between 19 and 21 base pairs in length. Similarly, the region of complementarity to the target sequence is between 15 and 30, more generally between 18 and 25, yet more generally between 19 and 24, and most generally between 19 and 21 nucleotides in length. The dsRNA of the invention may further comprise one or more single-stranded nucleotide overhang(s). The dsRNA can be synthesized by standard methods known in the art as further discussed below, e.g., by use of an automated DNA synthesizer, such as are commercially available from, for example, Biosearch, Applied Biosystems, Inc. In a preferred embodiment, the HPV Target gene is the human E6AP gene. In specific embodiments, the antisense strand of the dsRNA comprises a strand selected from the sense sequences of Table I and a second sequence selected from the group consisting of the antisense sequences of Table 1. Alternative antisense agents that target elsewhere in the target sequence provided in Table 1 can readily be determined using the target sequence and the flanking E6AP sequence.

In further embodiments, the dsRNA comprises at least one nucleotide sequence selected from the groups of sequences provided in Table 1. In other embodiments, the dsRNA comprises at least two sequences selected from this group, wherein one of the at least two sequences is complementary to another of the at least two sequences, and one of the at least two sequences is substantially complementary to a sequence of an mRNA generated in the expression of the E6AP gene. Generally, the dsRNA comprises two oligonucleotides, wherein one oligonucleotide is described as the sense strand in Table 1 and the second oligonucleotide is described as the antisense strand in Table 1. Table 1 provides a duplex name and sequence ID number for each preferred dsRNA.

The skilled person is well aware that dsRNAs comprising a duplex structure of between 20 and 23, but specifically 21, base pairs have been hailed as particularly effective in inducing DNA interference (Elbashir et al., EMBO 2001, 20: 6877-6888). However, others have found that shorter or longer dsRNAs can be effective as well. In the embodiments described above, by virtue of the nature of the oligonucleotide sequences provided in Table 1 the dsRNAs of the invention can comprise at least one strand of a length of minimally 21 nt. It can be reasonably expected that shorter dsRNAs comprising one of the sequences of Table 1, minus only a few nucleotides on one or both ends may be similarly effective as compared to the dsRNAs described above. Hence, dsRNAs comprising a partial sequence of at least 15, 16, 17, 18, 19, 20, or more contiguous nucleotides from one of the sequences of Table 1, and differing in their ability to inhibit the expression of the HPV Target gene in a FACS assay or other assay as described herein below by not more than 5, 10, 15, 20, 25, or 30 % inhibition from a dsRNA comprising the full sequence, are contemplated by the invention. Further dsRNAs that cleave within the target sequence provided in Table 1 can readily be made using the reference sequence and the target sequence provided.

In addition, the RNAi agents provided in Table 1 identify a site in the respective HPV Target mRNA that is susceptible to RNAi based cleavage. As such the present invention further includes RNAi agents that target within the sequence targeted by one of the agents of the present invention. As used herein a second RNAi agent is said to target within the sequence of a first RNAi agent if the second RNAi agent cleaves the message anywhere within the mRNA that is complementary to the antisense strand of the first RNAi agent. Such a second agent will generally consist of at least 15 contiguous nucleotides from one of the sequences provided in Table 1 coupled to additional nucleotide sequences taken from the region contiguous to the selected sequence in the HPV Target gene. For example, the last 15 nucleotides

of SEQ ID NO:1 I (minus the added AA sequences) combined with the next 6 nucleotides from the target E6AP gene produces a single strand agent of 21 nucleotides that is based on one of the sequences provided in Table 1.

The dsRNA of the invention can contain one or more mismatches to the target sequence. In a preferred embodiment, the dsRNA of the invention contains no more than 3 mismatches. If the antisense strand of the dsRNA contains mismatches to a target sequence, it is preferable that the area of mismatch not be located in the center of the region of complementarity. If the antisense strand of the dsRNA contains mismatches to the target sequence, it is preferable that the mismatch be restricted to 5 nucleotides from either end, for example 5, 4, 3, 2, or 1 nucleotide from either the 5' or 3' end of the region of complementarity. For example, for a 23 nucleotide dsRNA strand which is complementary to a region of the HPV Target gene, the dsRNA generally does not contain any mismatch within the central 13 nucleotides. The methods described within the invention can be used to determine whether a dsRNA containing a mismatch to a target sequence is effective in inhibiting the expression of the HPV Target gene. Consideration of the efficacy of dsRNAs with mismatches in inhibiting expression of the HPV Target gene is important, especially if the particular region of complementarity in the HPV Target gene is known to have polymorphic sequence variation in the virus (if E1 or E6) or within the human population (for E6AP).

In one embodiment, at least one end of the dsRNA has a single-stranded nucleotide overhang of 1 to 4, generally 1 or 2 nucleotides. dsRNAs having at least one nucleotide overhang have unexpectedly superior inhibitory properties than their blunt-ended counterparts. Moreover, the present inventors have discovered that the presence of only one nucleotide overhang strengthens the interference activity of the dsRNA, without affecting its overall stability. dsRNA having only one overhang has proven particularly stable and effective in vivo, as well as in a variety of cells, cell culture mediums, blood, and serum. Generally, the single-stranded overhang is located at the 3'-terminal end of the antisense strand or, alternatively, at the 3'-terminal end of the sense strand. The dsRNA may also have a blunt end, generally located at the 5'-end of the antisense strand. Such dsRNAs have improved stability and inhibitory activity, thus allowing administration at low dosages, i.e., less than 5 mg/kg body weight of the recipient per day. Generally, the antisense strand of the dsRNA has a nucleotide overhang at the 3'-end, and the 5'-end is blunt. In another embodiment, one or more of the nucleotides in the overhang is replaced with a nucleoside thiophosphate.

In yet another embodiment, the dsRNA is chemically modified to enhance stability. The nucleic acids of the invention may be synthesized and/or modified by methods well established in the art, such as those described in "Current protocols in nucleic acid chemistry", Beaucage, S.L. et al. (Edrs.), John Wiley & Sons, Inc., New York, NY, USA, which is hereby incorporated herein by reference. Chemical modifications may include, but are not limited to 2' modifications, modifications at other sites of the sugar or base of an oligonucleotide, introduction of non-natural bases into the oligonucleotide chain, covalent attachment to a ligand or chemical moiety, and replacement of internucleotide phosphate linkages with alternate linkages such as thiophosphates. More than one such modification may be employed.

Chemical linking of the two separate dsRNA strands may be achieved by any of a variety of well-known techniques, for example by introducing covalent, ionic or hydrogen bonds; hydrophobic interactions, van der Waals or stacking interactions; by means of metal-ion coordination, or through use of purine analogues. Generally, the chemical groups that can be used to modify the dsRNA include, without limitation, methylene blue; bifunctional groups, generally bis-(2-chloroethyl)amine; N-acetyl-N'-(p-glyoxylbenzoyl)cystamine; 4-thiouracil; and psoralen. In one embodiment, the linker is a hexa-ethylene glycol linker. In this case, the dsRNA are produced by solid phase synthesis and the hexa-ethylene glycol linker is incorporated according to standard methods (e.g., Williams, D.J., and K.B. Hall, Biochem. (1996) 35:14665-14670). In a particular embodiment, the 5'-end of the antisense strand and the 3'-end of the sense strand are chemically linked via a hexaethylene glycol linker. In another embodiment, at least one nucleotide of the dsRNA comprises a phosphorothioate or phosphorodithioate groups. The chemical bond at the ends of the dsRNA is generally formed by triple-helix bonds. Table 1 provides examples of modified RNAi agents of the invention.

In yet another embodiment, the nucleotides at one or both of the two single strands may be modified to prevent or inhibit the degradation activities of cellular enzymes, such as, for example, without limitation, certain nucleases. Techniques for inhibiting the degradation activity of cellular enzymes against nucleic acids are known in the art including, but not limited to, 2'-amino modifications, 2'-amino sugar modifications, 2'-F sugar modifications, 2'-F modifications, 2'-alkyl sugar modifications, uncharged backbone modifications, morpholino modifications, 2'-O-methyl modifications, and phosphoramidate (see, e.g., Wagner, Nat. Med. (1995) 1:1116-8). Thus, at least one 2'-hydroxyl group of the nucleotides on a dsRNA is replaced by a chemical group, generally by a 2'-amino or a 2'-methyl group. Also, at least one nucleotide may be modified to form a locked nucleotide. Such locked nucleotide contains a methylene bridge that connects the 2'-oxygen of ribose with the 4'-carbon of ribose. Oligonucleotides containing the locked nucleotide are described in Koshkin, A.A., et al., Tetrahedron (1998), 54: 3607-3630) and Obika, S. et al., Tetrahedron Lett. (1998), 39: 5401-5404). Introduction of a locked nucleotide into an oligonucleotide improves the affinity for complementary sequences and increases the melting temperature by several degrees (Braasch, D.A. and D.R Corey, Chem. Biol. (2001), 8:1-7).

Conjugating a ligand to a dsRNA can enhance its cellular absorption as well as targeting to a particular tissue or uptake by specific types of cells such as vaginal epithelium. In certain instances, a hydrophobic ligand is conjugated to the dsRNA to facilitate direct permeation of the cellular membrane. Alternatively, the ligand conjugated to the dsRNA

is a substrate for receptor-mediated endocytosis. These approaches have been used to facilitate cell permeation of antisense oligonucleotides as well as dsRNA agents. For example, cholesterol has been conjugated to various antisense oligonucleotides resulting in compounds that are substantially more active compared to their non-conjugated analogs. See M. Manoharan Antisense & Nucleic Acid Drug Development 2002, 12, 103. Other lipophilic compounds that have been conjugated to oligonucleotides include 1-pyrene butyric acid, 1,3-bis-O-(hexadecyl)glycerol, and menthol. One example of a ligand for receptor-mediated endocytosis is folic acid. Folic acid enters the cell by folate-receptor-mediated endocytosis. dsRNA compounds bearing folic acid would be efficiently transported into the cell via the folate-receptor-mediated endocytosis. Li and coworkers report that attachment of folic acid to the 3'-terminus of an oligonucleotide resulted in an 8-fold increase in cellular uptake of the oligonucleotide. Li, S.; Deshmukh, H. M.; Huang, L. Pharm. Res. 1998, 15, 1540. Other ligands that have been conjugated to oligonucleotides include polyethylene glycols, carbohydrate clusters, cross-linking agents, porphyrin conjugates, and delivery peptides.

In certain instances, conjugation of a cationic ligand to oligonucleotides results in improved resistance to nucleases. Representative examples of cationic ligands are propylammonium and dimethylpropylammonium. Interestingly, antisense oligonucleotides were reported to retain their high binding affinity to mRNA when the cationic ligand was dispersed throughout the oligonucleotide. See M. Manoharan Antisense & Nucleic Acid Drug Development 2002, 12, 103 and references therein.

The ligand-conjugated dsRNA of the invention may be synthesized by the use of a dsRNA that bears a pendant reactive functionality, such as that derived from the attachment of a linking molecule onto the dsRNA. This reactive oligonucleotide may be reacted directly with commercially-available ligands, ligands that are synthesized bearing any of a variety of protecting groups, or ligands that have a linking moiety attached thereto. The methods of the invention facilitate the synthesis of ligand-conjugated dsRNA by the use of, in some preferred embodiments, nucleoside monomers that have been appropriately conjugated with ligands and that may further be attached to a solid-support material. Such ligand-nucleoside conjugates, optionally attached to a solid-support material, are prepared according to some preferred embodiments of the methods of the invention via reaction of a selected serum-binding ligand with a linking moiety located on the 5' position of a nucleoside or oligonucleotide. In certain instances, an dsRNA bearing an aralkyl ligand attached to the 3'-terminus of the dsRNA is prepared by first covalently attaching a monomer building block to a controlled-pore-glass support via a long-chain aminoalkyl group. Then, nucleotides are bonded via standard solid-phase synthesis techniques to the monomer building-block bound to the solid support. The monomer building block may be a nucleoside or other organic compound that is compatible with solid-phase synthesis.

The dsRNA used in the conjugates of the invention may be conveniently and routinely made through the well-known technique of solid-phase synthesis. Equipment for such synthesis is sold by several vendors including, for example, Applied Biosystems (Foster City, CA). Any other means for such synthesis known in the art may additionally or alternatively be employed. It is also known to use similar techniques to prepare other oligonucleotides, such as the phosphorothioates and alkylated derivatives.

Teachings regarding the synthesis of particular modified oligonucleotides may be found in the following U.S. patents: U.S. Pat. Nos. 5,138,045 and 5,218,105, drawn to polyamine conjugated oligonucleotides; U.S. Pat. No. 5,212,295, drawn to monomers for the preparation of oligonucleotides having chiral phosphorus linkages; U.S. Pat. Nos. 5,378,825 and 5,541,307, drawn to oligonucleotides having modified backbones; U.S. Pat. No. 5,386,023, drawn to backbone-modified oligonucleotides and the preparation thereof through reductive coupling; U.S. Pat. No. 5,457,191, drawn to modified nucleobases based on the 3-deazapurine ring system and methods of synthesis thereof; U.S. Pat. No. 5,459,255, drawn to modified nucleobases based on N-2 substituted purines; U.S. Pat. No. 5,521,302, drawn to processes for preparing oligonucleotides having chiral phosphorus linkages; U.S. Pat. No. 5,539,082, drawn to peptide nucleic acids; U.S. Pat. No. 5,554,746, drawn to oligonucleotides having β-lactam backbones; U.S. Pat. No. 5,571,902, drawn to methods and materials for the synthesis of oligonucleotides; U.S. Pat. No. 5,578,718, drawn to nucleosides having alkylthio groups, wherein such groups may be used as linkers to other moieties attached at any of a variety of positions of the nucleoside; U.S. Pat. Nos. 5,587,361 and 5,599,797, drawn to oligonucleotides having phosphorothioate linkages of high chiral purity; U.S. Pat. No. 5,506,351, drawn to processes for the preparation of 2'-O-alkyl guanosine and related compounds, including 2,6-diaminopurine compounds; U.S. Pat. No. 5,587,469, drawn to oligonucleotides having N-2 substituted purines; U.S. Pat. No. 5,587,470, drawn to oligonucleotides having 3-deazapurines; U.S. Pat. No. 5,223,168, and U.S. Pat. No. 5,608,046, both drawn to conjugated 4'-desmethyl nucleoside analogs; U.S. Pat. Nos. 5,602,240, and 5,610,289, drawn to backbone-modified oligonucleotide analogs; U.S. Pat. Nos. 6,262,241, and 5,459,255, drawn to, inter alia, methods of synthesizing 2'-fluoro-oligonucleotides.

In the ligand-conjugated dsRNA and ligand-molecule bearing sequence-specific linked nucleosides of the invention, the oligonucleotides and oligonucleosides may be assembled on a suitable DNA synthesizer utilizing standard nucleotide or nucleoside precursors, or nucleotide or nucleoside conjugate precursors that already bear the linking moiety, ligand-nucleotide or nucleoside-conjugate precursors that already bear the ligand molecule, or non-nucleoside ligand-bearing building blocks.

When using nucleotide-conjugate precursors that already bear a linking moiety, the synthesis of the sequence-specific linked nucleosides is typically completed, and the ligand molecule is then reacted with the linking moiety to form the ligand-conjugated oligonucleotide. Oligonucleotide conjugates bearing a variety of molecules such as steroids, vitamins, lipids and reporter molecules, has previously been described (see Manoharan et al., PCT Application WO 93/07883). In a preferred embodiment, the oligonucleotides or linked nucleosides of the invention are synthesized by an automated synthesizer using phosphoramidites derived from ligand-nucleoside conjugates in addition to the standard phosphoramidites and non-standard phosphoramidites that are commercially available and routinely used in oligonucleotide synthesis.

The incorporation of a 2'-O-methyl, 2'-O-ethyl, 2'-O-propyl, 2'-O-allyl, 2'-O-aminoalkyl or 2'-deoxy-2'-fluoro group in nucleosides of an oligonucleotide confers enhanced hybridization properties to the oligonucleotide. Further, oligonucleotides containing phosphorothioate backbones have enhanced nuclease stability. Thus, functionalized, linked nucleosides of the invention can be augmented to include either or both a phosphorothioate backbone or a 2'-O-methyl, 2'-O-ethyl, 2'-O-propyl, 2'-O-aminoalkyl, 2'-O-allyl or 2'-deoxy-2'-fluoro group. A summary listing of some of the oligonucleotide modifications known in the art is found at, for example, PCT Publication WO 200370918.

In some embodiments, functionalized nucleoside sequences of the invention possessing an amino group at the 5'-terminus are prepared using a DNA synthesizer, and then reacted with an active ester derivative of a selected ligand. Active ester derivatives are well known to those skilled in the art. Representative active esters include N-hydrosuccinimide esters, tetrafluorophenolic esters, pentafluorophenolic esters and pentachlorophenolic esters. The reaction of the amino group and the active ester produces an oligonucleotide in which the selected ligand is attached to the 5'-position through a linking group. The amino group at the 5'-terminus can be prepared utilizing a 5'-Amino-Modifier C6 reagent. In one embodiment, ligand molecules may be conjugated to oligonucleotides at the 5'-position by the use of a ligand-nucleoside phosphoramidite wherein the ligand is linked to the 5'-hydroxy group directly or indirectly via a linker. Such ligand-nucleoside phosphoramidites are typically used at the end of an automated synthesis procedure to provide a ligand-conjugated oligonucleotide bearing the ligand at the 5'-terminus.

Examples of modified internucleoside linkages or backbones include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Various salts, mixed salts and free-acid forms are also included.

Representative United States Patents relating to the preparation of the above phosphorus-atom-containing linkages include, but are not limited to, U.S. Pat. Nos. 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; 5,625,050; and 5,697,248, each of which is herein incorporated by reference.

Examples of modified internucleoside linkages or backbones that do not include a phosphorus atom therein (i.e., oligonucleosides) have backbones that are formed by short chain alkyl or cycloalkyl intersugar linkages, mixed heteroatom and alkyl or cycloalkyl intersugar linkages, or one or more short chain heteroatomic or heterocyclic intersugar linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and $CH_2$ component parts.

Representative United States patents relating to the preparation of the above oligonucleosides include, but are not limited to, U.S. Pat. Nos. 5,034,506; 5,166,315; 5,185,444; 5,214,134; 5,216,141; 5,235,033; 5,264,562; 5,264,564; 5,405,938; 5,434,257; 5,466,677; 5,470;967; 5,489,677; 5,541,307; 5,561,225; 5,596,086; 5;602;240; 5,610,289; 5,602,240; 5,608,046; 5,610,289; 5,618,704; 5,623,070; 5,663,312; 5,633,360; 5,677,437; and 5,677,439, each of which is herein incorporated by reference.

In certain instances, the oligonucleotide may be modified by a non-ligand group. A number of non-ligand molecules have been conjugated to oligonucleotides in order to enhance the activity, cellular distribution or cellular uptake of the oligonucleotide, and procedures for performing such conjugations are available in the scientific literature. Such non-ligand moieties have included lipid moieties, such as cholesterol (Letsinger et al., Proc. Natl. Acad. Sci. USA, 1989, 86:6553), cholic acid (Manoharan et al., Bioorg. Med. Chem. Lett., 1994, 4:1053), a thioether, e.g., hexyl-S-tritylthiol (Manoharan et al., Ann. N.Y. Acad. Sci., 1992, 660:306; Manoharan et al., Bioorg. Med. Chem. Let., 1993, 3: 2765), a thiocholesterol (Oberhauser et al., Nucl. Acids Res., 1992, 20:533), an aliphatic chain, e.g., dodecandiol or undecyl residues (Saison-Behmoaras et al., EMBO J., 1991, 10:111; Kabanov et al., FEBS Lett., 1990, 259:327; Svinarchuk et al., Biochimie, 1993, 75:49), a phospholipid, e.g., di-hexadecyl-rac-glycerol or triethylammonium 1,2-di-O-hexadecyl-rac-glycero-3-H-phosphonate (Manoharan et al., Tetrahedron Lett., 1995, 36:3651; Shea et al., Nucl. Acids

Res., 1990, 18:3777), a polyamine or a polyethylene glycol chain (Manoharan et al., Nucleosides & Nucleotides, 1995, 14:969), or adamantane acetic acid (Manoharan et al., Tetrahedron Lett., 1995, 36:3651), a palmityl moiety (Mishra et al., Biochem. Biophys. Acta, 1995, 1264:229), or an octadecylamine or hexylamino-carbonyl-oxycholesterol moiety (Crooke et al., J. Pharmacol. Exp. Ther., 1996, 277:923). Representative United States patents that teach the preparation of such oligonucleotide conjugates have been listed above. Typical conjugation protocols involve the synthesis of oligonucleotides bearing an aminolinker at one or more positions of the sequence. The amino group is then reacted with the molecule being conjugated using appropriate coupling or activating reagents. The conjugation reaction may be performed either with the oligonucleotide still bound to the solid support or following cleavage of the oligonucleotide in solution phase. Purification of the oligonucleotide conjugate by HPLC typically affords the pure conjugate. The use of a cholesterol conjugate is particularly preferred since such a moiety can increase targeting vaginal epithelium cells, a site of HPV infection.

The instant disclosure describes a wide variety of embodiments of dsRNA that are useful to silence HPV Target genes and thus to treat HPV associated disorders. While the design of the specific therapeutic agent can take a variety of forms, certain functional characteristics will distinguish preferred dsRNA from other dsRNA. In particular, features such as good serum stability, high potency, lack of induced immune response, and good drug like behaviour, all measurable by those skilled in the art, will be tested to identify preferred dsRNA of the invention. In some situations, not all of these functional aspects will be present in the preferred dsRNA. But those skilled in the art are able to optimize these variables and others to select preferred compounds of the invention.

While many nucleotide modifications are possible, the inventors have identified patterns of chemical modifications which provide significantly improved pharmacological, immunological and ulitimately therapeutic benefit. Table 3 sets out patterns of chemical modifications preferred for use with the duplex dsRNA set out in Table 1 of the invention.

Table 3

| Chemical Modification Series | Changes made to sense strand (5'-3') | Changes made to antisense stand (5'-3') |
|---|---|---|
| 1 (single phosphorothioate at the ends of both strands) | dTsdT | dTsdT |
| 2 (single phosphorothioate at the ends of both strands plus, 2'OMe sense strand modification of all pyrimidines and 2'Ome modification of all U's followed by and A and all C's followed by A) | dTsdT, 2'OMe@all Py | dTsdT, 2'OMe@uA, cA |
| 3 (single phosphorothioate at the ends of both strands plus, 2'OMe sense strand modification of all pyrimidines and, 2'Ome of indicted bases all U's followed by an A, all C's followed by an A, all U's followed by a G and all U's followed by a U on the antisense strand) | dTsdT, 2'OMe@all Py | dTsdT,2'OMe@uA,cA,uG,uU |
| 4 (same as 1 except addition of cholesterol conjugated to the sense strand) | Chol ("exo") | dTsdT ("exo") |
| 5 (same as 2 except cholesterol conjugated to the sense strand) | Chol ("endo") | dTsdT, 2'OMe@uA, cA |
| 6 (same as 3 except cholesterol conjugated to the sense strand) | Chol ("endo") | dTsdT,2'OMe@uA,cA,uG,uU |

Vector encoded RNAi agents

The dsRNA of the invention can also be expressed from recombinant viral vectors intracellularly in vivo. The recombinant viral vectors of the invention comprise sequences encoding the dsRNA of the invention and any suitable promoter for expressing the dsRNA sequences. Suitable promoters include, for example, the U6 or H1 RNA pol III

promoter sequences and the cytomegalovirus promoter. Selection of other suitable promoters is within the skill in the art. The recombinant viral vectors of the invention can also comprise inducible or regulatable promoters for expression of the dsRNA in a particular tissue or in a particular intracellular environment. The use of recombinant viral vectors to deliver dsRNA of the invention to cells in vivo is discussed in more detail below.

dsRNA of the invention can be expressed from a recombinant viral vector either as two separate, complementary RNA molecules, or as a single RNA molecule with two complementary regions.

Any viral vector capable of accepting the coding sequences for the dsRNA molecule(s) to be expressed can be used, for example vectors derived from adenovirus (AV); adeno-associated virus (AAV); retroviruses (e.g, lentiviruses (LV), Rhabdoviruses, murine leukemia virus); herpes virus, and the like. The tropism of viral vectors can be modified by pseudotyping the vectors with envelope proteins or other surface antigens from other viruses, or by substituting different viral capsid proteins, as appropriate.

For example, lentiviral vectors of the invention can be pseudotyped with surface proteins from vesicular stomatitis virus (VSV), rabies, Ebola, Mokola, and the like. AAV vectors of the invention can be made to target different cells by engineering the vectors to express different capsid protein serotypes. For example, an AAV vector expressing a serotype 2 capsid on a serotype 2 genome is called AAV 2/2. This serotype 2 capsid gene in the AAV 2/2 vector can be replaced by a serotype 5 capsid gene to produce an AAV 2/5 vector. Techniques for constructing AAV vectors which express different capsid protein serotypes are within the skill in the art; see, e.g., Rabinowitz J E et al. (2002), J Virol 76:791-801, the entire disclosure of which is herein incorporated by reference.

Selection of recombinant viral vectors suitable for use in the invention, methods for inserting nucleic acid sequences for expressing the dsRNA into the vector, and methods of delivering the viral vector to the cells of interest are within the skill in the art. See, for example, Dornburg R (1995), Gene Therap. 2: 301-310; Eglitis M A (1988), Biotechniques 6: 608-614; Miller A D (1990), Hum Gene Therap. 1: 5-14; Anderson W F (1998), Nature 392: 25-30; and Rubinson D A et al., Nat. Genet. 33: 401-406, the entire disclosures of which are herein incorporated by reference.

Preferred viral vectors are those derived from AV and AAV. In a particularly preferred embodiment, the dsRNA of the invention is expressed as two separate, complementary single-stranded RNA molecules from a recombinant AAV vector comprising, for example, either the U6 or H1 RNA promoters, or the cytomegalovirus (CMV) promoter.

A suitable AV vector for expressing the dsRNA of the invention, a method for constructing the recombinant AV vector, and a method for delivering the vector into target cells, are described in Xia H et al. (2002), Nat. Biotech. 20: 1006-1010.

Suitable AAV vectors for expressing the dsRNA of the invention, methods for constructing the recombinant AV vector, and methods for delivering the vectors into target cells are described in Samulski R et al. (1987), J. Virol. 61: 3096-3101; Fisher K J et al. (1996), J. Virol, 70: 520-532; Samulsld R et al. (1989), J. Virol. 63: 3822-3826; U.S. Pat. No. 5,252,479; U.S. Pat. No. 5;139,941; International Patent Application No. WO 94/13788; and International Patent Application No. WO 93/24641, the entire disclosures of which are herein incorporated by reference.

III. Pharmaceutical compositions comprising dsRNA

In one embodiment, the invention provides pharmaceutical compositions comprising a dsRNA, as described herein, and a pharmaceutically acceptable carrier. The pharmaceutical composition comprising the dsRNA is useful for treating a disease or disorder associated with the expression or activity of the HPV Target gene, such as pathological processes mediated by HPV infection. Such pharmaceutical compositions are formulated based on the mode of delivery. One example is compositions that are formulated for either topical administration in the cervix or systemic administration via parenteral delivery.

The pharmaceutical compositions of the invention are administered in dosages sufficient to inhibit expression of the HPV Target gene. The present inventors have determined that, because of their improved efficiency, compositions comprising the dsRNA of the invention can be administered at surprisingly low dosages. A dosage of 5 mg dsRNA per kilogram body weight of recipient per day is sufficient to inhibit or suppress expression of the HPV Target gene, and in the case of warts or cervical or anal treatment, may be applied directly to the infected tissue.

In general, a suitable dose of dsRNA will be in the range of 0.01 to 5.0 milligrams per kilogram body weight of the recipient per day, generally in the range of 1 microgram to 1 mg per kilogram body weight per day. The pharmaceutical composition may be administered once daily, or the dsRNA may be administered as two, three, or more sub-doses at appropriate intervals throughout the day or even using continuous infusion or delivery through a controlled release formulation of vaginal gel. In that case, the dsRNA contained in each sub-dose must be correspondingly smaller in order to achieve the total daily dosage. The dosage unit can also be compounded for delivery over several days, e.g., using a conventional sustained release formulation which provides sustained release of the dsRNA over a several day period. Sustained release formulations are well known in the art and are particularly useful for vaginal delivery of agents, such as could be used with the agents of the present invention. In this embodiment, the dosage unit contains a corresponding multiple of the daily dose.

The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a composition can include a single treatment or a series of treatments. Estimates of effective dosages and in vivo half-lives for the individual dsRNAs encompassed by the invention can be made using conventional methodologies or on the basis of in vivo testing using an appropriate animal model, as described elsewhere herein.

The inventors recognize that for a variety of reasons, including the variability of HPV genotypes, it may be desireable to treat HPV infection with more than one dsRNA of the invention at the same time. In an embodiment, a combination of dsRNA are selected to target the widest range of HPV genotypes, with the least complex mixture of dsRNA. A pharmaceutical composition of the invention comprising more than one type of dsRNA would be expected to contain dosages of individual dsRNA as described herein.

Combinations of dsRNA may be provided together in a single dosage form pharmaceutical composition. Alternatively, combination dsRNA may be provided in separate dosage forms, in which case they may be administered at the same time or at different times, and possibly by different means. The invention therefore contemplates pharmaceutical compositions comprising the desired combinations of dsRNA of the invention; and it also contemplates pharmaceutical compositions of single dsRNA which are intended to be provided as part of a combination regimen. In this latter case, the combination therapy invention is thereby a method of administering rather than a composition of matter.

Advances in mouse genetics have generated a number of mouse models for the study of various human diseases, such as pathological processes mediated by HPV infection. Such models are used for in vivo testing of dsRNA, as well as for determining a therapeutically effective dose.

Any method can be used to administer a dsRNA of the present invention to a mammal containing cells infected with HPV. For example, administration can be topical (e.g., vaginal, transdermal, etc); oral; or parenteral (e.g., by subcutaneous, intraventricular, intramuscular, or intraperitoneal injection, or by intravenous drip). Administration can be rapid (e.g., by injection), or can occur over a period of time (e.g., by slow infusion or administration of slow release formulations).

Typically, when treating a mammal having cells infected with HPV, the dsRNA molecules are administered topically in a vaginal gel or cream. For example, dsRNAs formulated with or without liposomes can be topically applied directly to the cervix, anal tract or HPV lesions such as genital warts. For topical administration, a dsRNA molecule can be formulated into compositions such as sterile and non-sterile aqueous solutions, non-aqueous solutions in common solvents such as alcohols, or solutions in liquid or solid oil bases. Such solutions also can contain buffers, diluents, and other suitable additives. Compositions for topical administration can be formulated in the form of transdermal patches, ointments, lotions, creams, gels, drops, suppositories, sprays, liquids, and powders. Gels and creams may be formulated using polymers and permeabilizers known in the art. Gels or creams containing the dsRNA and associated excipients may be applied to the cervix using a cervical cap, vaginal diaphragm, coated condom, glove, and the like. Conventional pharmaceutical carriers, aqueous, powder or oily bases, thickeners, and the like can be added.

For parenteral, intrathecal, or intraventricular administration, a dsRNA molecule can be formulated into compositions such as sterile aqueous solutions, which also can contain buffers, diluents, and other suitable additives (e.g., penetration enhancers, carrier compounds, and other pharmaceutically acceptable carriers).

In addition, dsRNA molecules can be administered to a mammal containing HPV-infected cells using non-viral methods, such as biologic or abiologic means as described in, for example, U.S. Pat. No. 6,271,359. Abiologic delivery can be accomplished by a variety of methods including, without limitation, (1) loading liposomes with a dsRNA acid molecule provided herein and (2) complexing a dsRNA molecule with lipids or liposomes to form nucleic acid-lipid or nucleic acid-liposome complexes. The liposome can be composed of cationic and neutral lipids commonly used to transfect cells in vitro. Cationic lipids can complex (e.g., charge-associate) with negatively charged nucleic acids to form liposomes. Examples of cationic liposomes include, without limitation, lipofectin, lipofectamine, lipofectace, DOTAP (1,2-dioleoyl-3-trimethylammonium propane), DOTMA (N-[1,2(2,3-dioleyloxy)propyl]-N,N,N-trimethylammonium chloride), DOSPA (2,3-dioleoyloxy-N-[2-(sperminecarboxamido)ethyl]-N,N-deimethyl-1-propanaminium), DOGS (dioctadecyl amido glycil spermine), and DC-chol (3,[N-$N^1$,N-dimethylethylenediamine)-carbamoyl]cholesterol).

Procedures for forming liposomes are well known in the art. Liposome compositions can be formed, for example, from phosphatidylcholine, dimyristoyl phosphatidylcholine, dipalmitoyl phosphatidylcholine, dimyristoyl phosphatidylglycerol, or dioleoyl phosphatidylethanolamine. Numerous lipophilic agents are commercially available, including Lipofectin.RTM. (Invitrogen/Life Technologies, Carlsbad, Calif.) and Effectene.TM. (Qiagen, Valencia, Calif.). In addition, systemic delivery methods can be optimized using commercially available cationic lipids such as DDAB or DOTAP, each of which can be mixed with a neutral lipid such as DOPE or cholesterol. In some cases, liposomes such as those described by Templeton et al. (Nature Biotechnology, 15: 647-652 (1997)) can be used. In other embodiments, polycations such as polyethyleneimine can be used to achieve delivery in vivo and ex vivo (Boletta et al., J. Am Soc. Nephrol. 7: 1728 (1996)). Additional information regarding the use of liposomes to deliver nucleic acids can be found in U.S. Pat. No. 6,271,359, PCT Publication WO 96/40964 and Morrissey, D. et al. 2005. Nat Biotechnol. 23(8):1002-7.

Other non-viral methods of administering dsRNA molecules to a mammal containing HPV-infected cells include cationic lipid-based delivery systems (in addition to lipsomes) such as lipoplexes and nanoemulsions. Additionally, condensing polymeric delivery systems (i.e., DNA-polymer complexes, or "polyplexes") may be used, including but not limited to chitosans, poly(L-lysine)(PLL), polyethylenimine (PEI), dendrimers (e.g., polyamidoamine (PANAM) dendrimers), and poloxamines. Additionally, noncondensing polymeric delivery systems may be used, including but not limited to poloxamers, gelatin, PLGA (polylactic-co-glycolic acid), PVP (polyvinylpyrrolidone), and PVA (polyvinyl alcohol).

Procedures for the above-mentioned delivery or administration techniques are well known in the art. For instance, condensing polymeric delivery systems work by easily complexing with anionic DNA molecules; for example, poly(L-lysine)(PLL) works by forming a positively charged complex that interacts with negatively charged cell surface and subsequently undergoing rapid internalization.

Biologic delivery can be accomplished by a variety of methods including, without limitation, the use of viral vectors. For example, viral vectors (e.g., adenovirus and herpesvirus vectors) can be used to deliver dsRNA molecules to skin cells and cervical cells. Standard molecular biology techniques can be used to introduce one or more of the dsRNAs provided herein into one of the many different viral vectors previously developed to deliver nucleic acid to cells. These resulting viral vectors can be used to deliver the one or more dsRNAs to cells by, for example, infection.

dsRNAs of the present invention can be formulated in a pharmaceutically acceptable carrier or diluent. A "pharmaceutically acceptable carrier" (also referred to herein as an "excipient") is a pharmaceutically acceptable solvent, suspending agent, or any other pharmacologically inert vehicle. Pharmaceutically acceptable carriers can be liquid or solid, and can be selected with the planned manner of administration in mind so as to provide for the desired bulk, consistency, and other pertinent transport and chemical properties. Typical pharmaceutically acceptable carriers include, by way of example and not limitation: water; saline solution; binding agents (e.g., polyvinylpyrrolidone or hydroxypropyl methylcellulose); fillers (e.g., lactose and other sugars, gelatin, or calcium sulfate); lubricants (e.g., starch, polyethylene glycol, or sodium acetate); disintegrates (e.g., starch or sodium starch glycolate); and wetting agents (e.g., sodium lauryl sulfate).

In addition, dsRNA that target the HPV Target gene can be formulated into compositions containing the dsRNA admixed, encapsulated, conjugated, or otherwise associated with other molecules, molecular structures, or mixtures of nucleic acids. For example, a composition containing one or more dsRNA agents that target the E6AP gene can contain other therapeutic agents such as anti-inflammatory drugs (e.g., nonsteroidal anti-inflammatory drugs and corticosteroids) and antiviral drugs (e.g., ribivirin, vidarabine, acyclovir, and ganciclovir). In some embodiments, a composition can contain one or more dsRNAs having a sequence complementary to the HPV Target gene in combination with a keratolytic agent. Keratolytic agents are agents that separate or loosen the horny layer of the epidermis. An example of a keratolytic agent includes, without limitation, salicylic acid. Other examples are provided in U.S. Pat. No. 5,543,417. Keratolytic agents can be used in an amount effective to enhance the penetration of dsRNAs, for example, into tissues such as skin. For example, a keratolytic agent can be used in an amount that allows a dsRNA applied to a genital wart to penetrate throughout the wart.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred.

The data obtained from cell culture assays and animal studies can be used in formulation a range of dosage for use in humans. The dosage of compositions of the invention lies generally within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range of the compound or, when appropriate, of the polypeptide product of a target sequence (e.g., achieving a decreased concentration of the polypeptide) that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

In addition to their administration individually or as a plurality, as discussed above, the dsRNAs of the invention can be administered in combination with other known agents effective in treatment of pathological processes mediated by HPV infection. In any event, the administering physician can adjust the amount and timing of dsRNA administration on the basis of results observed using standard measures of efficacy known in the art or described herein.

Combinations of dsRNA can be tested in vitro and in vivo using the same methods employed for identification of preferred single dsRNA. Such combinations may be selected based on a purely bioinformatics basis, wherein the minimum number of siRNA are selected which provide coverage over the widest range of genotypes. Alternatively, such combinations may be selected based on in vitro or in vivo evaluations along the lines of those described herein for single

dsRNA agents. A preferred assay for testing combinations of dsRNA is to evaluate the phenotypic conseuqences of siRNA mediated HPV target knockdown in HPV 16 positive cancer cell lines (e.g. SiHa or Caski, as described in, e.g., Hengstermann et al. (2005) Journal Vir. 79(14): 9296; and Butz et al. (2003) Oncogene 22: 5938), or in organotypic culture systems, as described in, e.g., Jeon et al. (1995) Journal Vir. 69(5):2989.

The inventors have identified certain preferred combinations of dsRNA which may be used to treat HPV infection. In the most general terms, the combination of dsRNA comprises more than one dsRNA selected from among Table 1. Thus the invention contemplates the use of 2, 3, 4, 5 or more dsRNA duplexes selected from among Table 1 in a combination therapy. In principle, the smallest number of dsRNA is preferred for simplicity of the therapeutic product. This forces the selection of dsRNA which will cover the greatest number of deleterious or potentially deleterious HPV genotypes, and indeed may justify selection of a combination that does not necessarily cover all such HPV genotypes.

**Methods for treating diseases caused by HPV infection**

The methods and compositions described herein can be used to treat diseases and conditions caused by human papillomavirus, which can be the result of clinical or sub-clinical papillomavirus infections. Such diseases and conditions, herein sometimes called "HPV associated disorders" or "pathological processes mediated by HPV infection", include, e.g., epithelial malignancies, skin cancer (non-melanoma or melanoma), anogenital malignancies such as cervical cancer, HPV associated precancerous lesions (including LSIL or HSIL cervical tissue), anal carcinoma, malignant lesions, benign lesions, papillomacarcinomas, papilloadenocystomas, papilloma neuropathicum, papillomatosis, cutaneous and mucosal papillomas, condylomas, fibroblastic tumors, and other pathological conditions associated with papillomavirus.

For example, the compositions described herein can be used to treat warts caused by HPV. Such warts include, e.g., common warts (verruca vulgaris), for example, palmar, plantar, and periungual warts; flat and filiform warts; anal, oral, pharyngeal, laryngeal, and tongue papillomas; and venereal warts (condyloma accuminata), also known as genital warts (for example, penile, vulvar, vaginal and cervical warts), which are one of the most serious manifestations of HPV infection. HPV DNA can be found in all grades of cervical intraepithelial neoplasia (CIN I-III), and a specific subset of HPV types can be found in carcinoma in situ of the cervix. Consequently, women with genital warts, containing specific HPV types, are considered to be at high risk for the development of cervical cancer.

The most common disease associated with papillomavirus infection is benign skin warts, or common warts. Common warts generally contain HPV types 1, 2, 3, 4 or 10. Other conditions caused by papillomavirus include, e.g., laryngeal papillomas, which are benign epithelial tumors of the larynx. Two papillomavirus types, HPV-6 and HPV-11, are most commonly associated with laryngeal papillomas. The compositions described herein can be used to treat these diseases and conditions.

The compositions described herein can also be used in the treatment of epidermodysplasia verruciformis (EV), a rare genetically transmitted disease characterized by disseminated flat warts that appear as small reddish macules.

In addition, the compositions described herein can be used to treat lesions resulting from cellular transformation for which HPV is an etiological agent, e.g., in the treatment of cervical cancer.

The compositions described herein can also be used in the treatment of HPV-induced dysplasias, e.g., penile, vulvar, cervical, vaginal oral, anal, and pharyngeal dysplasias, and in the treatment of HPV-induced cancers, e.g., penile, vulvar, cervical, vaginal, anal, oral, pharyngeal, and head and neck cancers.

The invention can also be practiced by including a specific dsRNA in combination with another anti-cancer chemotherapeutic agent, such as any conventional chemotherapeutic agent. The combination of a specific binding agent with such other agents can potentiate the chemotherapeutic protocol. Numerous chemotherapeutic protocols will present themselves in the mind of the skilled practitioner as being capable of incorporation into the method of the invention. Any chemotherapeutic agent can be used, including alkylating agents, antimetabolites, hormones and antagonists, radioisotopes, as well as natural products. For example, the compound of the invention can be administered with antibiotics such as doxorubicin and other anthracycline analogs, nitrogen mustards such as cyclophosphamide, pyrimidine analogs such as 5-fluorouracil, cisplatin, hydroxyurea, taxol and its natural and synthetic derivatives, and the like. As another example, in the case of mixed tumors, such as adenocarcinoma of the breast, where the tumors include gonadotropin-dependent and gonadotropin-independent cells, the compound can be administered in conjunction with leuprolide or goserelin (synthetic peptide analogs of LH-RH). Other antineoplastic protocols include the use of a tetracycline compound with another treatment modality, e.g., surgery, radiation, etc., also referred to herein as "adjunct antineoplastic modalities." Thus, the method of the invention can be employed with such conventional regimens with the benefit of reducing side effects and enhancing efficacy.

In a further alternative, the dsRNA targeting E6AP may be employed to treat neurological and behavioural disorders. E6AP has been implicated in neurological and behavioural disorders through the identification of E6AP mutations in patients having Angelman syndrome. Angelman syndrome (AS) is an imprinted neurobehavioral disorder characterized by mental retardation, absent speech, excessive laughter, seizures, ataxia, and a characteristic EEG

pattern. (Hitchins, M.P. et al. 2004. Am J Med Genet A. 125(2):167-72.) It would not, presumably, be the intent of treatment to induce such conditions; rather, as observed in many hereditary defects, this evidence that E6AP has a critical role in neurological and behavioural conditions also indicates that this target may have a variety of roles in human pathologies and is likely a suitable target for other diseases in this class where silencing of E6AP will compensate for other biochemical defects or diseases. As used herein "E6AP associated disorders" include the HPV associated disorders noted above and other neurological and behavioural disorders.

Methods for inhibiting expression of the E6AP gene

In yet another aspect, the invention provides a method for inhibiting the expression of the E6AP gene in a mammal. The method comprises administering a composition of Table 1 of the invention to the mammal such that expression of the target E6AP gene is silenced. Because of their high specificity, such dsRNAs of the invention specifically target RNAs (primary or processed) of the target E6AP gene. Compositions and methods for inhibiting the expression of these E6AP genes using such dsRNAs can be performed as described elsewhere herein.

In one embodiment, the method comprises administering a composition comprising a dsRNA, wherein the dsRNA comprises a nucleotide sequence which is complementary to at least a part of an RNA transcript of the E6AP gene of the mammal to be treated. When the organism to be treated is a mammal such as a human, the composition may be administered by any means known in the art including, but not limited to oral or parenteral routes, including intravenous, intramuscular, subcutaneous, transdermal, airway (aerosol), nasal, rectal, vaginal and topical (including buccal and sublingual) administration. In preferred embodiments, the compositions are administered by topical/vaginal administration or by intravenous infusion or injection.

Unless otherwise defined, all technical and scientific terms used herein have the same meaning as commonly understood by one of ordinary skill in the art to which this invention belongs. Although methods and materials similar or equivalent to those described herein can be used in the practice or testing of the invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

## EXAMPLES

### Gene Walking of the E6AP gene

siRNA design was carried out to identify siRNAs targeting human ubiquitin protein ligase E3A (ube3A, also referred to as E6AP). Human mRNA sequences to E6AP representing different isoforms (NM_130838.1, NM_130839.1, NM_000462.2) were used.

The ClustalW multiple alignment function (Thompson J.D., et al., Nucleic Acids Res. 1994, 22:4673) of the BioEdit software was used with all human E6AP isoforms to identify mRNA sequence NM_130838.1 as shortest sequence as well as to confirm sequence conservation from position 5 to 4491 (end position) of the reference sequence, a requirement for efficient targeting of all E6AP isoforms.

All possible overlapping 19mers (representing siRNA sense strand sequences) spanning E6AP reference sequence NM_130838.1 were identified, resulting in 4473 19mer candidate sequences. Combined, these candidate target sequences cover the 5'UTR, coding and 3'UTR domains of the E6AP mRNA, and the junction sites of these domains.

In order to rank and select siRNAs out of the pool of candidates, the predicted potential for interacting with irrelevant targets (off-target potential) was taken as a ranking parameter. siRNAs with low off-target potential were defined as preferable and assumed to be more specific *in vivo.*

For predicting siRNA-specific off-target potential, the following assumptions were made:

1) complementarity to a target gene in positions 2 to 9 (counting 5' to 3') of a strand (seed region) may be sufficient for interaction of that strand with the mRNA transcribed from the target gene and subsequent downregulation (Jackson AL, et al. Nat Biotechnol. 2003 Jun;21(6):635-7)

2) positions 1 and 19 of each strand are not relevant for off-target interactions

3) seed region may contribute more to off-target potential than rest of sequence

4) cleavage site region positions 10 and 11 (counting 5' to 3') of a strand may contribute more to off-target potential than the sequences 3' to the cleavage site (non-seed region), but not as much as the seed region

5) an off-target score can be calculated for each gene and each strand, based on complementarity of siRNA strand sequence to the gene's sequence and position of mismatches while considering assumptions 1 to 4

6) assuming potential abortion of sense strand activity by internal modifications introduced, only off-target potential of antisense strand will be relevant

7) the off-target potential of an siRNA can be inferred from the gene displaying the highest homology according to our criteria (best off-target gene), thus can be expressed by the off-target score of the respective gene

To identify potential off-target genes, 19mer antisense sequences were subjected to a homology search against publicly available human mRNA sequences. To this purpose, FastA (version 3.4) searches were performed with all 19mer candidate antisense sequences against the human RefSeq database. A Perl script was used to generate antisense sequences from the candidate 19mer sequences (perl script 2). FastA search was executed with parameter/value pairs -g 30 -f30 -L-i -H in order to take into account the homology over the full length of the 19mer and to format the output suitable for the script analysis in the next step. The search resulted in a list of potential off-target genes for candidate siRNAs.

Further, FastA search parameters were applied with values -E 15000 in order to make database entries with more than 8 contiguous nucleobases identical to the 19mer sense strand sequences very likely to be transferred to a FastA output file while displaying the homology of the complete 19mer length (see assumption 1).

In order to identify the best off-target gene and its off-target score, the FastA output file was analyzed. The following off-target properties for each 19mer input sequence were extracted for each potential off-target gene:

Number of mismatches in seed region

Number of mismatches in non-seed region

Number of mismatches in cleavage site region

The off-target score for each off-target gene was calculated as follows:

(number of seed mismatches multiplied by 10) + (number of cleavage site mismatches multiplied by 1.2) + number of non-seed mismatches

The lowest off-target score was extracted for each input 19mer sequence and successively written into an output file resulting in a list of off-target scores for all siRNAs corresponding to the input 19mer sequences.

In order to generate a ranking of siRNAs, off-target scores were entered into a result table. All siRNAs were finally sorted in descending order according to the off-target score and sequences containing stretches with more than 3 Gs in a row were excluded from selection.

The 327 siRNAs with an off-target score of >=3 were selected and synthesized (Table 1).

Table 1 dsRNA targeting E6AP

| Target sequence of mRNA from human reference sequence NM_130838 (human iso3) | SEQ ID NO: | Sense strand (target sequence) having double overhang | SEQ ID NO: | Antisense strand (guide sequence) having double overhang | SEQ ID NO: | |
|---|---|---|---|---|---|---|
| sequence of total 19mer target site | | sequence (5´-3´) | | sequence (5´-3´) | | |
| GUGAUAGCUCACAGGGAGA | 1 | GUGAUAGCUCACAGGGAGATT | 328 | UCUCCCUGUGAGCUAUCACTT | 655 | ND-10118 |
| CUCAGCUUACCUUGAGAAC | 2 | CUCAGCUUACCUUGAGAACTT | 329 | GUUCUCAAGGUAAGCUGAGTT | 656 | ND-10119 |
| GAGGUUUUCAUAUGGUGAC | 3 | GAGGUUUUCAUAUGGUGACTT | 330 | GUCACCAUAUGAAAACCUCTT | 657 | ND-10120 |
| AAUCCUGCAGACUUGAAGA | 4 | AAUCCUGCAGACUUGAAGATT | 331 | UCUUCAAGUCUGCAGGAUUTT | 658 | ND-10121 |
| GGUGAUAGCUCACAGGGAG | 5 | GGUGAUAGCUCACAGGGAGTT | 332 | CUCCCUGUGAGCUAUCACCTT | 659 | ND-10122 |
| AAAUGGUUUACUAUGCAAA | 6 | AAAUGGUUUACUAUGCAAATT | 333 | UUUGCAUAGUAAACCAUUUTT | 660 | ND-10123 |
| CAGUUAACUGAGGGCUGUG | 7 | CAGUUAACUGAGGGCUGUGTT | 334 | CACAGCCCUCAGUUAACUGTT | 661 | ND-10124 |
| ACUACAUUCUCAAUAAAUC | 8 | ACUACAUUCUCAAUAAAUCTT | 335 | GAUUUAUUGAGAAUGUAGUTT | 662 | ND-10125 |
| CAUCCCUCCAAGAAAGGAG | 9 | CAUCCCUCCAAGAAAGGAGTT | 336 | CUCCUUUCUUGGAGGGAUGTT | 663 | ND-10126 |
| GAUUAGGGAGUUCUGGGAA | 10 | GAUUAGGGAGUUCUGGGAATT | 337 | UUCCCAGAACUCCCUAAUCTT | 664 | ND-10127 |
| UGAUAGCUCACAGGGAGAC | 11 | UGAUAGCUCACAGGGAGACTT | 338 | GUCUCCCUGUGAGCUAUCATT | 665 | ND-10128 |
| UCCACAGUCCUGAAUAUCU | 12 | UCCACAGUCCUGAAUAUCUTT | 339 | AGAUAUUCAGGACUGUGGATT | 666 | ND-10129 |
| CUCCACAGUCCUGAAUAUC | 13 | CUCCACAGUCCUGAAUAUCTT | 340 | GAUAUUCAGGACUGUGGAGTT | 667 | ND-10130 |
| UGGGUCUGGCUAUUUACAA | 14 | UGGGUCUGGCUAUUUACAATT | 341 | UUGUAAAUAGCCAGACCCATT | 668 | ND-10131 |
| AAAAUCCUGCAGACUUGAA | 15 | AAAAUCCUGCAGACUUGAATT | 342 | UUCAAGUCUGCAGGAUUUUTT | 669 | ND-10132 |
| AAAAUGGUUUACUAUGCAA | 16 | AAAAUGGUUUACUAUGCAATT | 343 | UUGCAUAGUAAACCAUUUUTT | 670 | ND-10133 |
| CAAAGGAUUUGGCAUGCUG | 17 | CAAAGGAUUUGGCAUGCUGTT | 344 | CAGCAUGCCAAAUCCUUUGTT | 671 | ND-10134 |
| CACGAAUGAGUUUUGUGCU | 18 | CACGAAUGAGUUUUGUGCUTT | 345 | AGCACAAAACUCAUUCGUGTT | 672 | ND-10135 |
| CUUUUCGUGACUUGGGAGA | 19 | CUUUUCGUGACUUGGGAGATT | 346 | UCUCCCAAGUCACGAAAAGTT | 673 | ND-10136 |
| UGGUCUAAAUACAAUGCAG | 20 | UGGUCUAAAUACAAUGCAGTT | 347 | CUGCAUUGUAUUUAGACCATT | 674 | ND-10137 |
| GUGGUCUAAAUACAAUGCA | 21 | GUGGUCUAAAUACAAUGCATT | 348 | UGCAUUGUAUUUAGACCACTT | 675 | ND-10138 |
| AGGUGAUAGCUCACAGGGA | 22 | AGGUGAUAGCUCACAGGGATT | 349 | UCCCUGUGAGCUAUCACCUTT | 676 | ND-10139 |
| CCUCGAGCUUUAUAAGAUU | 23 | CCUCGAGCUUUAUAAGAUUTT | 350 | AAUCUUAUAAAGCUCGAGGTT | 677 | ND-10140 |

| | | | | | | |
|---|---|---|---|---|---|---|
| GAGAACUCGAAAGGUGCCC | 24 | GAGAACUCGAAAGGUGCCCTT | 351 | GGGCACCUUUCGAGUUCUCTT | 678 | ND-10141 |
| ACCAGUUAACUGAGGGCUG | 25 | ACCAGUUAACUGAGGGCUGTT | 352 | CAGCCCUCAGUUAACUGGUTT | 679 | ND-10142 |
| CCUGCACGAAUGAGUUUUG | 26 | CCUGCACGAAUGAGUUUUGTT | 353 | CAAAACUCAUUCGUGCAGGTT | 680 | ND-10143 |
| CCCUCGAGCUUUAUAAGAU | 27 | CCCUCGAGCUUUAUAAGAUTT | 354 | AUCUUAUAAAGCUCGAGGGTT | 681 | ND-10144 |
| CUUACCUUGAGAACUCGAA | 28 | CUUACCUUGAGAACUCGAATT | 355 | UUCGAGUUCUCAAGGUAAGTT | 682 | ND-10145 |
| CUGUGGUCUAAAUACAAUG | 29 | CUGUGGUCUAAAUACAAUGTT | 356 | CAUUGUAUUUAGACCACAGTT | 683 | ND-10146 |
| UCAGACUGUGGUCUAAAUA | 30 | UCAGACUGUGGUCUAAAUATT | 357 | UAUUUAGACCACAGUCUGATT | 684 | ND-10147 |
| UUGAGAACUCGAAAGGUGC | 31 | UUGAGAACUCGAAAGGUGCTT | 358 | GCACCUUUCGAGUUCUCAATT | 685 | ND-10148 |
| AGCUUAGUUCAAGGACAGC | 32 | AGCUUAGUUCAAGGACAGCTT | 359 | GCUGUCCUUGAACUAAGCUTT | 686 | ND-10149 |
| CAGCUUAGUUCAAGGACAG | 33 | CAGCUUAGUUCAAGGACAGTT | 360 | CUGUCCUUGAACUAAGCUGTT | 687 | ND-10150 |
| GACUUACUUAACAGAAGAG | 34 | GACUUACUUAACAGAAGAGTT | 361 | CUCUUCUGUUAAGUAAGUCTT | 688 | ND-10151 |
| UUCAUAUGGUGACCAAUGA | 35 | UUCAUAUGGUGACCAAUGATT | 362 | UCAUUGGUCACCAUAUGAATT | 689 | ND-10152 |
| CAUAUGGUGACCAAUGAAU | 36 | CAUAUGGUGACCAAUGAAUTT | 363 | AUUCAUUGGUCACCAUAUGTT | 690 | ND-10153 |
| AUAGAAAUCUCCACAGUCC | 37 | AUAGAAAUCUCCACAGUCCTT | 364 | GGACUGUGGAGAUUUCUAUTT | 691 | ND-10154 |
| AUUCUGACUACAUUCUCAA | 38 | AUUCUGACUACAUUCUCAATT | 365 | UUGAGAAUGUAGUCAGAAUTT | 692 | ND-10155 |
| GCACGAAUGAGUUUUGUGC | 39 | GCACGAAUGAGUUUUGUGCTT | 366 | GCACAAAACUCAUUCGUGCTT | 693 | ND-10156 |
| GCAUUGGUACAGAGCUUCC | 40 | GCAUUGGUACAGAGCUUCCTT | 367 | GGAAGCUCUGUACCAAUGCTT | 694 | ND-10157 |
| CACCAGUUAACUGAGGGCU | 41 | CACCAGUUAACUGAGGGCUTT | 368 | AGCCCUCAGUUAACUGGUGTT | 695 | ND-10158 |
| GCUUACCUUGAGAACUCGA | 42 | GCUUACCUUGAGAACUCGATT | 369 | UCGAGUUCUCAAGGUAAGCTT | 696 | ND-10159 |
| UUACCUUGAGAACUCGAAA | 43 | UUACCUUGAGAACUCGAAATT | 370 | UUUCGAGUUCUCAAGGUAATT | 697 | ND-10160 |
| GAUUUGGCAUGCUGUAAAA | 44 | GAUUUGGCAUGCUGUAAAATT | 371 | UUUUACAGCAUGCCAAAUCTT | 698 | ND-10161 |
| UGCUUGAAAAUGGUUUACU | 45 | UGCUUGAAAAUGGUUUACUTT | 372 | AGUAAACCAUUUUCAAGCATT | 699 | ND-10162 |
| UGCACGAAUGAGUUUUGUG | 46 | UGCACGAAUGAGUUUUGUGTT | 373 | CACAAAACUCAUUCGUGCATT | 700 | ND-10163 |
| UUUCGUGACUUGGGAGACU | 47 | UUUCGUGACUUGGGAGACUTT | 374 | AGUCUCCCAAGUCACGAAATT | 701 | ND-10164 |
| UGAUCAGACUGUGGUCUAA | 48 | UGAUCAGACUGUGGUCUAATT | 375 | UUAGACCACAGUCUGAUCATT | 702 | ND-10165 |
| UUCUGACUACAUUCUCAAU | 49 | UUCUGACUACAUUCUCAAUTT | 376 | AUUGAGAAUGUAGUCAGAATT | 703 | ND-10166 |
| GUGCUUGAAAAUGGUUUAC | 50 | GUGCUUGAAAAUGGUUUACTT | 377 | GUAAACCAUUUUCAAGCACTT | 704 | ND-10167 |
| UUAUCUGAAUUUGUUCAUU | 51 | UUAUCUGAAUUUGUUCAUUTT | 378 | AAUGAACAAAUUCAGAUAATT | 705 | ND-10168 |
| AAUAGAAAUCUCCACAGUC | 52 | AAUAGAAAUCUCCACAGUCTT | 379 | GACUGUGGAGAUUUCUAUUTT | 706 | ND-10169 |
| GACUGUGGUCUAAAUACAA | 53 | GACUGUGGUCUAAAUACAATT | 380 | UUGUAUUUAGACCACAGUCTT | 707 | ND-10170 |
| CUACAUUCUCAAUAAAUCA | 54 | CUACAUUCUCAAUAAAUCATT | 381 | UGAUUUAUUGAGAAUGUAGTT | 708 | ND-10171 |
| AGAUGUGACUUACUUAACA | 55 | AGAUGUGACUUACUUAACATT | 382 | UGUUAAGUAAGUCACAUCUTT | 709 | ND-10172 |

| | | | | | | |
|---|---|---|---|---|---|---|
| AUUUGGCAUGCUGUAAAAC | 56 | AUUUGGCAUGCUGUAAAACTT | 383 | GUUUUACAGCAUGCCAAAUTT | 710 | ND-10173 |
| GUACAUUUUCCCAUGGUUG | 57 | GUACAUUUUCCCAUGGUUGTT | 384 | CAACCAUGGGAAAAUGUACTT | 711 | ND-10174 |
| UCGUAAUGGAGAAUAGAAA | 58 | UCGUAAUGGAGAAUAGAAATT | 385 | UUUCUAUUCUCCAUUACGATT | 712 | ND-10175 |
| CUUAGUUCAAGGACAGCAG | 59 | CUUAGUUCAAGGACAGCAGTT | 386 | CUGCUGUCCUUGAACUAAGTT | 713 | ND-10176 |
| GUCUAAAUACAAUGCAGAC | 60 | GUCUAAAUACAAUGCAGACTT | 387 | GUCUGCAUUGUAUUUAGACTT | 714 | ND-10177 |
| UGUACAUUUUCCCAUGGUU | 61 | UGUACAUUUUCCCAUGGUUTT | 388 | AACCAUGGGAAAAUGUACATT | 715 | ND-10178 |
| UUGGUGUUAAAACCCUGGA | 62 | UUGGUGUUAAAACCCUGGATT | 389 | UCCAGGGUUUUAACACCAATT | 716 | ND-10179 |
| UUUUCCCAUGGUUGUCUAC | 63 | UUUUCCCAUGGUUGUCUACTT | 390 | GUAGACAACCAUGGGAAAATT | 717 | ND-10180 |
| UUUAUUAAUGAACCACUGA | 64 | UUUAUUAAUGAACCACUGATT | 391 | UCAGUGGUUCAUUAAUAAATT | 718 | ND-10181 |
| GGGUCUACACCAGAUUGCU | 65 | GGGUCUACACCAGAUUGCUTT | 392 | AGCAAUCUGGUGUAGACCCTT | 719 | ND-10182 |
| CUGACUACAUUCUCAAUAA | 66 | CUGACUACAUUCUCAAUAATT | 393 | UUAUUGAGAAUGUAGUCAGTT | 720 | ND-10183 |
| GCCCAAGGAAAACUGAUCA | 67 | GCCCAAGGAAAACUGAUCATT | 394 | UGAUCAGUUUUCCUUGGGCTT | 721 | ND-10184 |
| AUCAGACUGUGGUCUAAAU | 68 | AUCAGACUGUGGUCUAAAUTT | 395 | AUUUAGACCACAGUCUGAUTT | 722 | ND-10185 |
| GAAUUCGCAUGUACAGUGA | 69 | GAAUUCGCAUGUACAGUGATT | 396 | UCACUGUACAUGCGAAUUCTT | 723 | ND-10186 |
| GAUCGCUAUGGAAAAUCCU | 70 | GAUCGCUAUGGAAAAUCCUTT | 397 | AGGAUUUUCCAUAGCGAUCTT | 724 | ND-10187 |
| AUCGCUAUGGAAAAUCCUG | 71 | AUCGCUAUGGAAAAUCCUGTT | 398 | CAGGAUUUUCCAUAGCGAUTT | 725 | ND-10188 |
| UUAGGGAGUUCUGGGAAAU | 72 | UUAGGGAGUUCUGGGAAAUTT | 399 | AUUUCCCAGAACUCCCUAATT | 726 | ND-10189 |
| GAUUAUAGCCAAAAAUGGC | 73 | GAUUAUAGCCAAAAAUGGCTT | 400 | GCCAUUUUUGGCUAUAAUCTT | 727 | ND-10190 |
| AGAUGAUCGCUAUGGAAAA | 74 | AGAUGAUCGCUAUGGAAAATT | 401 | UUUUCCAUAGCGAUCAUCUTT | 728 | ND-10191 |
| AGAUAUCACAGACAGAUCU | 75 | AGAUAUCACAGACAGAUCUTT | 402 | AGAUCUGUCUGUGAUAUCUTT | 729 | ND-10192 |
| GGUAUGUUCACAUACGAUG | 76 | GGUAUGUUCACAUACGAUGTT | 403 | CAUCGUAUGUGAACAUACCTT | 730 | ND-10193 |
| ACAAUAGAAUUCGCAUGUA | 77 | ACAAUAGAAUUCGCAUGUATT | 404 | UACAUGCGAAUUCUAUUGUTT | 731 | ND-10194 |
| GGCUAGAGAUGAUCGCUAU | 78 | GGCUAGAGAUGAUCGCUAUTT | 405 | AUAGCGAUCAUCUCUAGCCTT | 732 | ND-10195 |
| CUGUCCAACUUUUCUUCGU | 79 | CUGUCCAACUUUUCUUCGUTT | 406 | ACGAAGAAAAGUUGGACAGTT | 733 | ND-10196 |
| AUGCACUUGUCCGGCUAGA | 80 | AUGCACUUGUCCGGCUAGATT | 407 | UCUAGCCGGACAAGUGCAUTT | 734 | ND-10197 |
| AAUCCAGAUAUUGGUAUGU | 81 | AAUCCAGAUAUUGGUAUGUTT | 408 | ACAUACCAAUAUCUGGAUUTT | 735 | ND-10198 |
| UCCGGCUAGAGAUGAUCGC | 82 | UCCGGCUAGAGAUGAUCGCTT | 409 | GCGAUCAUCUCUAGCCGGATT | 736 | ND-10199 |
| GAUUGUCGAAAACCACUUA | 83 | GAUUGUCGAAAACCACUUATT | 410 | UAAGUGGUUUUCGACAAUCTT | 737 | ND-10200 |
| UGAUCGCUAUGGAAAAUCC | 84 | UGAUCGCUAUGGAAAAUCCTT | 411 | GGAUUUUCCAUAGCGAUCATT | 738 | ND-10201 |
| AUGAUCGCUAUGGAAAAUC | 85 | AUGAUCGCUAUGGAAAAUCTT | 412 | GAUUUUCCAUAGCGAUCAUTT | 739 | ND-10202 |
| CUUAUAUGUGGAAGCCGGA | 86 | CUUAUAUGUGGAAGCCGGATT | 413 | UCCGGCUUCCACAUAUAAGTT | 740 | ND-10203 |
| GAUAUCACAGACAGAUCUU | 87 | GAUAUCACAGACAGAUCUUTT | 414 | AAGAUCUGUCUGUGAUAUCTT | 741 | ND-10204 |

| | | | | | | |
|---|---|---|---|---|---|---|
| ACUUAUUCAGACCAGAAGA | 88 | ACUUAUUCAGACCAGAAGATT | 415 | UCUUCUGGUCUGAAUAAGUTT | 742 | ND-10205 |
| UGUCCAACUUUUCUUCGUA | 89 | UGUCCAACUUUUCUUCGUATT | 416 | UACGAAGAAAAGUUGGACATT | 743 | ND-10206 |
| UUAUAUGUGGAAGCCGGAA | 90 | UUAUAUGUGGAAGCCGGAATT | 417 | UUCCGGCUUCCACAUAUAATT | 744 | ND-10207 |
| AGCAGUUGAAUCCAUAUUU | 91 | AGCAGUUGAAUCCAUAUUUTT | 418 | AAAUAUGGAUUCAACUGCUTT | 745 | ND-10208 |
| UGCAAAUGUAGUGGGAGGG | 92 | UGCAAAUGUAGUGGGAGGGTT | 419 | CCCUCCCACUACAUUUGCATT | 746 | ND-10209 |
| GAUGCACUUGUCCGGCUAG | 93 | GAUGCACUUGUCCGGCUAGTT | 420 | CUAGCCGGACAAGUGCAUCTT | 747 | ND-10210 |
| GAUGAUGCACUUGUCCGGC | 94 | GAUGAUGCACUUGUCCGGCTT | 421 | GCCGGACAAGUGCAUCAUCTT | 748 | ND-10211 |
| ACUUGUCCGGCUAGAGAUG | 95 | ACUUGUCCGGCUAGAGAUGTT | 422 | CAUCUCUAGCCGGACAAGUTT | 749 | ND-10212 |
| AAGGUUACCUACAUCUCAU | 96 | AAGGUUACCUACAUCUCAUTT | 423 | AUGAGAUGUAGGUAACCUUTT | 750 | ND-10213 |
| AGAUGAUUAUAGCCAAAAA | 97 | AGAUGAUUAUAGCCAAAAATT | 424 | UUUUUGGCUAUAAUCAUCUTT | 751 | ND-10214 |
| GACUUGGGAGACUCUCACC | 98 | GACUUGGGAGACUCUCACCTT | 425 | GGUGAGAGUCUCCCAAGUCTT | 752 | ND-10215 |
| ACUUUUCUUCGUAUGGAUA | 99 | ACUUUUCUUCGUAUGGAUATT | 426 | UAUCCAUACGAAGAAAAGUTT | 753 | ND-10216 |
| AAAGGUUACCUACAUCUCA | 100 | AAAGGUUACCUACAUCUCATT | 427 | UGAGAUGUAGGUAACCUUUTT | 754 | ND-10217 |
| GACAAUAGAAUUCGCAUGU | 101 | GACAAUAGAAUUCGCAUGUTT | 428 | ACAUGCGAAUUCUAUUGUCTT | 755 | ND-10218 |
| UUUACAAUAACUGUAUACU | 102 | UUUACAAUAACUGUAUACUTT | 429 | AGUAUACAGUUAUUGUAAATT | 756 | ND-10219 |
| UUACUCUGAUUGGCAUAGU | 103 | UUACUCUGAUUGGCAUAGUTT | 430 | ACUAUGCCAAUCAGAGUAATT | 757 | ND-10220 |
| CUGGCUAUUUACAAUAACU | 104 | CUGGCUAUUUACAAUAACUTT | 431 | AGUUAUUGUAAAUAGCCAGTT | 758 | ND-10221 |
| GUAUGUUCACAUACGAUGA | 105 | GUAUGUUCACAUACGAUGATT | 432 | UCAUCGUAUGUGAACAUACTT | 759 | ND-10222 |
| AGUCAUAAGCAAUGAAUUU | 106 | AGUCAUAAGCAAUGAAUUUTT | 433 | AAAUUCAUUGCUUAUGACUTT | 760 | ND-10223 |
| GUUGAAUCCAUAUUUGAGA | 107 | GUUGAAUCCAUAUUUGAGATT | 434 | UCUCAAAUAUGGAUUCAACTT | 761 | ND-10224 |
| UUAAUGCAAAACUCUGUGA | 108 | UUAAUGCAAAACUCUGUGATT | 435 | UCACAGAGUUUUGCAUUAATT | 762 | ND-10225 |
| UGCACUUGUCCGGCUAGAG | 109 | UGCACUUGUCCGGCUAGAGTT | 436 | CUCUAGCCGGACAAGUGCATT | 763 | ND-10226 |
| CCCAAUGAUGUAUGAUCUA | 110 | CCCAAUGAUGUAUGAUCUATT | 437 | UAGAUCAUACAUCAUUGGGTT | 764 | ND-10227 |
| UACUUAUUCAGACCAGAAG | 111 | UACUUAUUCAGACCAGAAGTT | 438 | CUUCUGGUCUGAAUAAGUATT | 765 | ND-10228 |
| AUAUUGGUAUGUUCACAUA | 112 | AUAUUGGUAUGUUCACAUATT | 439 | UAUGUGAACAUACCAAUAUTT | 766 | ND-10229 |
| AAACUCUGUGAUCCUCAUC | 113 | AAACUCUGUGAUCCUCAUCTT | 440 | GAUGAGGAUCACAGAGUUUTT | 767 | ND-10230 |
| UUCCAGAUAUCACAGACAG | 114 | UUCCAGAUAUCACAGACAGTT | 441 | CUGUCUGUGAUAUCUGGAATT | 768 | ND-10231 |
| GAUCACUUUCCAGAUAUCA | 115 | GAUCACUUUCCAGAUAUCATT | 442 | UGAUAUCUGGAAAGUGAUCTT | 769 | ND-10232 |
| AUCACUUUCCAGAUAUCAC | 116 | AUCACUUUCCAGAUAUCACTT | 443 | GUGAUAUCUGGAAAGUGAUTT | 770 | ND-10233 |
| CACUUUCCAGAUAUCACAG | 117 | CACUUUCCAGAUAUCACAGTT | 444 | CUGUGAUAUCUGGAAAGUGTT | 771 | ND-10234 |
| CCAGACACAGAAAGGUUAC | 118 | CCAGACACAGAAAGGUUACTT | 445 | GUAACCUUUCUGUGUCUGGTT | 772 | ND-10235 |
| AAUAACUGUAUACUGGAUG | 119 | AAUAACUGUAUACUGGAUGTT | 446 | CAUCCAGUAUACAGUUAUUTT | 773 | ND-10236 |

| | | | | | | |
|---|---|---|---|---|---|---|
| UCACUUUCCAGAUAUCACA | 120 | UCACUUUCCAGAUAUCACATT | 447 | UGUGAUAUCUGGAAAGUGATT | 774 | ND-10237 |
| UUUAUGACAUGUCCCUUUA | 121 | UUUAUGACAUGUCCCUUUATT | 448 | UAAAGGGACAUGUCAUAAATT | 775 | ND-10238 |
| AGACACAGAAAGGUUACCU | 122 | AGACACAGAAAGGUUACCUTT | 449 | AGGUAACCUUUCUGUGUCUTT | 776 | ND-10239 |
| CCAAAGGAUUUGGCAUGCU | 123 | CCAAAGGAUUUGGCAUGCUTT | 450 | AGCAUGCCAAAUCCUUUGGTT | 777 | ND-10240 |
| CCUACAUCUCAUACUUGCU | 124 | CCUACAUCUCAUACUUGCUTT | 451 | AGCAAGUAUGAGAUGUAGGTT | 778 | ND-10241 |
| CCCAGACACAGAAAGGUUA | 125 | CCCAGACACAGAAAGGUUATT | 452 | UAACCUUUCUGUGUCUGGGTT | 779 | ND-10242 |
| AUAAAAUUCCAAUUACAAA | 126 | AUAAAAUUCCAAUUACAAATT | 453 | UUUGUAAUUGGAAUUUUAUTT | 780 | ND-10243 |
| AUUAUCGUAAUGGAGAAUA | 127 | AUUAUCGUAAUGGAGAAUATT | 454 | UAUUCUCCAUUACGAUAAUTT | 781 | ND-10244 |
| AGUUAGACGUGACCAUAUC | 128 | AGUUAGACGUGACCAUAUCTT | 455 | GAUAUGGUCACGUCUAACUTT | 782 | ND-10245 |
| AUCGUAAUGGAGAAUAGAA | 129 | AUCGUAAUGGAGAAUAGAATT | 456 | UUCUAUUCUCCAUUACGAUTT | 783 | ND-10246 |
| UUCGUAUGGAUAAUAAUGC | 130 | UUCGUAUGGAUAAUAAUGCTT | 457 | GCAUUAUUAUCCAUACGAATT | 784 | ND-10247 |
| ACGUAUCACAAUGUAUACU | 131 | ACGUAUCACAAUGUAUACUTT | 458 | AGUAUACAUUGUGAUACGUTT | 785 | ND-10248 |
| CAUCACGUAUGCCAAAGGA | 132 | CAUCACGUAUGCCAAAGGATT | 459 | UCCUUUGGCAUACGUGAUGTT | 786 | ND-10249 |
| UGUCGAAAACCACUUAUCC | 133 | UGUCGAAAACCACUUAUCCTT | 460 | GGAUAAGUGGUUUUCGACATT | 787 | ND-10250 |
| ACGAUGAAUCUACAAAAUU | 134 | ACGAUGAAUCUACAAAAUUTT | 461 | AAUUUUGUAGAUUCAUCGUTT | 788 | ND-10251 |
| UUACAACGGGCACAGACAG | 135 | UUACAACGGGCACAGACAGTT | 462 | CUGUCUGUGCCCGUUGUAATT | 789 | ND-10252 |
| GAAAUCGUUCAUUCAUUUA | 136 | GAAAUCGUUCAUUCAUUUATT | 463 | UAAAUGAAUGAACGAUUUCTT | 790 | ND-10253 |
| UCUUCGUAUGGAUAAUAAU | 137 | UCUUCGUAUGGAUAAUAAUTT | 464 | AUUAUUAUCCAUACGAAGATT | 791 | ND-10254 |
| CACAUACGAUGAAUCUACA | 138 | CACAUACGAUGAAUCUACATT | 465 | UGUAGAUUCAUCGUAUGUGTT | 792 | ND-10255 |
| UCGAAGUGCUUGAAAAUGG | 139 | UCGAAGUGCUUGAAAAUGGTT | 466 | CCAUUUUCAAGCACUUCGATT | 793 | ND-10256 |
| GUCGAAAACCACUUAUCCC | 140 | GUCGAAAACCACUUAUCCCTT | 467 | GGGAUAAGUGGUUUUCGACTT | 794 | ND-10257 |
| UCACAUACGAUGAAUCUAC | 141 | UCACAUACGAUGAAUCUACTT | 468 | GUAGAUUCAUCGUAUGUGATT | 795 | ND-10258 |
| ACGAAGAAUCACUGUUCUC | 142 | ACGAAGAAUCACUGUUCUCTT | 469 | GAGAACAGUGAUUCUUCGUTT | 796 | ND-10259 |
| ACUCUCGAGAUCCUAAUUA | 143 | ACUCUCGAGAUCCUAAUUATT | 470 | UAAUUAGGAUCUCGAGAGUTT | 797 | ND-10260 |
| UGUAUACUGGAUGUACAUU | 144 | UGUAUACUGGAUGUACAUUTT | 471 | AAUGUACAUCCAGUAUACATT | 798 | ND-10261 |
| UGACCAUAUCAUAGAUGAU | 145 | UGACCAUAUCAUAGAUGAUTT | 472 | AUCAUCUAUGAUAUGGUCATT | 799 | ND-10262 |
| UGCUUAUAUGUGGAAGCCG | 146 | UGCUUAUAUGUGGAAGCCGTT | 473 | CGGCUUCCACAUAUAAGCATT | 800 | ND-10263 |
| UAUACCAGGGACUCUGUUC | 147 | UAUACCAGGGACUCUGUUCTT | 474 | GAACAGAGUCCCUGGUAUATT | 801 | ND-10264 |
| GUACUUAUUCAGACCAGAA | 148 | GUACUUAUUCAGACCAGAATT | 475 | UUCUGGUCUGAAUAAGUACTT | 802 | ND-10265 |
| AUUGGCAUAGUACUGGGUC | 149 | AUUGGCAUAGUACUGGGUCTT | 476 | GACCCAGUACUAUGCCAAUTT | 803 | ND-10266 |
| CUGUAUACUGGAUGUACAU | 150 | CUGUAUACUGGAUGUACAUTT | 477 | AUGUACAUCCAGUAUACAGTT | 804 | ND-10267 |
| UUUUCUUCGUAUGGAUAAU | 151 | UUUUCUUCGUAUGGAUAAUTT | 478 | AUUAUCCAUACGAAGAAAATT | 805 | ND-10268 |

| | | | | | | |
|---|---|---|---|---|---|---|
| UCGGAGAGGUUUUCAUAUG | 152 | UCGGAGAGGUUUUCAUAUGTT | 479 | CAUAUGAAAACCUCUCCGATT | 806 | ND-10269 |
| GGGUCAGUUUACUCUGAUU | 153 | GGGUCAGUUUACUCUGAUUTT | 480 | AAUCAGAGUAAACUGACCCTT | 807 | ND-10270 |
| GGUCAGUUUACUCUGAUUG | 154 | GGUCAGUUUACUCUGAUUGTT | 481 | CAAUCAGAGUAAACUGACCTT | 808 | ND-10271 |
| GAAUCUCCCUUAAAGUACU | 155 | GAAUCUCCCUUAAAGUACUTT | 482 | AGUACUUUAAGGGAGAUUCTT | 809 | ND-10272 |
| CCAAUGAAUCUCCCUUAAA | 156 | CCAAUGAAUCUCCCUUAAATT | 483 | UUUAAGGGAGAUUCAUUGGTT | 810 | ND-10273 |
| GACGUGACCAUAUCAUAGA | 157 | GACGUGACCAUAUCAUAGATT | 484 | UCUAUGAUAUGGUCACGUCTT | 811 | ND-10274 |
| ACGUGACCAUAUCAUAGAU | 158 | ACGUGACCAUAUCAUAGAUTT | 485 | AUCUAUGAUAUGGUCACGUTT | 812 | ND-10275 |
| GUUCACAUACGAUGAAUCU | 159 | GUUCACAUACGAUGAAUCUTT | 486 | AGAUUCAUCGUAUGUGAACTT | 813 | ND-10276 |
| UUUAACAGUCGAAAUCUAG | 160 | UUUAACAGUCGAAAUCUAGTT | 487 | CUAGAUUUCGACUGUUAAATT | 814 | ND-10277 |
| GACGGUGGCUAUACCAGGG | 161 | GACGGUGGCUAUACCAGGGTT | 488 | CCCUGGUAUAGCCACCGUCTT | 815 | ND-10278 |
| UCACAAUGUAUACUCUCGA | 162 | UCACAAUGUAUACUCUCGATT | 489 | UCGAGAGUAUACAUUGUGATT | 816 | ND-10279 |
| AUUUAACAGUCGAAAUCUA | 163 | AUUUAACAGUCGAAAUCUATT | 490 | UAGAUUUCGACUGUUAAAUTT | 817 | ND-10280 |
| CCUGGAUUGUCGAAAACCA | 164 | CCUGGAUUGUCGAAAACCATT | 491 | UGGUUUUCGACAAUCCAGGTT | 818 | ND-10281 |
| AAGAUGUGACUUACUUAAC | 165 | AAGAUGUGACUUACUUAACTT | 492 | GUUAAGUAAGUCACAUCUUTT | 819 | ND-10282 |
| CAGACCAGAUUCGGAGAAU | 166 | CAGACCAGAUUCGGAGAAUTT | 493 | AUUCUCCGAAUCUGGUCUGTT | 820 | ND-10283 |
| CUUCGUAUGGAUAAUAAUG | 167 | CUUCGUAUGGAUAAUAAUGTT | 494 | CAUUAUUAUCCAUACGAAGTT | 821 | ND-10284 |
| GACGUAUCACAAUGUAUAC | 168 | GACGUAUCACAAUGUAUACTT | 495 | GUAUACAUUGUGAUACGUCTT | 822 | ND-10285 |
| UCGUAUGGAUAAUAAUGCA | 169 | UCGUAUGGAUAAUAAUGCATT | 496 | UGCAUUAUUAUCCAUACGATT | 823 | ND-10286 |
| CAUACGAUGAAUCUACAAA | 170 | CAUACGAUGAAUCUACAAATT | 497 | UUUGUAGAUUCAUCGUAUGTT | 824 | ND-10287 |
| GACUUGACGUAUCACAAUG | 171 | GACUUGACGUAUCACAAUGTT | 498 | CAUUGUGAUACGUCAAGUCTT | 825 | ND-10288 |
| GCUUUUCGGAGAGGUUUUC | 172 | GCUUUUCGGAGAGGUUUUCTT | 499 | GAAAACCUCUCCGAAAAGCTT | 826 | ND-10289 |
| GGAAAUCGUUCAUUCAUUU | 173 | GGAAAUCGUUCAUUCAUUUTT | 500 | AAAUGAAUGAACGAUUUCCTT | 827 | ND-10290 |
| UCGAAAACCACUUAUCCCU | 174 | UCGAAAACCACUUAUCCCUTT | 501 | AGGGAUAAGUGGUUUUCGATT | 828 | ND-10291 |
| UGUCAAUCUUUAUUCUGAC | 175 | UGUCAAUCUUUAUUCUGACTT | 502 | GUCAGAAUAAAGAUUGACATT | 829 | ND-10292 |
| ACCACUUAUCCCUUUUGAA | 176 | ACCACUUAUCCCUUUUGAATT | 503 | UUCAAAAGGGAUAAGUGGUTT | 830 | ND-10293 |
| GCUUCGAAGUGCUUGAAAA | 177 | GCUUCGAAGUGCUUGAAAATT | 504 | UUUUCAAGCACUUCGAAGCTT | 831 | ND-10294 |
| UUGUCAAUCUUUAUUCUGA | 178 | UUGUCAAUCUUUAUUCUGATT | 505 | UCAGAAUAAAGAUUGACAATT | 832 | ND-10295 |
| UAGACGUGACCAUAUCAUA | 179 | UAGACGUGACCAUAUCAUATT | 506 | UAUGAUAUGGUCACGUCUATT | 833 | ND-10296 |
| CUGCUUCGAAGUGCUUGAA | 180 | CUGCUUCGAAGUGCUUGAATT | 507 | UUCAAGCACUUCGAAGCAGTT | 834 | ND-10297 |
| CAAGGAUAGGUGAUAGCUC | 181 | CAAGGAUAGGUGAUAGCUCTT | 508 | GAGCUAUCACCUAUCCUUGTT | 835 | ND-10298 |
| UGCACUUGUAUAUUUGUCA | 182 | UGCACUUGUAUAUUUGUCATT | 509 | UGACAAAUAUACAAGUGCATT | 836 | ND-10299 |
| CGUAUCACAAUGUAUACUC | 183 | CGUAUCACAAUGUAUACUCTT | 510 | GAGUAUACAUUGUGAUACGTT | 837 | ND-10300 |

| | | | | | | |
|---|---|---|---|---|---|---|
| CGCAUGUACAGUGAACGAA | 184 | CGCAUGUACAGUGAACGAATT | 511 | UUCGUUCACUGUACAUGCGTT | 838 | ND-10301 |
| AUUAGAAGGGUCUACACCA | 185 | AUUAGAAGGGUCUACACCATT | 512 | UGGUGUAGACCCUUCUAAUTT | 839 | ND-10302 |
| UUUGGCAUGCUGUAAAACA | 186 | UUUGGCAUGCUGUAAAACATT | 513 | UGUUUUACAGCAUGCCAAATT | 840 | ND-10303 |
| CCCUUAAAGUACUUAUUCA | 187 | CCCUUAAAGUACUUAUUCATT | 514 | UGAAUAAGUACUUUAAGGGTT | 841 | ND-10304 |
| ACAAUGUAUACUCUCGAGA | 188 | ACAAUGUAUACUCUCGAGATT | 515 | UCUCGAGAGUAUACAUUGUTT | 842 | ND-10305 |
| UGACGGUGGCUAUACCAGG | 189 | UGACGGUGGCUAUACCAGGTT | 516 | CCUGGUAUAGCCACCGUCATT | 843 | ND-10306 |
| UCGAGAUCCUAAUUAUCUG | 190 | UCGAGAUCCUAAUUAUCUGTT | 517 | CAGAUAAUUAGGAUCUCGATT | 844 | ND-10307 |
| UGAGGGUCAGUUUACUCUG | 191 | UGAGGGUCAGUUUACUCUGTT | 518 | CAGAGUAAACUGACCCUCATT | 845 | ND-10308 |
| AUCUCCCUUAAAGUACUUA | 192 | AUCUCCCUUAAAGUACUUATT | 519 | UAAGUACUUUAAGGGAGAUTT | 846 | ND-10309 |
| GAAUUGCUUAUAUGUGGAA | 193 | GAAUUGCUUAUAUGUGGAATT | 520 | UUCCACAUAUAAGCAAUUCTT | 847 | ND-10310 |
| CUCAAGGAUAGGUGAUAGC | 194 | CUCAAGGAUAGGUGAUAGCTT | 521 | GCUAUCACCUAUCCUUGAGTT | 848 | ND-10311 |
| AAAACAGUUCAAGGCUUUU | 195 | AAAACAGUUCAAGGCUUUUTT | 522 | AAAAGCCUUGAACUGUUUUTT | 849 | ND-10312 |
| AGGGAGACAACAAUUUGCA | 196 | AGGGAGACAACAAUUUGCATT | 523 | UGCAAAUUGUUGUCUCCCUTT | 850 | ND-10313 |
| UGUGACUUGACGUAUCACA | 197 | UGUGACUUGACGUAUCACATT | 524 | UGUGAUACGUCAAGUCACATT | 851 | ND-10314 |
| AAUGUGACUUGACGUAUCA | 198 | AAUGUGACUUGACGUAUCATT | 525 | UGAUACGUCAAGUCACAUUTT | 852 | ND-10315 |
| CAAGGCUUUUCGGAGAGGU | 199 | CAAGGCUUUUCGGAGAGGUTT | 526 | ACCUCUCCGAAAAGCCUUGTT | 853 | ND-10316 |
| UCGCAUGUACAGUGAACGA | 200 | UCGCAUGUACAGUGAACGATT | 527 | UCGUUCACUGUACAUGCGATT | 854 | ND-10317 |
| CUAACGUGGAAUGUGACUU | 201 | CUAACGUGGAAUGUGACUUTT | 528 | AAGUCACAUUCCACGUUAGTT | 855 | ND-10318 |
| CACUUAUCCCUUUUGAAGA | 202 | CACUUAUCCCUUUUGAAGATT | 529 | UCUUCAAAAGGGAUAAGUGTT | 856 | ND-10319 |
| AGGACUAGGAAAAUUAAAG | 203 | AGGACUAGGAAAAUUAAAGTT | 530 | CUUUAAUUUUCCUAGUCCUTT | 857 | ND-10320 |
| AGUGAACGAAGAAUCACUG | 204 | AGUGAACGAAGAAUCACUGTT | 531 | CAGUGAUUCUUCGUUCACUTT | 858 | ND-10321 |
| CCAUAUCAUAGAUGAUGCA | 205 | CCAUAUCAUAGAUGAUGCATT | 532 | UGCAUCAUCUAUGAUAUGGTT | 859 | ND-10322 |
| CAACGGGCACAGACAGAGC | 206 | CAACGGGCACAGACAGAGCTT | 533 | GCUCUGUCUGUGCCCGUUGTT | 860 | ND-10323 |
| ACUUAUCCCUUUUGAAGAG | 207 | ACUUAUCCCUUUUGAAGAGTT | 534 | CUCUUCAAAAGGGAUAAGUTT | 861 | ND-10324 |
| UGGAUAAUAAUGCAGCAGC | 208 | UGGAUAAUAAUGCAGCAGCTT | 535 | GCUGCUGCAUUAUUAUCCATT | 862 | ND-10325 |
| GAAUUUAACAGUCGAAAUC | 209 | GAAUUUAACAGUCGAAAUCTT | 536 | GAUUUCGACUGUUAAAUUCTT | 863 | ND-10326 |
| AGAUUUAUUGGAGUAUGAA | 210 | AGAUUUAUUGGAGUAUGAATT | 537 | UUCAUACUCCAAUAAAUCUTT | 864 | ND-10327 |
| CGAAGAAUCACUGUUCUCU | 211 | CGAAGAAUCACUGUUCUCUTT | 538 | AGAGAACAGUGAUUCUUCGTT | 865 | ND-10328 |
| ACUGAGGGUCAGUUUACUC | 212 | ACUGAGGGUCAGUUUACUCTT | 539 | GAGUAAACUGACCCUCAGUTT | 866 | ND-10329 |
| CAGUUUACAACGGGCACAG | 213 | CAGUUUACAACGGGCACAGTT | 540 | CUGUGCCCGUUGUAAACUGTT | 867 | ND-10330 |
| UGAAUUGCUUAUAUGUGGA | 214 | UGAAUUGCUUAUAUGUGGATT | 541 | UCCACAUAUAAGCAAUUCATT | 868 | ND-10331 |
| ACUCAAAGUUAGACGUGAC | 215 | ACUCAAAGUUAGACGUGACTT | 542 | GUCACGUCUAACUUUGAGUTT | 869 | ND-10332 |

| | | | | | | |
|---|---|---|---|---|---|---|
| GUGACUUGACGUAUCACAA | 216 | GUGACUUGACGUAUCACAATT | 543 | UUGUGAUACGUCAAGUCACTT | 870 | ND-10333 |
| AUGAAUUUAACAGUCGAAA | 217 | AUGAAUUUAACAGUCGAAATT | 544 | UUUCGACUGUUAAAUUCAUTT | 871 | ND-10334 |
| AUAUGACGGUGGCUAUACC | 218 | AUAUGACGGUGGCUAUACCTT | 545 | GGUAUAGCCACCGUCAUAUTT | 872 | ND-10335 |
| AACGAAGAAUCACUGUUCU | 219 | AACGAAGAAUCACUGUUCUTT | 546 | AGAACAGUGAUUCUUCGUUTT | 873 | ND-10336 |
| GCCAUUAGAAGGGUCUACA | 220 | GCCAUUAGAAGGGUCUACATT | 547 | UGUAGACCCUUCUAAUGGCTT | 874 | ND-10337 |
| UUUAUAAGAUUAAUGCAAA | 221 | UUUAUAAGAUUAAUGCAAATT | 548 | UUUGCAUUAAUCUUAUAAATT | 875 | ND-10338 |
| GAAUCCAUAUUUGAGACUC | 222 | GAAUCCAUAUUUGAGACUCTT | 549 | GAGUCUCAAAUAUGGAUUCTT | 876 | ND-10339 |
| UUAUUGGAGUAUGAAGGGA | 223 | UUAUUGGAGUAUGAAGGGATT | 550 | UCCCUUCAUACUCCAAUAATT | 877 | ND-10340 |
| AGCUUCCGGAAAGUUAAAC | 224 | AGCUUCCGGAAAGUUAAACTT | 551 | GUUUAACUUUCCGGAAGCUTT | 878 | ND-10341 |
| UACUCUGAUUGGCAUAGUA | 225 | UACUCUGAUUGGCAUAGUATT | 552 | UACUAUGCCAAUCAGAGUATT | 879 | ND-10342 |
| AGUUUUUUCUAGUGCUGAG | 226 | AGUUUUUUCUAGUGCUGAGTT | 553 | CUCAGCACUAGAAAAAACUTT | 880 | ND-10343 |
| UGUUCACAUACGAUGAAUC | 227 | UGUUCACAUACGAUGAAUCTT | 554 | GAUUCAUCGUAUGUGAACATT | 881 | ND-10344 |
| AAUCUCCCUUAAAGUACUU | 228 | AAUCUCCCUUAAAGUACUUTT | 555 | AAGUACUUUAAGGGAGAUUTT | 882 | ND-10345 |
| GACUCAAAGUUAGACGUGA | 229 | GACUCAAAGUUAGACGUGATT | 556 | UCACGUCUAACUUUGAGUCTT | 883 | ND-10346 |
| AAUUUAACAGUCGAAAUCU | 230 | AAUUUAACAGUCGAAAUCUTT | 557 | AGAUUUCGACUGUUAAAUUTT | 884 | ND-10347 |
| AUGUGCUUUUACUUCCGGA | 231 | AUGUGCUUUUACUUCCGGATT | 558 | UCCGGAAGUAAAAGCACAUTT | 885 | ND-10348 |
| UUGUCACCUAACGUGGAAU | 232 | UUGUCACCUAACGUGGAAUTT | 559 | AUUCCACGUUAGGUGACAATT | 886 | ND-10349 |
| AUGUGACUUGACGUAUCAC | 233 | AUGUGACUUGACGUAUCACTT | 560 | GUGAUACGUCAAGUCACAUTT | 887 | ND-10350 |
| GUGAACGAAGAAUCACUGU | 234 | GUGAACGAAGAAUCACUGUTT | 561 | ACAGUGAUUCUUCGUUCACTT | 888 | ND-10351 |
| GAAUCUAGAUUUCCAAGCA | 235 | GAAUCUAGAUUUCCAAGCATT | 562 | UGCUUGGAAAUCUAGAUUCTT | 889 | ND-10352 |
| GUCAAUCUUUAUUCUGACU | 236 | GUCAAUCUUUAUUCUGACUTT | 563 | AGUCAGAAUAAAGAUUGACTT | 890 | ND-10353 |
| ACACAAAUCACAAUGAAGA | 237 | ACACAAAUCACAAUGAAGATT | 564 | UCUUCAUUGUGAUUUGUGUTT | 891 | ND-10354 |
| GUGACCAAUGAAUCUCCCU | 238 | GUGACCAAUGAAUCUCCCUTT | 565 | AGGGAGAUUCAUUGGUCACTT | 892 | ND-10355 |
| UGAAUCUCCCUUAAAGUAC | 239 | UGAAUCUCCCUUAAAGUACTT | 566 | GUACUUUAAGGGAGAUUCATT | 893 | ND-10356 |
| AUGAACCACUGAAUGAGGU | 240 | AUGAACCACUGAAUGAGGUTT | 567 | ACCUCAUUCAGUGGUUCAUTT | 894 | ND-10357 |
| GCUUCCGGAAAGUUAAACA | 241 | GCUUCCGGAAAGUUAAACATT | 568 | UGUUUAACUUUCCGGAAGCTT | 895 | ND-10358 |
| GAGGGUCAGUUUACUCUGA | 242 | GAGGGUCAGUUUACUCUGATT | 569 | UCAGAGUAAACUGACCCUCTT | 896 | ND-10359 |
| CAUGUACAGUGAACGAAGA | 243 | CAUGUACAGUGAACGAAGATT | 570 | UCUUCGUUCACUGUACAUGTT | 897 | ND-10360 |
| AAGAGUUUUUUCUAGUGCU | 244 | AAGAGUUUUUUCUAGUGCUTT | 571 | AGCACUAGAAAAAACUCUUTT | 898 | ND-10361 |
| CAAUGCAGACCAGAUUCGG | 245 | CAAUGCAGACCAGAUUCGGTT | 572 | CCGAAUCUGGUCUGCAUUGTT | 899 | ND-10362 |
| AUUUCAGCAACUUAUUACU | 246 | AUUUCAGCAACUUAUUACUTT | 573 | AGUAAUAAGUUGCUGAAAUTT | 900 | ND-10363 |
| UAUAUUUGUCACCUAACGU | 247 | UAUAUUUGUCACCUAACGUTT | 574 | ACGUUAGGUGACAAAUAUATT | 901 | ND-10364 |

| | | | | | | |
|---|---|---|---|---|---|---|
| CUGAGAUAAAAAUGAACAA | 248 | CUGAGAUAAAAAUGAACAATT | 575 | UUGUUCAUUUUUAUCUCAGTT | 902 | ND-10365 |
| CCUUAAAGUACUUAUUCAG | 249 | CCUUAAAGUACUUAUUCAGTT | 576 | CUGAAUAAGUACUUUAAGGTT | 903 | ND-10366 |
| GUGACCAUAUCAUAGAUGA | 250 | GUGACCAUAUCAUAGAUGATT | 577 | UCAUCUAUGAUAUGGUCACTT | 904 | ND-10367 |
| GCUUUAAUGUGCUUUUACU | 251 | GCUUUAAUGUGCUUUUACUTT | 578 | AGUAAAAGCACAUUAAAGCTT | 905 | ND-10368 |
| GAAUCUACAAAAUUGUUUU | 252 | GAAUCUACAAAAUUGUUUUTT | 579 | AAAACAAUUUUGUAGAUUCTT | 906 | ND-10369 |
| UUUUCUAGUGCUGAGGCAU | 253 | UUUUCUAGUGCUGAGGCAUTT | 580 | AUGCCUCAGCACUAGAAAATT | 907 | ND-10370 |
| UGACCAAUGAAUCUCCCUU | 254 | UGACCAAUGAAUCUCCCUUTT | 581 | AAGGGAGAUUCAUUGGUCATT | 908 | ND-10371 |
| GGGAAAUCGUUCAUUCAUU | 255 | GGGAAAUCGUUCAUUCAUUTT | 582 | AAUGAAUGAACGAUUUCCCTT | 909 | ND-10372 |
| AUGGAUAAUAAUGCAGCAG | 256 | AUGGAUAAUAAUGCAGCAGTT | 583 | CUGCUGCAUUAUUAUCCAUTT | 910 | ND-10373 |
| GAAGGGUCUACACCAGAUU | 257 | GAAGGGUCUACACCAGAUUTT | 584 | AAUCUGGUGUAGACCCUUCTT | 911 | ND-10374 |
| CAAUGUAUACUCUCGAGAU | 258 | CAAUGUAUACUCUCGAGAUTT | 585 | AUCUCGAGAGUAUACAUUGTT | 912 | ND-10375 |
| AGUGCUGAGGCAUUGGUAC | 259 | AGUGCUGAGGCAUUGGUACTT | 586 | GUACCAAUGCCUCAGCACUTT | 913 | ND-10376 |
| UGUAUACUCUCGAGAUCCU | 260 | UGUAUACUCUCGAGAUCCUTT | 587 | AGGAUCUCGAGAGUAUACATT | 914 | ND-10377 |
| CGAGCUUUAUAAGAUUAAU | 261 | CGAGCUUUAUAAGAUUAAUTT | 588 | AUUAAUCUUAUAAAGCUCGTT | 915 | ND-10378 |
| UAUCACAAUGUAUACUCUC | 262 | UAUCACAAUGUAUACUCUCTT | 589 | GAGAGUAUACAUUGUGAUATT | 916 | ND-10379 |
| CGAAGUGCUUGAAAAUGGU | 263 | CGAAGUGCUUGAAAAUGGUTT | 590 | ACCAUUUUCAAGCACUUCGTT | 917 | ND-10380 |
| GCUGUCACAAAGAAUUUGG | 264 | GCUGUCACAAAGAAUUUGGTT | 591 | CCAAAUUCUUUGUGACAGCTT | 918 | ND-10381 |
| AUCACAAUGUAUACUCUCG | 265 | AUCACAAUGUAUACUCUCGTT | 592 | CGAGAGUAUACAUUGUGAUTT | 919 | ND-10382 |
| AUGUACAGUGAACGAAGAA | 266 | AUGUACAGUGAACGAAGAATT | 593 | UUCUUCGUUCACUGUACAUTT | 920 | ND-10383 |
| CUACAGAAUAUGACGGUGG | 267 | CUACAGAAUAUGACGGUGGTT | 594 | CCACCGUCAUAUUCUGUAGTT | 921 | ND-10384 |
| UUCAGCAACUUAUUACUUA | 268 | UUCAGCAACUUAUUACUUATT | 595 | UAAGUAAUAAGUUGCUGAATT | 922 | ND-10385 |
| AAAGAUGUGACUUACUUAA | 269 | AAAGAUGUGACUUACUUAATT | 596 | UUAAGUAAGUCACAUCUUUTT | 923 | ND-10386 |
| GUGCUGAGGCAUUGGUACA | 270 | GUGCUGAGGCAUUGGUACATT | 597 | UGUACCAAUGCCUCAGCACTT | 924 | ND-10387 |
| AUAUUUGUCACCUAACGUG | 271 | AUAUUUGUCACCUAACGUGTT | 598 | CACGUUAGGUGACAAAUAUTT | 925 | ND-10388 |
| UGGAAGCCGGAAUCUAGAU | 272 | UGGAAGCCGGAAUCUAGAUTT | 599 | AUCUAGAUUCCGGCUUCCATT | 926 | ND-10389 |
| AGAGAUUGUUGAAGGCCAU | 273 | AGAGAUUGUUGAAGGCCAUTT | 600 | AUGGCCUUCAACAAUCUCUTT | 927 | ND-10390 |
| UACAGAAUAUGACGGUGGC | 274 | UACAGAAUAUGACGGUGGCTT | 601 | GCCACCGUCAUAUUCUGUATT | 928 | ND-10391 |
| GGGAGACAACAAUUUGCAA | 275 | GGGAGACAACAAUUUGCAATT | 602 | UUGCAAAUUGUUGUCUCCCTT | 929 | ND-10392 |
| AAUUUGUUCAUUAUCGUAA | 276 | AAUUUGUUCAUUAUCGUAATT | 603 | UUACGAUAAUGAACAAAUUTT | 930 | ND-10393 |
| UGAACAGAAAAGACUCUUC | 277 | UGAACAGAAAAGACUCUUCTT | 604 | GAAGAGUCUUUUCUGUUCATT | 931 | ND-10394 |
| AAUCAGUAGAAAAACAGUU | 278 | AAUCAGUAGAAAAACAGUUTT | 605 | AACUGUUUUUCUACUGAUUTT | 932 | ND-10395 |
| GACUCUUCUUGCAGUUUAC | 279 | GACUCUUCUUGCAGUUUACTT | 606 | GUAAACUGCAAGAAGAGUCTT | 933 | ND-10396 |

| | | | | | | |
|---|---|---|---|---|---|---|
| UAAUGUGCUUUUACUUCCG | 280 | UAAUGUGCUUUUACUUCCGTT | 607 | CGGAAGUAAAAGCACAUUATT | 934 | ND-10397 |
| UUUCAGCAACUUAUUACUU | 281 | UUUCAGCAACUUAUUACUUTT | 608 | AAGUAAUAAGUUGCUGAAATT | 935 | ND-10398 |
| GGAGACAACAAUUUGCAAA | 282 | GGAGACAACAAUUUGCAAATT | 609 | UUUGCAAAUUGUUGUCUCCTT | 936 | ND-10399 |
| CAUAUUUGAGACUCAAAGU | 283 | CAUAUUUGAGACUCAAAGUTT | 610 | ACUUUGAGUCUCAAAUAUGTT | 937 | ND-10400 |
| UCAUAGAUGAUGCACUUGU | 284 | UCAUAGAUGAUGCACUUGUTT | 611 | ACAAGUGCAUCAUCUAUGATT | 938 | ND-10401 |
| CAUAGAUGAUGCACUUGUC | 285 | CAUAGAUGAUGCACUUGUCTT | 612 | GACAAGUGCAUCAUCUAUGTT | 939 | ND-10402 |
| AAACUACAGAAUAUGACGG | 286 | AAACUACAGAAUAUGACGGTT | 613 | CCGUCAUAUUCUGUAGUUUTT | 940 | ND-10403 |
| UGAACCACUGAAUGAGGUU | 287 | UGAACCACUGAAUGAGGUUTT | 614 | AACCUCAUUCAGUGGUUCATT | 941 | ND-10404 |
| GAAAUCUAGUGAAUGAUGA | 288 | GAAAUCUAGUGAAUGAUGATT | 615 | UCAUCAUUCACUAGAUUUCTT | 942 | ND-10405 |
| GAAGCGAGCAGCUGCAAAG | 289 | GAAGCGAGCAGCUGCAAAGTT | 616 | CUUUGCAGCUGCUCGCUUCTT | 943 | ND-10406 |
| AUAUUGAAUGCUGUCACAA | 290 | AUAUUGAAUGCUGUCACAATT | 617 | UUGUGACAGCAUUCAAUAUTT | 944 | ND-10407 |
| UUAAUCCAUCUUCUUUUGA | 291 | UUAAUCCAUCUUCUUUUGATT | 618 | UCAAAAGAAGAUGGAUUAATT | 945 | ND-10408 |
| UGGUUUAAUCCAUCUUCUU | 292 | UGGUUUAAUCCAUCUUCUUTT | 619 | AAGAAGAUGGAUUAAACCATT | 946 | ND-10409 |
| CGAAAUCUAGUGAAUGAUG | 293 | CGAAAUCUAGUGAAUGAUGTT | 620 | CAUCAUUCACUAGAUUUCGTT | 947 | ND-10410 |
| UAUUGAAUGCUGUCACAAA | 294 | UAUUGAAUGCUGUCACAAATT | 621 | UUUGUGACAGCAUUCAAUATT | 948 | ND-10411 |
| UUUGGUUUAAUCCAUCUUC | 295 | UUUGGUUUAAUCCAUCUUCTT | 622 | GAAGAUGGAUUAAACCAAATT | 949 | ND-10412 |
| CCCUUUAUAUUGAAUGCUG | 296 | CCCUUUAUAUUGAAUGCUGTT | 623 | CAGCAUUCAAUAUAAAGGGTT | 950 | ND-10413 |
| UUUUGGUUUAAUCCAUCUU | 297 | UUUUGGUUUAAUCCAUCUUTT | 624 | AAGAUGGAUUAAACCAAAATT | 951 | ND-10414 |
| AGCCGAAUGAAGCGAGCAG | 298 | AGCCGAAUGAAGCGAGCAGTT | 625 | CUGCUCGCUUCAUUCGGCUTT | 952 | ND-10415 |
| UGCGUGAAAGUGUUACAUA | 299 | UGCGUGAAAGUGUUACAUATT | 626 | UAUGUAACACUUUCACGCATT | 953 | ND-10416 |
| GUUACAUAUUCUUUCACUU | 300 | GUUACAUAUUCUUUCACUUTT | 627 | AAGUGAAAGAAUAUGUAACTT | 954 | ND-10417 |
| UUACUGCUUGAGGUUGAGC | 301 | UUACUGCUUGAGGUUGAGCTT | 628 | GCUCAACCUCAAGCAGUAATT | 955 | ND-10418 |
| UGAAGCUAGCCGAAUGAAG | 302 | UGAAGCUAGCCGAAUGAAGTT | 629 | CUUCAUUCGGCUAGCUUCATT | 956 | ND-10419 |
| GGGUUAAAUCACUUUUGCU | 303 | GGGUUAAAUCACUUUUGCUTT | 630 | AGCAAAAGUGAUUUAACCCTT | 957 | ND-10420 |
| AGGGUUAAAUCACUUUUGC | 304 | AGGGUUAAAUCACUUUUGCTT | 631 | GCAAAAGUGAUUUAACCCUTT | 958 | ND-10421 |
| UUAGUAACAGCACAACAAA | 305 | UUAGUAACAGCACAACAAATT | 632 | UUUGUUGUGCUGUUACUAATT | 959 | ND-10422 |
| UAUUACUGCUUGAGGUUGA | 306 | UAUUACUGCUUGAGGUUGATT | 633 | UCAACCUCAAGCAGUAAUATT | 960 | ND-10423 |
| UGUUUCAGUAGCCAAUCCU | 307 | UGUUUCAGUAGCCAAUCCUTT | 634 | AGGAUUGGCUACUGAAACATT | 961 | ND-10424 |
| CAUUGAAGCUAGCCGAAUG | 308 | CAUUGAAGCUAGCCGAAUGTT | 635 | CAUUCGGCUAGCUUCAAUGTT | 962 | ND-10425 |
| GACAUUGAAGCUAGCCGAA | 309 | GACAUUGAAGCUAGCCGAATT | 636 | UUCGGCUAGCUUCAAUGUCTT | 963 | ND-10426 |
| ACAUUGAAGCUAGCCGAAU | 310 | ACAUUGAAGCUAGCCGAAUTT | 637 | AUUCGGCUAGCUUCAAUGUTT | 964 | ND-10427 |
| AAUGAAGCGAGCAGCUGCA | 311 | AAUGAAGCGAGCAGCUGCATT | 638 | UGCAGCUGCUCGCUUCAUUTT | 965 | ND-10428 |
| AGUGUUACAUAUUCUUUCA | 312 | AGUGUUACAUAUUCUUUCATT | 639 | UGAAAGAAUAUGUAACACUTT | 966 | ND-10429 |
| GCUUGAGGUUGAGCCUUUU | 313 | GCUUGAGGUUGAGCCUUUUTT | 640 | AAAAGGCUCAACCUCAAGCTT | 967 | ND-10430 |
| GUCUUGCAAUGAACUGUUU | 314 | GUCUUGCAAUGAACUGUUUTT | 641 | AAACAGUUCAUUGCAAGACTT | 968 | ND-10431 |
| GCCGAAUGAAGCGAGCAGC | 315 | GCCGAAUGAAGCGAGCAGCTT | 642 | GCUGCUCGCUUCAUUCGGCTT | 969 | ND-10432 |
| UUGCGUGAAAGUGUUACAU | 316 | UUGCGUGAAAGUGUUACAUTT | 643 | AUGUAACACUUUCACGCAATT | 970 | ND-10433 |
| UCUUGCAAUGAACUGUUUC | 317 | UCUUGCAAUGAACUGUUUCTT | 644 | GAAACAGUUCAUUGCAAGATT | 971 | ND-10434 |
| AGUAUAUGAAAGGACAGGG | 318 | AGUAUAUGAAAGGACAGGGTT | 645 | CCCUGUCCUUUCAUAUACUTT | 972 | ND-10435 |
| UUGAAGCUAGCCGAAUGAA | 319 | UUGAAGCUAGCCGAAUGAATT | 646 | UUCAUUCGGCUAGCUUCAATT | 973 | ND-10436 |
| AGAACUUUAGUAACAGCAC | 320 | AGAACUUUAGUAACAGCACTT | 647 | GUGCUGUUACUAAAGUUCUTT | 974 | ND-10437 |
| UUAAGGGUUAAAUCACUUU | 321 | UUAAGGGUUAAAUCACUUUTT | 648 | AAAGUGAUUUAACCCUUAATT | 975 | ND-10438 |
| UUGUAUGUACAGAGAGGUU | 322 | UUGUAUGUACAGAGAGGUUTT | 649 | AACCUCUCUGUACAUACAATT | 976 | ND-10439 |
| AAUCACUUUUGCUUGUGUU | 323 | AAUCACUUUUGCUUGUGUUTT | 650 | AACACAAGCAAAAGUGAUUTT | 977 | ND-10440 |
| UACAUAUUCUUUCACUUGU | 324 | UACAUAUUCUUUCACUUGUTT | 651 | ACAAGUGAAAGAAUAUGUATT | 978 | ND-10441 |
| CAGAGAGGUUUUUCUGAAU | 325 | CAGAGAGGUUUUUCUGAAUTT | 652 | AUUCAGAAAAACCUCUCUGTT | 979 | ND-10442 |
| UUUGCUUGUGUUUAUUACU | 326 | UUUGCUUGUGUUUAUUACUTT | 653 | AGUAAUAAACACAAGCAAATT | 980 | ND-10443 |
| GCAAUGAACUGUUUCAGUA | 327 | GCAAUGAACUGUUUCAGUATT | 654 | UACUGAAACAGUUCAUUGCTT | 981 | ND-10444 |

## dsRNA synthesis

### Source of reagents

Where the source of a reagent is not specifically given herein, such reagent may be obtained from any supplier of reagents for molecular biology at a quality/purity standard for application in molecular biology.

siRNA synthesis

Single-stranded RNAs were produced by solid phase synthesis on a scale of 1 μmole using an Expedite 8909 synthesizer (Applied Biosystems, Applera Deutschland GmbH, Darmstadt, Germany) and controlled pore glass (CPG, 500A, Proligo Biochemie GmbH, Hamburg, Germany) as solid support. RNA and RNA containing *2'-O*-methyl nucleotides were generated by solid phase synthesis employing the corresponding phosphoramidites and *2'-O*-methyl phosphoramidites, respectively (Proligo Biochemie GmbH, Hamburg, Germany). These building blocks were incorporated at selected sites within the sequence of the oligoribonucleotide chain using standard nucleoside phosphoramidite chemistry such as described in Current protocols in nucleic acid chemistry, Beaucage, S.L. et al. (Edrs.), John Wiley & Sons, Inc., New York, NY, USA. Phosphorothioate linkages were introduced by replacement of the iodine oxidizer solution with a solution of the Beaucage reagent (Chruachem Ltd, Glasgow, UK) in acetonitrile (1%). Further ancillary reagents were obtained from Mallinckrodt Baker (Griesheim, Germany).

Deprotection and purification of the crude oligoribonucleotides by anion exchange HPLC were carried out according to established procedures. Yields and concentrations were determined by UV absorption of a solution of the respective RNA at a wavelength of 260 nm using a spectral photometer (DU 640B, Beckman Coulter GmbH, Unterschleißheim, Germany). Double stranded RNA was generated by mixing an equimolar solution of complementary strands in annealing buffer (20 mM sodium phosphate, pH 6.8; 100 mM sodium chloride), heated in a water bath at 85 - 90°C for 3 minutes and cooled to room temperature over a period of 3 - 4 hours. The annealed RNA solution was stored at -20 °C until use.

For the synthesis of 3'-cholesterol-conjugated siRNAs (herein referred to as -Chol-3'), an appropriately modified solid support was used for RNA synthesis. The modified solid support was prepared as follows:

Diethyl-2-azabutane-1,4-dicarboxylate **AA**

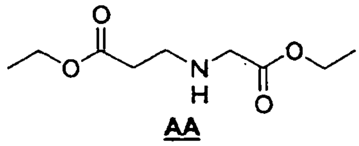

**AA**

A 4.7 M aqueous solution of sodium hydroxide (50 mL) was added into a stirred, ice-cooled solution of ethyl glycinate hydrochloride (32.19 g, 0.23 mole) in water (50 mL). Then, ethyl acrylate (23.1 g, 0.23 mole) was added and the mixture was stirred at room temperature until completion of the reaction was ascertained by TLC. After 19 h the solution was partitioned with dichloromethane (3 x 100 mL). The organic layer was dried with anhydrous sodium sulfate, filtered and evaporated. The residue was distilled to afford AA (28.8 g, 61%).

3- {Ethoxycarbonylmethyl-[6-(9H-fluoren-9-ylmethoxycarbonyl-amino)-hexanoyl]-amino}-propionic acid ethyl ester **AB**

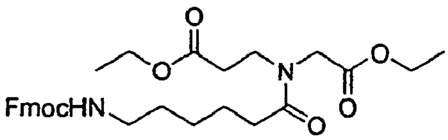

**AB**

Fmoc-6-amino-hexanoic acid (9.12 g, 25.83 mmol) was dissolved in dichloromethane (50 mL) and cooled with ice. Diisopropylcarbodiimde (3.25 g, 3.99 mL, 25.83 mmol) was added to the solution at 0°C. It was then followed by the addition of Diethyl-azabutane-1,4-dicarboxylate (5 g, 24.6 mmol) and dimethylamino pyridine (0.305 g, 2.5 mmol). The solution was brought to room temperature and stirred further for 6 h. Completion of the reaction was ascertained by TLC. The reaction mixture was concentrated under vacuum and ethyl acetate was added to precipitate diisopropyl urea. The suspension was filtered. The filtrate was washed with 5% aqueous hydrochloric acid, 5% sodium bicarbonate and water. The combined organic layer was dried over sodium sulfate and concentrated to give the crude product which was purified by column chromatography (50 % EtOAC/Hexanes) to yield 11.87 g (88%) of AB.

3-[(6-Amino-hexanoyl)-ethoxycarbonylmethyl-amino]-propionic acid ethyl ester **AC**

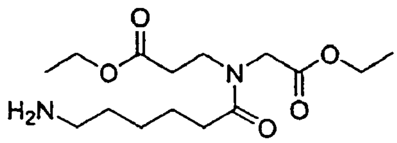

**AC**

3- {Ethoxycarbonylmethyl-[6-(9H-fluoren-9-ylmethoxycarbonylamino)-hexanoyl]-amino}-propionic acid ethyl ester AB (11.5 g, 21.3 mmol) was dissolved in 20% piperidine in dimethylformamide at 0°C. The solution was continued stirring for I h. The reaction mixture was concentrated under vacuum, water was added to the residue, and the product was extracted with ethyl acetate. The crude product was purified by conversion into its hydrochloride salt.

3-({6-[17-(1,5- Dimethyl- hexyl)- 10,13- dimethyl- 2,3,4,7,8,9,10,11,12,13,14,15,16,17- tetradecahydro- 1H- cyclopenta[a]phenanthren-3-yloxycarbonylamino]-hexanoyl}ethoxycarbonylmethyl-amino)-propionic acid ethyl ester **AD**

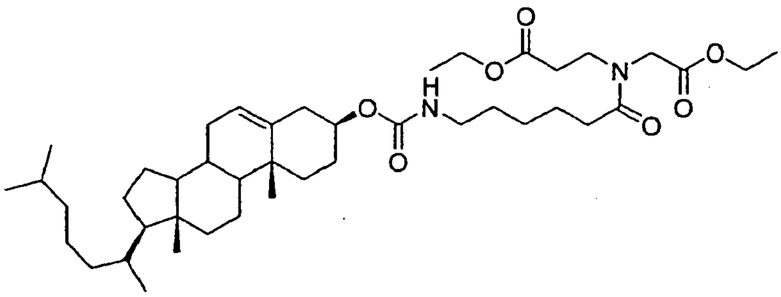

**AD**

The hydrochloride salt of 3-[(6-Amino-hexanoyl)-ethoxycarbonylmethyl-amino]-propionic acid ethyl ester AC (4.7 g, 14.8 mmol) was taken up in dichloromethane. The suspension was cooled to 0°C on ice. To the suspension diisopropylethylamine (3.87 g, 5.2 mL, 30 mmol) was added. To the resulting solution cholesteryl chloroformate (6.675 g, 14.8 mmol) was added. The reaction mixture was stirred overnight. The reaction mixture was diluted with dichloromethane and washed with 10% hydrochloric acid. The product was purified by flash chromatography (10.3 g, 92%).

1-{6-[17-(1,5- Dimethyl- hexyl)- 10,13- dimethyl- 2,3,4,7,8,9,10,11,12,13,14,15,16,17- tetradecahydro- 1H- cyclopenta[a] phenanthren-3-yloxycarbonylamino]-hexanoyl}-4-oxo-pyrrolidine-3-carboxylic acid ethyl ester **AE**

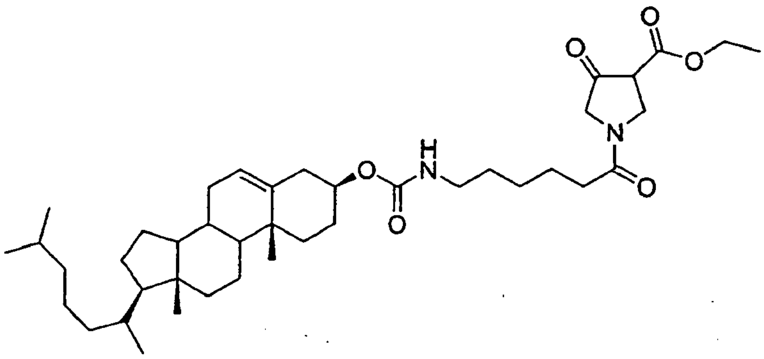

**AE**

Potassium t-butoxide (1.1 g, 9.8 mmol) was slurried in 30 mL of dry toluene. The mixture was cooled to 0°C on ice and 5 g (6.6 mmol) of diester AD was added slowly with stirring within 20 mins. The temperature was kept below 5°C during the addition. The stirring was continued for 30 mins at 0°C and 1 mL of glacial acetic acid was added, immediately followed by 4 g of $NaH_2PO_4 \cdot H_2O$ in 40 mL of water The resultant mixture was extracted twice with 100 mL

of dichloromethane each and the combined organic extracts were washed twice with 10 mL of phosphate buffer each, dried, and evaporated to dryness. The residue was dissolved in 60 mL of toluene, cooled to 0°C and extracted with three 50 mL portions of cold pH 9.5 carbonate buffer. The aqueous extracts were adjusted to pH 3 with phosphoric acid, and extracted with five 40 mL portions of chloroform which were combined, dried and evaporated to dryness. The residue was purified by column chromatography using 25% ethylacetate/hexane to afford 1.9 g of b-ketoester (3 9%).

[6-(3-Hydroxy-4-hydroxymethyl-pyrrolidin-1-yl)-6-oxo-hexyl]-carbamic acid 17-(1,5-dimethyl-hexyl)-10,13-dimethyl-2,3,4,7,8,9,10,11,12,13,14,15,16,17-tetradecahydro-1H-cyclopenta[a]phenanthren-3-yl ester **AF**

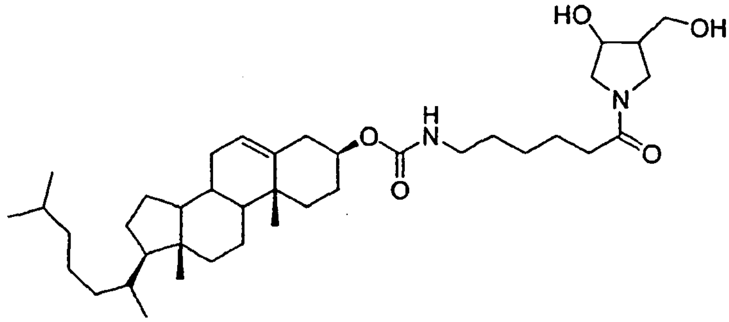

**AF**

Methanol (2 mL) was added dropwise over a period of 1 h to a refluxing mixture of b-ketoester AE (1.5 g, 2.2 mmol) and sodium borohydride (0.226 g, 6 mmol) in tetrahydrofuran (10 mL). Stirring was continued at reflux temperature for 1 h. After cooling to room temperature, 1 N HCl (12.5 mL) was added, the mixture was extracted with ethylacetate (3 x 40 mL). The combined ethylacetate layer was dried over anhydrous sodium sulfate and concentrated under vacuum to yield the product which was purified by column chromatography (10% MeOH/$CHCl_3$) (89%).

(6- {3-[Bis-(4-methoxy-phenyl)-phenyl-methoxymethyl]-4-hydroxy-pyrrolidin-1-yl}-6-oxo-hexyl)-carbamic acid 17-(1,5-dimethyl-hexyl)-10,13-dimethyl-2,3,4,7,8,9,10,11,12,13,14,15,16,17-tetradecahydro-1H-cyclopenta[a]phenanthren-3-yl ester **AG**

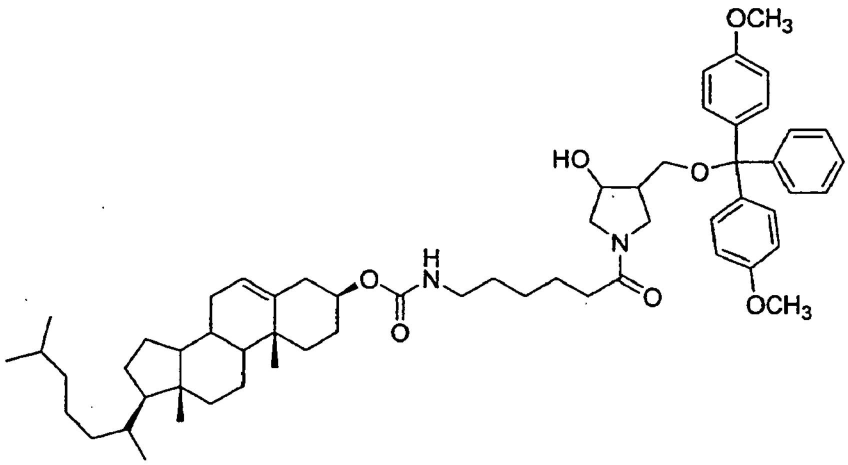

**AG**

Diol AF (1.25 gm 1.994 mmol) was dried by evaporating with pyridine (2 x 5 mL) *in vacuo.* Anhydrous pyridine (10 mL) and 4,4'-dimethoxytritylchloride (0.724 g, 2.13 mmol) were added with stirring. The reaction was carried out at room temperature overnight. The reaction was quenched by the addition of methanol. The reaction mixture was concentrated under vacuum and to the residue dichloromethane (50 mL) was added. The organic layer was washed with 1M aqueous sodium bicarbonate. The organic layer was dried over anhydrous sodium sulfate, filtered and concentrated. The residual pyridine was removed by evaporating with toluene. The crude product was purified by column chromatography (2% MeOH/Chloroform, Rf = 0.5 in 5% MeOH/$CHCl_3$) (1.75 g, 95%).

Succinic acid mono-(4-[bis-(4-methoxy-phenyl)-phenyl-methoxymethyl]-1-{6-[17-(1,5-dimethyl-hexyl)-10,13-dimethyl 2,3,4,7,8,9,10,11,12,13,14,15,16,17-tetradecahydro-1H cyclopenta[a]phenanthren-3-yloxycarbonylamino]-hexanoyl}-pyrrolidin-3-yl) ester **AH**

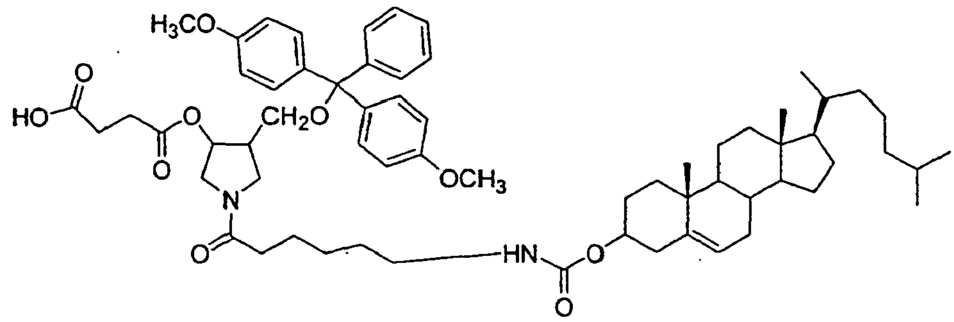

**AH**

CompoundAG (1.0 g, 1.05 mmol) was mixed with succinic anhydride (0.150 g, 1.5 mmol) and DMAP (0.073 g, 0.6 mmol) and dried in a vacuum at 40°C overnight. The mixture was dissolved in anhydrous dichloroethane (3 mL), triethylamine (0.318 g, 0.440 mL, 3.15 mmol) was added and the solution was stirred at room temperature under argon atmosphere for 16 h. It was then diluted with dichloromethane (40 mL) and washed with ice cold aqueous citric acid (5 wt%, 30 mL) and water (2 X 20 mL). The organic phase was dried over anhydrous sodium sulfate and concentrated to dryness. The residue was used as such for the next step.

Cholesterol derivatised CPG **AI**

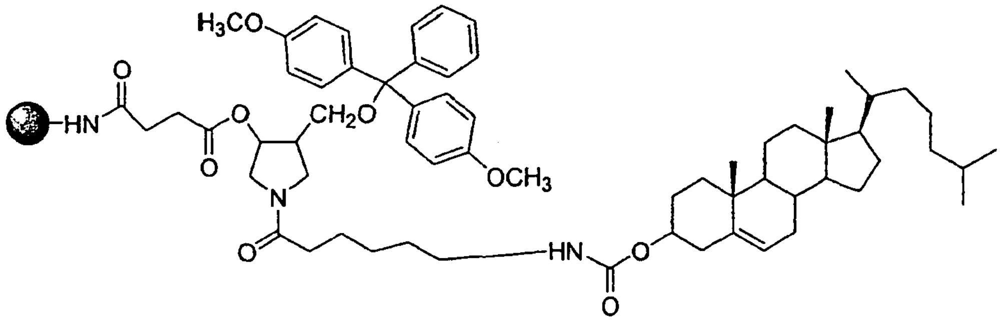

**AI**

Succinate AH (0.254 g, 0.242 mmol) was dissolved in a mixture of dichloromethane/acetonitrile (3:2, 3 mL). To that solution DMAP (0.0296 g, 0.242 mmol) in acetonitrile (1.25 mL), 2,2'-Dithio-bis(5-nitropyridine) (0.075 g, 0.242 mmol) in acetonitrile/dichloroethane (3:1, 1.25 mL) were added successively. To the resulting solution triphenylphosphine (0.064 g, 0.242 mmol) in acetonitrile (0.6 ml) was added. The reaction mixture turned bright orange in color. The solution was agitated briefly using a wrist-action shaker (5 mins). Long chain alkyl amine-CPG (LCAA-CPG) (1.5 g, 61 mM) was added. The suspension was agitated for 2 h. The CPG was filtered through a sintered funnel and washed with acetonitrile, dichloromethane and ether successively. Unreacted amino groups were masked using acetic anhydride/pyridine. The achieved loading of the CPG was measured by taking UV measurement (37 mM/g).

The synthesis of siRNAs bearing a 5'-12-dodecanoic acid bisdecylamide group (herein referred to as "5'-C32-") or a 5'-cholesteryl derivative group (herein referred to as "5'-Chol-") was performed as described in WO 2004/065601, except that, for the cholesteryl derivative, the oxidation step was performed using the Beaucage reagent in order to introduce a phosphorothioate linkage at the 5'-end of the nucleic acid oligomer.

**dsRNA expression vectors**

In another aspect of the invention, E6AP specific dsRNA molecules that modulate E6AP gene expression activity are expressed from transcription units inserted into DNA or RNA vectors (see, e.g., Couture, A, et al., TIG. (1996), 12:5-10; Skillern, A., et al., International PCT Publication No. WO 00/22113, Conrad, International PCT Publication No. WO 00/22114, and Conrad, US Pat. No. 6,054,299). These transgenes can be introduced as a linear construct, a circular plasmid, or a viral vector, which can be incorporated and inherited as a transgene integrated into the host genome. The transgene can also be constructed to permit it to be inherited as an extrachromosomal plasmid (Gassmann, et al., Proc. Natl. Acad. Sci. USA (1995) 92:1292).

The individual strands of a dsRNA can be transcribed by promoters on two separate expression vectors and co-transfected into a target cell. Alternatively each individual strand of the dsRNA can be transcribed by promoters both of which are located on the same expression plasmid. In a preferred embodiment, a dsRNA is expressed as an inverted repeat joined by a linker polynucleotide sequence such that the dsRNA has a stem and loop structure.

The recombinant dsRNA expression vectors are generally DNA plasmids or viral vectors. dsRNA expressing viral vectors can be constructed based on, but not limited to, adeno-associated virus (for a review, see Muzyczka, et al., Curr. Topics Micro. Immunol. (1992) 158:97-129)); adenovirus (see, for example, Berkner, et al., BioTechniques (1998) 6:616), Rosenfeld et al. (1991, Science 252:431-434), and Rosenfeld et al. (1992), Cell 68:143-155)); or alphavirus as well as others known in the art. Retroviruses have been used to introduce a variety of genes into many different cell types, including epithelial cells, in vitro and/or in vivo (see, e.g., Eglitis, et al., Science (1985) 230:1395-1398; Danos and Mulligan, Proc. Natl. Acad. Sci. USA (1998) 85:6460-6464; Wilson et al., 1988, Proc. Natl. Acad. Sci. USA 85: 3014-3018; Armentano et al., 1990, Proc. Natl. Acad. Sci. USA 87:61416145; Huber et al., 1991, Proc. Natl. Acad. Sci. USA 88:8039-8043; Ferry et al., 1991, Proc. Natl. Acad. Sci. USA 88:8377-8381; Chowdhury et al., 1991, Science 254: 1802-1805; van Beusechem. et al., 1992, Proc. Nad. Acad. Sci. USA 89:7640-19 ; Kay et al., 1992, Human Gene Therapy 3:641-647; Dai et al., 1992, Proc. Natl.Acad. Sci. USA 89:10892-10895; Hwu et al., 1993, J. Immunol. 150:4104-4115; U.S. Patent No. 4,868,116; U.S. Patent No. 4,980,286; PCT Application WO 89/07136; PCT Application WO 89/02468; PCT Application WO 89/05345; and PCT Application WO 92/07573). Recombinant retroviral vectors capable of transducing and expressing genes inserted into the genome of a cell can be produced by transfecting the recombinant retroviral genome into suitable packaging cell lines such as PA317 and Psi-CRIP (Comette et al., 1991, Human Gene Therapy 2:5-10; Cone et al., 1984, Proc. Natl. Acad. Sci. USA 81:6349). Recombinant adenoviral vectors can be used to infect a wide variety of cells and tissues in susceptible hosts (e.g., rat, hamster, dog, and chimpanzee) (Hsu et al., 1992, J. Infectious Disease, 166:769), and also have the advantage of not requiring mitotically active cells for infection.

The promoter driving dsRNA expression in either a DNA plasmid or viral vector of the invention may be a eukaryotic RNA polymerase I (e.g. ribosomal RNA promoter), RNA polymerase II (e.g. CMV early promoter or actin promoter or U1 snRNA promoter) or generally RNA polymerase III promoter (e.g. U6 snRNA or 7SK RNA promoter) or a prokaryotic promoter, for example the T7 promoter, provided the expression plasmid also encodes T7 RNA polymerase required for transcription from a T7 promoter. The promoter can also direct transgene expression to the pancreas (see, e.g. the insulin regulatory sequence for pancreas (Bucchini et al., 1986, Proc. Natl. Acad. Sci. USA 83:2511-2515)).

In addition, expression of the transgene can be precisely regulated, for example, by using an inducible regulatory sequence and expression systems such as a regulatory sequence that is sensitive to certain physiological regulators, e.g., circulating glucose levels, or hormones (Docherty et al., 1994, FASEB J. 8:20-24). Such inducible expression systems, suitable for the control of transgene expression in cells or in mammals include regulation by ecdysone, by estrogen, progesterone, tetracycline, chemical inducers of dimerization, and isopropyl-beta-D1 - thiogalactopyranoside (EPTG). A person skilled in the art would be able to choose the appropriate regulatory/promoter sequence based on the intended use of the dsRNA transgene.

Generally, recombinant vectors capable of expressing dsRNA molecules are delivered as described below, and persist in target cells. Alternatively, viral vectors can be used that provide for transient expression of dsRNA molecules. Such vectors can be repeatedly administered as necessary. Once expressed, the dsRNAs bind to target RNA and modulate its function or expression. Delivery of dsRNA expressing vectors can be systemic, such as by intravenous or intramuscular administration, by administration to target cells ex-planted from the patient followed by reintroduction into the patient, or by any other means that allows for introduction into a desired target cell.

dsRNA expression DNA plasmids are typically transfected into target cells as a complex with cationic lipid carriers (e.g. Oligofectamine) or non-cationic lipid-based carriers (e.g. Transit-TKO™). Multiple lipid transfections for dsRNA-mediated knockdowns targeting different regions of a single E6AP gene or multiple E6AP genes over a period of a week or more are also contemplated by the invention. Successful introduction of the vectors of the invention into host cells can be monitored using various known methods. For example, transient transfection. can be signaled with a reporter, such as a fluorescent marker, such as Green Fluorescent Protein (GFP). Stable transfection. of ex vivo cells can be ensured using markers that provide the transfected cell with resistance to specific environmental factors (e.g., antibiotics and drugs), such as hygromycin B resistance.

The E6AP specific dsRNA molecules can also be inserted into vectors and used as gene therapy vectors for human patients. Gene therapy vectors can be delivered to a subject by, for example, intravenous injection, local administration (see U.S. Patent 5,328,470) or by stereotactic injection (see e.g., Chen et al. (1994) Proc. Natl. Acad. Sci. USA 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

## E6AP siRNA screening in HCT-116 cells

HCT-116 cells were obtained from DSMZ (Deutsche Sammlung von Mikroorganismen und Zellkulturen) (Braunschweig, Germany, cat. No. ACC 581) and cultured in McCoys (Biochrom AG, Berlin, Germany, cat. No. F1015) supplemented to contain 10% fetal calf serum (FCS), Penicillin 100 U/ml, Streptomycin 100 μg/ml and 2mM L-Glutamin at 37°C in an atmosphere with 5% $CO_2$ in a humidified incubator.

For transfection with siRNA, HCT-116 cells were seeded at a density of 2.0 x $10^4$ cells/well in 96-well plates and transfected directly. Transfection of siRNA (30nM and 3nM for single dose screen) was carried out with lipofectamine 2000 (Invitrogen) as described by the manufacturer.

24 hours after transfection HCT-116 cells were lysed and E6AP mRNA expression levels were quantified with the Quantigene Explore Kit (Panomics, Inc. (Fremont, CA)(formerly Genospectra, Inc.)) according to the standard protocol. E6AP mRNA levels were normalized to GAP-DH mRNA. For each siRNA four individual datapoints were collected. siRNA duplexes unrelated to E6AP gene were used as control. The activity of a given E6AP specific siRNA duplex was expressed as percent E6AP mRNA concentration in treated cells relative to E6AP mRNA concentration in cells treated with the control siRNA duplex.

Table 2 below provides the results. Many active siRNA molecules that target the E6AP gene were identified.

Table 2 Activity of dsRNA targeting E6AP

| duplex name | mean % of transcript remaining at 30nM | Standard deviation at 30nM | duplex name | mean % of transcript remaining at 30nM | Standard deviation at 30nM |
|---|---|---|---|---|---|
| ND-10118 | **17.66** | 1.99 | ND-10283 | **17.85** | 2.99 |
| ND-10119 | **22.63** | 2.07 | ND-10284 | **27.81** | 3.86 |
| ND-10120 | **25.30** | 2.17 | ND-10285 | **14.01** | 1.66 |
| ND-10121 | **22.16** | 2.12 | ND-10286 | **25.23** | 2.10 |
| ND-10122 | **21.29** | 3.19 | ND-10287 | **6.37** | 0.50 |
| ND-10123 | **14.25** | 2.24 | ND-10288 | **14.58** | 1.39 |
| ND-10124 | **29.18** | 2.04 | ND-10289 | **16.54** | 2.25 |
| ND-10125 | **21.76** | 2.58 | ND-10290 | **12.05** | 2.53 |
| ND-10126 | **34.49** | 2.87 | ND-10291 | **22.07** | 3.30 |
| ND-10127 | **20.06** | 1.59 | ND-10292 | **70.58** | 9.44 |
| ND-10128 | **43.10** | 4.23 | ND-10293 | **14.72** | 2.05 |
| ND-10129 | **21.23** | 3.33 | ND-10294 | **11.42** | 0.42 |
| ND-10130 | **26.72** | 2.38 | ND-10295 | **19.95** | 1.07 |
| ND-10131 | **14.42** | 1.85 | ND-10296 | **14.97** | 0.38 |
| ND-10132 | **22.26** | 4.11 | ND-10297 | **15.64** | 1.55 |
| ND-10133 | **25.69** | 1.98 | ND-10298 | **16.99** | 0.80 |
| ND-10134 | **28.12** | 0.88 | ND-10299 | **18.26** | 2.01 |
| ND-10135 | **38.42** | 3.24 | ND-10300 | **20.01** | 1.31 |
| ND-10136 | **15.54** | 2.55 | ND-10301 | **14.44** | 0.64 |
| ND-10137 | **36.32** | 4.42 | ND-10302 | **41.22** | 3.73 |
| ND-10138 | **36.77** | 5.69 | ND-10303 | **37.74** | 6.03 |
| ND-10139 | **19.09** | 2.75 | ND-10304 | **10.90** | 0.75 |
| ND-10140 | **10.30** | 1.79 | ND-10305 | **14.71** | 3.13 |
| ND-10141 | **48.13** | 12.88 | ND-10306 | **52.68** | 4.91 |

(continued)

| duplex name | mean % of transcript remaining at 30nM | Standard deviation at 30nM | duplex name | mean % of transcript remaining at 30nM | Standard deviation at 30nM |
|---|---|---|---|---|---|
| ND-10142 | **35.73** | 2.65 | ND-10307 | **24.24** | 0.33 |
| ND-10143 | **21.24** | 2.26 | ND-10308 | **54.90** | 3.97 |
| ND-10144 | **17.64** | 3.47 | ND-10309 | **54.76** | 10.28 |
| ND-10145 | **19.02** | 3.28 | ND-10310 | **22.17** | 1.68 |
| ND-10146 | **20.39** | 3.57 | ND-10311 | **24.79** | 2.11 |
| ND-10147 | **20.99** | 1.21 | ND-10312 | **17.33** | 2.88 |
| ND-10148 | **76.62** | 11.31 | ND-10313 | **17.55** | 4.00 |
| ND-10149 | **39.27** | 5.88 | ND-10314 | **23.02** | 4.28 |
| ND-10150 | **26.90** | 1.86 | ND-10315 | **13.33** | 1.88 |
| ND-10151 | **18.88** | 1.08 | ND-10316 | **19.61** | 3.16 |
| ND-10152 | **21.77** | 2.79 | ND-10317 | **17.29** | 1.13 |
| ND-10153 | **15.93** | 2.07 | ND-10318 | **18.78** | 1.73 |
| ND-10154 | **45.64** | 3.75 | ND-10319 | **17.46** | 4.72 |
| ND-10155 | **15.85** | 1.61 | ND-10320 | **19.37** | 2.32 |
| ND-10156 | **17.86** | 2.88 | ND-10321 | **64.52** | 3.68 |
| ND-10157 | **25.83** | 2.64 | ND-10322 | **17.02** | 3.52 |
| ND-10158 | **46.13** | 3.63 | ND-10323 | **47.08** | 4.36 |
| ND-10159 | **12.76** | 1.03 | ND-10324 | **20.06** | 1.65 |
| ND-10160 | **17.45** | 1.40 | ND-10325 | **93.65** | 11.68 |
| ND-10161 | **11.21** | 0.96 | ND-10326 | **18.09** | 3.77 |
| ND-10162 | **42.00** | 5.65 | ND-10327 | **23.52** | 4.09 |
| ND-10163 | **38.02** | 4.20 | ND-10328 | **19.58** | 4.04 |
| ND-10164 | **58.42** | 8.32 | ND-10329 | **71.26** | 4.08 |
| ND-10165 | **18.58** | 2.63 | ND-10330 | **22.76** | 1.92 |
| ND-10166 | **26.03** | 3.03 | ND-10331 | **23.96** | 1.40 |
| ND-10167 | **16.87** | 5.53 | ND-10332 | **31.98** | 2.53 |
| ND-10168 | **18.86** | 1.96 | ND-10333 | **19.35** | 2.76 |
| ND-10169 | **32.31** | 3.92 | ND-10334 | **22.21** | 2.20 |
| ND-10170 | **22.71** | 3.45 | ND-10335 | **58.81** | 6.69 |
| ND-10171 | **12.39** | 3.02 | ND-10336 | **25.38** | 3.14 |
| ND-10172 | **13.53** | 1.72 | ND-10337 | **14.70** | 1.37 |
| ND-10173 | **67.35** | 4.47 | ND-10338 | **30.50** | 3.75 |
| ND-10174 | **13.44** | 1.15 | ND-10339 | **36.81** | 5.22 |
| ND-10175 | **19.24** | 1.89 | ND-10340 | **65.85** | 2.35 |
| ND-10176 | **34.75** | 2.35 | ND-10341 | **57.67** | 2.06 |
| ND-10177 | **27.58** | 3.78 | ND-10342 | **16.25** | 1.61 |
| ND-10178 | **10.76** | 1.32 | ND-10343 | **22.54** | 1.78 |
| ND-10179 | **45.45** | 7.56 | ND-10344 | **17.76** | 1.90 |
| ND-10180 | **88.36** | 11.08 | ND-10345 | **31.65** | 2.34 |
| ND-10181 | **33.41** | 5.36 | ND-10346 | **15.63** | 1.69 |
| ND-10182 | **16.39** | 1.93 | ND-10347 | **29.52** | 2.48 |
| ND-10183 | **14.21** | 1.26 | ND-10348 | **15.28** | 1.01 |
| ND-10184 | **17.63** | 1.94 | ND-10349 | **20.37** | 2.26 |
| ND-10185 | **31.21** | 2.96 | ND-10350 | **36.74** | 4.34 |
| ND-10186 | **15.85** | 1.59 | ND-10351 | **42.98** | 2.06 |
| ND-10187 | **20.72** | 3.28 | ND-10352 | **10.78** | 1.82 |
| ND-10188 | **43.08** | 3.80 | ND-10353 | **18.53** | 1.48 |

(continued)

| duplex name | mean % of transcript remaining at 30nM | Standard deviation at 30nM | duplex name | mean % of transcript remaining at 30nM | Standard deviation at 30nM |
|---|---|---|---|---|---|
| ND-10189 | **18.50** | 2.95 | ND-10354 | **14.95** | 0.66 |
| ND-10190 | **43.59** | 2.94 | ND-10355 | **34.81** | 3.09 |
| ND-10191 | **13.33** | 1.46 | ND-10356 | **86.73** | 4.10 |
| ND-10192 | **14.69** | 2.18 | ND-10357 | **28.86** | 3.35 |
| ND-10193 | **9.86** | 0.91 | ND-10358 | **10.04** | 1.16 |
| ND-10194 | **19.71** | 3.49 | ND-10359 | **9.50** | 0.88 |
| ND-10195 | **11.75** | 1.75 | ND-10360 | **14.63** | 0.18 |
| ND-10196 | **16.32** | 3.59 | ND-10361 | **26.72** | 1.84 |
| ND-10197 | **21.00** | 3.62 | ND-10362 | **24.12** | 1.32 |
| ND-10198 | **24.06** | 3.53 | ND-10363 | **17.32** | 1.54 |
| ND-10199 | **57.92** | 11.64 | ND-10364 | **25.85** | 2.53 |
| ND-10200 | **14.31** | 1.47 | ND-10365 | **15.99** | 2.50 |
| ND-10201 | **42.05** | 2.20 | ND-10366 | **20.32** | 3.27 |
| ND-10202 | **58.23** | 7.77 | ND-10367 | **18.23** | 3.67 |
| ND-10203 | **13.85** | 1.41 | ND-10368 | **11.45** | 1.91 |
| ND-10204 | **16.18** | 1.26 | ND-10369 | **15.69** | 1.49 |
| ND-10205 | **15.76** | 1.71 | ND-10370 | **53.37** | 4.67 |
| ND-10206 | **14.78** | 2.55 | ND-10371 | **24.71** | 2.42 |
| ND-10207 | **17.67** | 2.81 | ND-10372 | **14.08** | 1.42 |
| ND-10208 | **7.50** | 2.37 | ND-10373 | **85.92** | 6.67 |
| ND-10209 | **91.36** | 15.11 | ND-10374 | **17.84** | 1.52 |
| ND-10210 | **27.56** | 2.65 | ND-10375 | **15.51** | 2.28 |
| ND-10211 | **41.43** | 5.64 | ND-10376 | **17.49** | 1.75 |
| ND-10212 | **30.82** | 4.81 | ND-10377 | **15.98** | 2.79 |
| ND-10213 | **12.97** | 1.52 | ND-10378 | **16.99** | 2.71 |
| ND-10214 | **19.53** | 2.31 | ND-10379 | **65.20** | 6.45 |
| ND-10215 | **47.38** | 9.79 | ND-10380 | **12.26** | 1.75 |
| ND-10216 | **18.12** | 2.03 | ND-10381 | **15.82** | 1.59 |
| ND-10217 | **12.51** | 1.97 | ND-10382 | **36.31** | 2.72 |
| ND-10218 | **25.56** | 3.83 | ND-10383 | **15.15** | 1.72 |
| ND-10219 | **81.47** | 13.73 | ND-10384 | **18.34** | 1.31 |
| ND-10220 | **42.93** | 3.74 | ND-10385 | **17.14** | 1.51 |
| ND-10221 | **9.22** | 2.09 | ND-10386 | **14.83** | 1.67 |
| ND-10222 | **13.38** | 1.89 | ND-10387 | **13.41** | 2.42 |
| ND-10223 | **14.84** | 0.96 | ND-10388 | **25.62** | 3.19 |
| ND-10224 | **15.56** | 1.67 | ND-10389 | **21.84** | 1.17 |
| ND-10225 | **35.58** | 3.00 | ND-10390 | **12.71** | 2.10 |
| ND-10226 | **48.38** | 3.47 | ND-10391 | **48.10** | 4.21 |
| ND-10227 | **8.24** | 1.03 | ND-10392 | **14.42** | 1.77 |
| ND-10228 | **48.31** | 3.86 | ND-10393 | **24.34** | 2.27 |
| ND-10229 | **16.51** | 1.79 | ND-10394 | **21.08** | 2.07 |
| ND-10230 | **43.19** | 4.51 | ND-10395 | **16.74** | 3.30 |
| ND-10231 | **41.29** | 3.49 | ND-10396 | **12.06** | 1.13 |
| ND-10232 | **14.21** | 1.34 | ND-10397 | **44.74** | 4.77 |
| ND-10233 | **54.04** | 4.03 | ND-10398 | **60.44** | 7.22 |
| ND-10234 | **19.76** | 1.64 | ND-10399 | **9.71** | 1.59 |
| ND-10235 | **16.66** | 1.24 | ND-10400 | **23.92** | 3.78 |

(continued)

| duplex name | mean % of transcript remaining at 30nM | Standard deviation at 30nM | duplex name | mean % of transcript remaining at 30nM | Standard deviation at 30nM |
|---|---|---|---|---|---|
| ND-10236 | **93.09** | 4.20 | ND-10401 | **22.11** | 2.14 |
| ND-10237 | **12.28** | 0.91 | ND-10402 | **20.63** | 1.38 |
| ND-10238 | **13.66** | 1.20 | ND-10403 | **65.43** | 5.95 |
| ND-10239 | **21.61** | 1.86 | ND-10404 | **27.26** | 2.88 |
| ND-10240 | **55.26** | 3.89 | ND-10405 | **14.97** | 1.44 |
| ND-10241 | **18.24** | 1.87 | ND-10406 | **13.69** | 0.97 |
| ND-10242 | **16.05** | 2.18 | ND-10407 | **27.65** | 1.98 |
| ND-10243 | **33.65** | 4.73 | ND-10408 | **25.92** | 1.30 |
| ND-10244 | **42.58** | 6.33 | ND-10409 | **6.55** | 0.76 |
| ND-10245 | **11.90** | 0.94 | ND-10410 | **17.62** | 2.42 |
| ND-10246 | **19.15** | 2.37 | ND-10411 | **14.78** | 2.72 |
| ND-10247 | **73.91** | 9.75 | ND-10412 | **92.60** | 9.76 |
| ND-10248 | **37.44** | 5.96 | ND-10413 | **13.62** | 0.69 |
| ND-10249 | **37.62** | 4.47 | ND-10414 | **34.45** | 1.77 |
| ND-10250 | **68.05** | 8.23 | ND-10415 | **29.44** | 4.14 |
| ND-10251 | **10.47** | 1.51 | ND-10416 | **14.70** | 1.87 |
| ND-10252 | **89.61** | 8.08 | ND-10417 | **21.80** | 2.69 |
| ND-10253 | **21.85** | 3.32 | ND-10418 | **107.83** | 9.24 |
| ND-10254 | **19.81** | 2.12 | ND-10419 | **36.97** | 5.30 |
| ND-10255 | **7.20** | 0.84 | ND-10420 | **15.00** | 2.31 |
| ND-10256 | **45.03** | 3.65 | ND-10421 | **19.17** | 3.65 |
| ND-10257 | **20.71** | 2.14 | ND-10422 | **23.86** | 2.63 |
| ND-10258 | **30.07** | 2.59 | ND-10423 | **21.55** | 0.35 |
| ND-10259 | **24.85** | 2.94 | ND-10424 | **31.81** | 3.81 |
| ND-10260 | **14.58** | 1.79 | ND-10425 | **27.34** | 2.29 |
| ND-10261 | **32.58** | 3.24 | ND-10426 | **42.72** | 2.80 |
| ND-10262 | **26.63** | 3.78 | ND-10427 | **24.49** | 2.04 |
| ND-10263 | **72.06** | 6.75 | ND-10428 | **24.97** | 1.00 |
| ND-10264 | **93.19** | 12.63 | ND-10429 | **15.47** | 2.36 |
| ND-10265 | **19.70** | 1.96 | ND-10430 | **17.65** | 2.25 |
| ND-10266 | **108.26** | 10.65 | ND-10431 | **20.23** | 3.72 |
| ND-10267 | **14.69** | 1.22 | ND-10432 | **30.69** | 5.52 |
| ND-10268 | **28.16** | 3.50 | ND-10433 | **16.89** | 1.30 |
| ND-10269 | **43.98** | 7.03 | ND-10434 | **44.68** | 4.63 |
| ND-10270 | **13.09** | 1.36 | ND-10435 | **50.13** | 4.22 |
| ND-10271 | **13.92** | 0.99 | ND-10436 | **35.13** | 1.63 |
| ND-10272 | **14.43** | 1.25 | ND-10437 | **48.34** | 3.87 |
| ND-10273 | **17.91** | 1.03 | ND-10438 | **35.36** | 2.60 |
| ND-10274 | **17.81** | 2.72 | ND-10439 | **17.92** | 1.54 |
| ND-10275 | **19.72** | 3.09 | ND-10440 | **21.45** | 2.96 |
| ND-10276 | **8.92** | 1.34 | ND-10441 | **25.77** | 4.43 |
| ND-10277 | **49.34** | 5.11 | ND-10442 | **16.20** | 2.39 |
| ND-10278 | **48.61** | 6.12 | ND-10443 | **27.37** | 1.63 |
| ND-10279 | **16.49** | 1.61 | ND-10444 | **20.49** | 1.49 |
| ND-10280 | **14.14** | 0.91 | | | |
| ND-10281 | **13.55** | 1.86 | | | |
| ND-10282 | **27.29** | 4.14 | | | |

Those skilled in the art are familiar with methods and compositions in addition to those specifically set out in the instant disclosure which will allow them to practice this invention to the full scope of the claims hereinafter appended.

The invention furthermore comprises the following items:

1. A double-stranded ribonucleic acid (dsRNA) for inhibiting the expression of a human E6AP gene in a cell, wherein said dsRNA comprises at least two sequences that are complementary to each other and wherein a sense strand comprises a first sequence and an antisense strand comprises a second sequence comprising a region of complementarity which is substantially complementary to at least a part of a mRNA encoding E6AP, and wherein said region of complementarity is less than 30 nucleotides in length and wherein said dsRNA, upon contact with a cell expressing said E6AP, inhibits expression of said E6AP gene by at least 40%.

2. The dsRNA of item 1, wherein said first sequence is selected from the group consisting of Table 1 and said second sequence is selected from the group consisting of Table 1.

3. The dsRNA of item 1, wherein said dsRNA comprises at least one modified nucleotide.

4. The dsRNA of item 2, wherein said dsRNA comprises at least one modified nucleotide.

5. The dsRNA of items 3 or 4, wherein said modified nucleotide is chosen from the group of: a 2'-O-methyl modified nucleotide, a nucleotide comprising a 5'-phosphorothioate group, and a terminal nucleotide linked to a cholesteryl derivative or dodecanoic acid bisdecylamide group.

6. The dsRNA of items 3 or 4, wherein said modified nucleotide is chosen from the group of: a 2'-deoxy-2'-fluoro modified nucleotide, a 2'-deoxy-modified nucleotide, a locked nucleotide, an abasic nucleotide, 2'-amino-modified nucleotide, 2'-alkyl-modified nucleotide, morpholino nucleotide, a phosphoramidate, and a non-natural base comprising nucleotide.

7. The dsRNA of items 3 or 4, wherein said first sequence is selected from the group consisting of Table 1 and said second sequence is selected from the group consisting of Table 1.

8. The dsRNA of items 6 or 7, wherein said first sequence is selected from the group consisting of Table 1 and said second sequence is selected from the group consisting of Table 1.

9. A cell comprising the dsRNA of item 1.

10. A pharmaceutical composition for inhibiting the expression of the E6AP gene in an organism, comprising a dsRNA and a pharmaceutically acceptable carrier, wherein the dsRNA comprises at least two sequences that are complementary to each other and wherein a sense strand comprises a first sequence and an anti sense strand comprises a second sequence comprising a region of complementarity which is substantially complementary to at least a part of a mRNA encoding E6AP, and wherein said region of complementarity is less than 30 nucleotides in length and wherein said dsRNA, upon contact with a cell expressing said E6AP, inhibits expression of said E6AP gene by at least 20%.

11. The pharmaceutical composition of item 10, wherein said first sequence of said dsRNA is selected from the group consisting of Table 1 and said second sequence of said dsRNA is selected from the group consisting of Table 1.

12. The pharmaceutical composition of item 10, wherein said first sequence of said dsRNA is selected from the group consisting of Table 1 and said second sequence of said dsRNA is selected from the group consisting of Table 1.

13. A method for inhibiting the expression of the E6AP gene in a cell, the method comprising:

(a) introducing into the cell a double-stranded ribonucleic acid (dsRNA), wherein the dsRNA comprises at least two sequences that are complementary to each other and wherein a sense strand comprises a first sequence and an antisense strand comprises a second sequence comprising a region of complementarity which is substantially complementary to at least a part of a mRNA encoding E6AP, and wherein said region of complementarity is less than 30 nucleotides in length and wherein said dsRNA, upon contact with a cell expressing said E6AP, inhibits expression of said E6AP gene by at least 40%; and
(b) maintaining the cell produced in step (a) for a time sufficient to obtain degradation of the mRNA transcript

of the E6AP gene, thereby inhibiting expression of the E6AP gene in the cell.

14. A method of treating, preventing or managing pathological processes mediated by HPV infection comprising administering to a patient in need of such treatment, prevention or management a therapeutically or prophylactically effective amount of a dsRNA, wherein the dsRNA comprises at least two sequences that are complementary to each other and wherein a sense strand comprises a first sequence and an antisense strand comprises a second sequence comprising a region of complementarity which is substantially complementary to at least a part of a mRNA encoding E6AP, and wherein said region of complementarity is less than 30 nucleotides in length and wherein said dsRNA, upon contact with a cell expressing said E6AP, inhibits expression of said E6AP gene by at least 40%.

15. A vector for inhibiting the expression of the E6AP gene in a cell, said vector comprising a regulatory sequence operably linked to a nucleotide sequence that encodes at least one strand of a dsRNA, wherein one of the strands of said dsRNA is substantially complementary to at least a part of a mRNA encoding E6AP and wherein said dsRNA is less than 30 base pairs in length and wherein said dsRNA, upon contact with a cell expressing said E6AP, inhibits the expression of said E6AP gene by at least 40%.

16. A cell comprising the vector of item 16.

17. A double-stranded ribonucleic acid (dsRNA) for reducing the expression level of a human E6AP gene in a cell, wherein said dsRNA comprises at least two sequences that are complementary to each other and wherein a sense strand comprises a first sequence and an antisense strand comprises a second sequence comprising a region of complementarity which is substantially complementary to at least a part of a mRNA encoding E6AP, and wherein said dsRNA, upon contact with a cell expressing said E6AP, reduces the expression level of said E6AP gene.

18. The dsRNA of item 17, wherein said contact reduces the expression level of said E6AP gene by at least 40%.

19. The dsRNA of item 17, wherein said contact is performed in vitro at 30 nM or less.

20. A pharmaceutical composition for reducing the expression level of the E6AP gene in an organism, comprising the dsRNA of item 17 and a pharmaceutically acceptable carrier.

21. A method of treating an HPV associated disorder comprising administering to a patient in need of such treatment, a therapeutically effective amount of a dsRNA of item 17.

22. A method of treating an E6AP-associated disorder comprising administering to a patient in need of such treatment, a therapeutically effective amount of a dsRNA of item 17.

23. A pharmaceutical composition comprising at least two dsRNA selected from among the dsRNA of item 2.

24. A method of treating an HPV associated disorder comprising administering to a patient in need of such treatment a therapeutically effective amount of the pharmaceutical composition of item 23.

## Claims

1. A double-stranded ribonucleic acid (dsRNA), wherein said dsRNA comprises at least two sequences that are complementary to each other and wherein a sense strand comprises a first sequence and an antisense strand comprises a second sequence comprising a region of complementarity which is substantially complementary to at least a part of a mRNA encoding E6AP, and wherein said region of complementarity is less than 30 nucleotides in length and wherein said dsRNA, upon contact with a cell expressing said E6AP, inhibits expression of said E6AP gene by at least 40%.

2. The dsRNA of claim 1, wherein said first sequence is the sequence of SEQ ID NO: 497 and said second sequence is the sequence of SEQ ID NO: 824.

3. The dsRNA of claim 1 or 2, wherein said dsRNA comprises at least one modified nucleotide.

4. The dsRNA of claim 3, wherein said modified nucleotide is chosen from the group of: a 2'-O-methyl-modified nucleotide, a nucleotide comprising a 5'-phosphorothioate group, a terminal nucleotide linked to a cholesteryl derivative or dodecanoic acid bisdecylamide group, a 2'-deoxy-2'-fluoro-modified nucleotide, a 2'-deoxy-modified nucleotide, a locked nucleotide, an abasic nucleotide, 2'-amino-modified nucleotide, 2'-alkyl-modified nucleotide, morpholino nucleotide, a phosphoramidate, and a non-natural base comprising nucleotide.

5. A cell comprising the dsRNA as defined in any one of claims 1 to 4.

6. A pharmaceutical composition comprising a dsRNA and a pharmaceutically acceptable carrier, wherein said dsRNA is as defined in any one of claims 1 to 4.

7. An in vitro method for inhibiting the expression of the E6AP gene in a cell, the method comprising:

   (a) introducing into the cell a double-stranded ribonucleic acid (dsRNA), wherein said dsRNA is as defined in any one of claims 1 to 4; and
   (b) maintaining the cell produced in step (a) for a time sufficient to obtain degradation of the mRNA transcript of the E6AP gene, thereby inhibiting expression of the E6AP gene in the cell.

8. A dsRNA as defined in any one of claims 1 to 4 for use in method of treating, preventing or managing pathological processes mediated by HPV infection.

**9.** A vector, said vector comprising a regulatory sequence operably linked to a nucleotide sequence that encodes at least one strand of a dsRNA as defined in any one of claims 1 to 4.

**10.** A cell comprising the vector of claim 9.

**11.** A pharmaceutical composition comprising at least two dsRNAs as defined in claim 1, wherein said first sequence is selected from the group consisting of Table 1 and said second sequence is selected from the group consisting of Table 1, and wherein one of the dsRNAs is as defined in any one of claims 2 to 4.

**12.** A pharmaceutical composition of claim 11 for use in a method of treating an HPV associated disorder.

Europäisches Patentamt
European Patent Office
Office européen des brevets

# EUROPEAN SEARCH REPORT

Application Number
EP 11 00 9592

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | EP 1 752 536 A1 (RNAI CO LTD [JP] ALPHAGEN CO LTD [JP]) 14 February 2007 (2007-02-14)<br>* paragraph [0042]; claims 1-7,15-18; sequence 231371 *<br>----- | 1,2,5-7,11<br>3,4,8-10,12 | INV.<br>C12N15/113 |
| X<br><br>Y | HENGSTERMANN ARND ET AL: "Growth suppression induced by downregulation of E6-AP expression in human papillomavirus-positive cancer cell lines depends on p53.", JOURNAL OF VIROLOGY JUL 2005, vol. 79, no. 14, July 2005 (2005-07), pages 9296-9300, XP002495831, ISSN: 0022-538X<br>* page 9296 - page 9297; figures 1-5 *<br>----- | 1,5-7,11<br><br>3,4,8-10,12 | |
| X<br><br>Y | SHIRAKURA MASAYUKI ET AL: "E6AP ubiquitin ligase mediates ubiquitylation and degradation of hepatitis C virus core protein.", JOURNAL OF VIROLOGY FEB 2007, vol. 81, no. 3, February 2007 (2007-02), pages 1174-1185, XP002495832, ISSN: 0022-538X<br>* page 1176, left-hand column; figure 4C *<br>----- | 1,5-7,11<br><br>3,4,8-10,12 | TECHNICAL FIELDS SEARCHED (IPC)<br>C12N |

-/--

The present search report has been drawn up for all claims

1

| Place of search | Date of completion of the search | Examiner |
|---|---|---|
| Munich | 6 March 2012 | Deleu, Laurent |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 00 9592

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LIU XUEFENG ET AL: "The E6AP ubiquitin ligase is required for transactivation of the hTERT promoter by the human papillomavirus E6 oncoprotein", JOURNAL OF BIOLOGICAL CHEMISTRY, AMERICAN SOCIETY OF BIOLOCHEMICAL BIOLOGISTS, BIRMINGHAM,; US, vol. 280, no. 11, 1 March 2005 (2005-03-01), pages 10807-10816, XP002462387, ISSN: 0021-9258 | 1,5-7,11 | |
| Y | * figure 5 * | 3,4, 8-10,12 | |
| X | KELLEY MELISSA L ET AL: "The global transcriptional effects of the human papillomavirus E6 protein in cervical carcinoma cell lines are mediated by the E6AP ubiquitin ligase", JOURNAL OF VIROLOGY, THE AMERICAN SOCIETY FOR MICROBIOLOGY, US, vol. 79, no. 6, 1 March 2005 (2005-03-01), pages 3737-3747, XP002462388, ISSN: 0022-538X | 1,5-7,11 | TECHNICAL FIELDS SEARCHED (IPC) |
| Y | * page 3738; figure 1 * | 3,4, 8-10,12 | |
| Y | PATZEL ET AL: "In silico selection of active siRNA", DRUG DISCOVERY TODAY, ELSEVIER, RAHWAY, NJ, US, vol. 12, no. 3-4, 31 January 2007 (2007-01-31), pages 139-148, XP005867980, ISSN: 1359-6446 * the whole document * | 3,4 | |
| | -/-- | | |

The present search report has been drawn up for all claims

1

| Place of search | Date of completion of the search | Examiner |
|---|---|---|
| Munich | 6 March 2012 | Deleu, Laurent |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 00 9592

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GEWIN LINDY ET AL: "Identification of a novel telomerase repressor that interacts with the human papillomavirus type-16 E6/E6-AP complex", GENES & DEVELOPMENT, vol. 18, no. 18, 15 September 2004 (2004-09-15), pages 2269-2282, 2255, XP002495833, ISSN: 0890-9369 * page 2278, left-hand column * ----- | 9,10 | |
| Y | WO 96/34950 A (MITOTIX INC [US]) 7 November 1996 (1996-11-07) * claims 27-35 * ----- | 8,12 | |
| Y | US 2006/275903 A1 (MCSWIGGEN JAMES [US] ET AL) 7 December 2006 (2006-12-07) * paragraph [0011] - paragraph [0032]; claims 1-18 * ----- | 3,4 | |
| X,P | WO 2007/111998 A (NOVARTIS AG [CH]; NOVARTIS PHARMA GMBH [AT]; BENSON JOHN [US]; BRAMLAG) 4 October 2007 (2007-10-04) * claims 1-36; compounds AL-DP-7493; sequences 33,189,345 * ----- | 1-12 | TECHNICAL FIELDS SEARCHED (IPC) |

The present search report has been drawn up for all claims

1

| Place of search | Date of completion of the search | Examiner |
|---|---|---|
| Munich | 6 March 2012 | Deleu, Laurent |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 11 00 9592

This annex lists the patent family members relating to the patent documents cited in the above-mentioned European search report.
The members are as contained in the European Patent Office EDP file on
The European Patent Office is in no way liable for these particulars which are merely given for the purpose of information.

06-03-2012

| Patent document cited in search report | Publication date | Patent family member(s) | Publication date |
|---|---|---|---|
| EP 1752536 A1 | 14-02-2007 | AU 2005248147 A1 | 08-12-2005 |
| | | CA 2566286 A1 | 08-12-2005 |
| | | CN 101052717 A | 10-10-2007 |
| | | EP 1752536 A1 | 14-02-2007 |
| | | KR 20070085113 A | 27-08-2007 |
| | | US 2008113351 A1 | 15-05-2008 |
| | | US 2011054005 A1 | 03-03-2011 |
| | | WO 2005116204 A1 | 08-12-2005 |
| WO 9634950 A | 07-11-1996 | AU 5671596 A | 21-11-1996 |
| | | US 5858987 A | 12-01-1999 |
| | | WO 9634950 A1 | 07-11-1996 |
| US 2006275903 A1 | 07-12-2006 | US 2004192626 A1 | 30-09-2004 |
| | | US 2006275903 A1 | 07-12-2006 |
| | | US 2006281175 A1 | 14-12-2006 |
| | | US 2006292691 A1 | 28-12-2006 |
| | | US 2006293271 A1 | 28-12-2006 |
| | | US 2006293272 A1 | 28-12-2006 |
| | | US 2007004663 A1 | 04-01-2007 |
| | | US 2007004664 A1 | 04-01-2007 |
| | | US 2007004665 A1 | 04-01-2007 |
| | | US 2007004667 A1 | 04-01-2007 |
| | | US 2007167393 A1 | 19-07-2007 |
| WO 2007111998 A | 04-10-2007 | AU 2007230995 A1 | 04-10-2007 |
| | | BR PI0709147 A2 | 28-06-2011 |
| | | CA 2644621 A1 | 04-10-2007 |
| | | EP 1999260 A2 | 10-12-2008 |
| | | JP 2009531433 A | 03-09-2009 |
| | | KR 20080106554 A | 08-12-2008 |
| | | RU 2008141977 A | 27-05-2010 |
| | | US 2009247607 A1 | 01-10-2009 |
| | | US 2011230546 A1 | 22-09-2011 |
| | | WO 2007111998 A2 | 04-10-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9932619 A, Fire
- WO 9953050 A, Waterhouse
- WO 9961631 A, Heifetz
- WO 0044895 A, Limmer
- DE 10100586, Kreutzer
- WO 03008573 A
- US 5138045 A
- US 5218105 A
- US 5212295 A
- US 5378825 A
- US 5541307 A
- US 5386023 A
- US 5457191 A
- US 5459255 A
- US 5521302 A
- US 5539082 A
- US 5554746 A
- US 5571902 A
- US 5578718 A
- US 5587361 A
- US 5599797 A
- US 5506351 A
- US 5587469 A
- US 5587470 A
- US 5223168 A
- US 5608046 A
- US 5602240 A
- US 5610289 A
- US 6262241 B
- US 5459255 B
- WO 9307883 A, Manoharan
- WO 200370918 A
- US 3687808 A
- US 4469863 A
- US 4476301 A
- US 5023243 A
- US 5177196 A
- US 5188897 A
- US 5264423 A
- US 5276019 A
- US 5278302 A
- US 5286717 A
- US 5321131 A
- US 5399676 A
- US 5405939 A
- US 5453496 A
- US 5455233 A
- US 5466677 A
- US 5476925 A
- US 5519126 A
- US 5536821 A
- US 5541306 A
- US 5550111 A
- US 5563253 A
- US 5571799 A
- US 5625050 A
- US 5697248 A
- US 5034506 A
- US 5166315 A
- US 5185444 A
- US 5214134 A
- US 5216141 A
- US 5235033 A
- US 5264562 A
- US 5264564 A
- US 5405938 A
- US 5434257 A
- US 5470967 A
- US 5489677 A
- US 5561225 A
- US 5596086 A
- US 5618704 A
- US 5623070 A
- US 5663312 A
- US 5633360 A
- US 5677437 A
- US 5677439 A
- US 5252479 A
- US 5139941 A
- WO 9413788 A
- WO 9324641 A
- US 6271359 B
- WO 9640964 A
- US 5543417 A
- WO 2004065601 A
- WO 0022113 A, Skillern, A.
- WO 0022114 A, Conrad
- US 6054299 A, Conrad
- US 4868116 A
- US 4980286 A
- WO 8907136 A
- WO 8902468 A
- WO 8905345 A
- WO 9207573 A
- US 5328470 A

**Non-patent literature cited in the description**


- Fields. Virology. Lippincott--Raven Pub, 1996
- **BERNARD, H-U.** *J. Clin. Virol.,* 2005, vol. 328, S1-S6
- **MUNOZ, N. et al.** *N. Engl. J. Med.,* 2003, vol. 348 (6), 518-27
- **BERNARD, H-U.** *J. Clin. Virol.,* 2005, vol. 328, S1-S6
- **YANG, D. et al.** *Curr. Biol.,* 2000, vol. 10, 1191-1200
- **JIANG, M. et al.** *N. A. R.,* 2005, vol. 33 (18), e151
- **CHAKRABARTI, O. ; KRISHNA, S.** *J. Biosci.,* 2003, vol. 28, 337-348
- **SCHEFFNER, M. et al.** *Cell,* 1990, vol. 63, 1129-1136
- **SCHEFFNER, M. et al.** *Cell,* 1993, vol. 75, 495-505
- **LEPIK, D. et al.** *J. Virol.,* 1998, vol. 72, 6822-6831
- **THOMAS, M. et al.** *Oncogene,* 1999, vol. 18, 7690-7700
- Papillomaviridae: The Viruses and Their Replication. **PETER M. HOWLEY.** Fundamental Virology. Lippincott-Raven Publishers, 1996, 947-978
- **ELBASHIR et al.** *EMBO,* 2001, vol. 20, 6877-6888
- Current protocols in nucleic acid chemistry. John Wiley & Sons, Inc,
- **WILLIAMS, D.J. ; K.B. HALL.** *Biochem.,* 1996, vol. 35, 14665-14670
- **WAGNER.** *Nat. Med.,* 1995, vol. 1, 1116-8
- **KOSHKIN, A.A. et al.** *Tetrahedron,* 1998, vol. 54, 3607-3630
- **OBIKA, S. et al.** *Tetrahedron Lett.,* 1998, vol. 39, 5401-5404
- **BRAASCH, D.A. ; D.R COREY.** *Chem. Biol.,* 2001, vol. 8, 1-7
- **M. MANOHARAN.** *Antisense & Nucleic Acid Drug Development,* 2002, vol. 12, 103
- **LI, S. ; DESHMUKH, H. M. ; HUANG, L.** *Pharm. Res.,* 1998, vol. 15, 1540
- **LETSINGER et al.** *Proc. Natl. Acad. Sci. USA,* 1989, vol. 86, 6553
- **MANOHARAN et al.** *Bioorg. Med. Chem. Lett.,* 1994, vol. 4, 1053
- **MANOHARAN et al.** *Ann. N.Y. Acad. Sci.,* 1992, vol. 660, 306
- **MANOHARAN et al.** *Bioorg. Med. Chem. Let.,* 1993, vol. 3, 2765
- **OBERHAUSER et al.** *Nucl. Acids Res.,* 1992, vol. 20, 533
- **SAISON-BEHMOARAS et al.** *EMBO J.,* 1991, vol. 10, 111
- **KABANOV et al.** *FEBS Lett.,* 1990, vol. 259, 327
- **SVINARCHUK et al.** *Biochimie,* 1993, vol. 75, 49
- **MANOHARAN et al.** *Tetrahedron Lett.,* 1995, vol. 36, 3651
- **SHEA et al.** *Nucl. Acids Res.,* 1990, vol. 18, 3777
- **MANOHARAN et al.** *Nucleosides & Nucleotides,* 1995, vol. 14, 969
- **MISHRA et al.** *Biochem. Biophys. Acta,* 1995, vol. 1264, 229
- **CROOKE et al.** *J. Pharmacol. Exp. Ther.,* 1996, vol. 277, 923
- **RABINOWITZ J E et al.** *J Virol,* 2002, vol. 76, 791-801
- **DORNBURG R.** *Gene Therap.,* 1995, vol. 2, 301-310
- **EGLITIS M A.** *Biotechniques,* 1988, vol. 6, 608-614
- **MILLER A D.** *Hum Gene Therap.,* 1990, vol. 1, 5-14
- **ANDERSON W F.** *Nature,* 1998, vol. 392, 25-30
- **RUBINSON D A et al.** *Nat. Genet,* vol. 33, 401-406
- **XIA H et al.** *Nat. Biotech.,* 2002, vol. 20, 1006-1010
- **SAMULSKI R et al.** *J. Virol.,* 1987, vol. 61, 3096-3101
- **FISHER K J et al.** *J. Virol,* 1996, vol. 70, 520-532
- **SAMULSLD R et al.** *J. Virol.,* 1989, vol. 63, 3822-3826
- **TEMPLETON et al.** *Nature Biotechnology,* 1997, vol. 15, 647-652
- **BOLETTA et al.** *J. Am Soc. Nephrol.,* 1996, vol. 7, 1728
- **MORRISSEY, D. et al.** *Nat Biotechnol.,* 2005, vol. 23 (8), 1002-7
- **HENGSTERMANN et al.** *Journal Vir.,* 2005, vol. 79 (14), 9296
- **BUTZ et al.** *Oncogene,* 2003, vol. 22, 5938
- **JEON et al.** *Journal Vir.,* 1995, vol. 69 (5), 2989
- **HITCHINS, M.P. et al.** *Am J Med Genet A.,* 2004, vol. 125 (2), 167-72
- **THOMPSON J.D. et al.** *Nucleic Acids Res.,* 1994, vol. 22, 4673
- **JACKSON AL et al.** *Nat Biotechnol.,* June 2003, vol. 21 (6), 635-7
- **COUTURE, A et al.** *TIG,* 1996, vol. 12, 5-10
- **GASSMANN et al.** *Proc. Natl. Acad. Sci. USA,* 1995, vol. 92, 1292
- **MUZYCZKA et al.** *Curr. Topics Micro. Immunol.,* 1992, vol. 158, 97-129
- **BERKNER et al.** *BioTechniques,* 1998, vol. 6, 616

- **ROSENFELD et al.** *Science,* 1991, vol. 252, 431-434
- **ROSENFELD et al.** *Cell,* 1992, vol. 68, 143-155
- **EGLITIS et al.** *Science,* 1985, vol. 230, 1395-1398
- **DANOS ; MULLIGAN.** *Proc. Natl. Acad. Sci. USA,* 1998, vol. 85, 6460-6464
- **WILSON et al.** *Proc. Natl. Acad. Sci. USA,* 1988, vol. 85, 3014-3018
- **ARMENTANO et al.** *Proc. Natl. Acad. Sci. USA,* 1990, vol. 87, 61416145
- **HUBER et al.** *Proc. Natl. Acad. Sci. USA,* 1991, vol. 88, 8039-8043
- **FERRY et al.** *Proc. Natl. Acad. Sci. USA,* 1991, vol. 88, 8377-8381
- **CHOWDHURY et al.** *Science,* 1991, vol. 254, 1802-1805
- **VAN BEUSECHEM. et al.** *Proc. Nad. Acad. Sci. USA,* 1992, vol. 89, 7640-19
- **KAY et al.** *Human Gene Therapy,* 1992, vol. 3, 641-647
- **DAI et al.** *Proc. Natl.Acad. Sci. USA,* 1992, vol. 89, 10892-10895
- **HWU et al.** *J. Immunol.,* 1993, vol. 150, 4104-4115
- **COMETTE et al.** *Human Gene Therapy,* 1991, vol. 2, 5-10
- **CONE et al.** *Proc. Natl. Acad. Sci. USA,* 1984, vol. 81, 6349
- **HSU et al.** *J. Infectious Disease,* 1992, vol. 166, 769
- **BUCCHINI et al.** *Proc. Natl. Acad. Sci. USA,* 1986, vol. 83, 2511-2515
- **DOCHERTY et al.** *FASEB J.,* 1994, vol. 8, 20-24
- **CHEN et al.** *Proc. Natl. Acad. Sci. USA,* 1994, vol. 91, 3054-3057

SEQUENCE LISTING

<110> ALNYLAM PHARMACEUTICALS, INC.

<120> DSRNA COMPOSITIONS AND METHODS FOR TREATING HPV INFECTIONS

<130> R2665 EP/1

<150> US 60/908,098
<151> 2007-03-26

<160> 981

<170> PatentIn version 3.5

<210> 1
<211> 19
<212> RNA
<213> Homo sapiens

<400> 1
gugauagcuc acagggaga 19

<210> 2
<211> 19
<212> RNA
<213> Homo sapiens

<400> 2
cucagcuuac cuugagaac 19

<210> 3
<211> 19
<212> RNA
<213> Homo sapiens

<400> 3
gagguuuuca uauggugac 19

<210> 4
<211> 19
<212> RNA
<213> Homo sapiens

<400> 4
aauccugcag acuugaaga 19

<210> 5
<211> 19
<212> RNA
<213> Homo sapiens

<400> 5
ggugauagcu cacagggag 19

<210> 6
<211> 19
<212> RNA
<213> Homo sapiens

<400> 6
aaaugguuua cuaugcaaa 19

<210> 7
<211> 19
<212> RNA
<213> Homo sapiens

<400> 7
caguuaacug agggcugug 19

<210> 8
<211> 19
<212> RNA
<213> Homo sapiens

<400> 8
acuacauucu caauaaauc 19

<210> 9
<211> 19
<212> RNA
<213> Homo sapiens

<400> 9
caucccucca agaaaggag 19

<210> 10
<211> 19
<212> RNA
<213> Homo sapiens

<400> 10
gauuagggag uucugggaa 19

<210> 11
<211> 19
<212> RNA
<213> Homo sapiens

<400> 11
ugauagcuca cagggagac 19

<210> 12
<211> 19
<212> RNA
<213> Homo sapiens

<400> 12
uccacagucc ugaauaucu 19

<210> 13
<211> 19
<212> RNA
<213> Homo sapiens

<400> 13
cuccacaguc cugaauauc 19

<210> 14
<211> 19
<212> RNA
<213> Homo sapiens

<400> 14
ugggucuggc uauuuacaa 19

<210> 15
<211> 19
<212> RNA
<213> Homo sapiens

<400> 15
aaaauccugc agacuugaa 19

<210> 16
<211> 19
<212> RNA
<213> Homo sapiens

<400> 16
aaaaugguuu acuaugcaa 19

<210> 17
<211> 19
<212> RNA
<213> Homo sapiens

<400> 17
caaaggauuu ggcaugcug 19

<210> 18
<211> 19
<212> RNA
<213> Homo sapiens

<400> 18
cacgaaugag uuuugugcu 19

<210> 19
<211> 19
<212> RNA
<213> Homo sapiens

<400> 19
cuuuucguga cuugggaga 19

<210> 20
<211> 19
<212> RNA
<213> Homo sapiens

<400> 20
uggucuaaau acaaugcag 19

<210> 21
<211> 19
<212> RNA
<213> Homo sapiens

<400> 21
guggucuaaa uacaaugca 19

<210> 22
<211> 19
<212> RNA
<213> Homo sapiens

<400> 22
aggugauagc ucacaggga 19

<210> 23
<211> 19
<212> RNA
<213> Homo sapiens

<400> 23
ccucgagcuu uauaagauu 19

<210> 24
<211> 19
<212> RNA
<213> Homo sapiens

<400> 24
gagaacucga aaggugccc 19

<210> 25
<211> 19
<212> RNA
<213> Homo sapiens

<400> 25
accaguuaac ugagggcug 19

<210> 26
<211> 19
<212> RNA
<213> Homo sapiens

<400> 26
ccugcacgaa ugaguuuug 19

<210> 27
<211> 19
<212> RNA
<213> Homo sapiens

<400> 27
cccucgagcu uuauaagau 19

<210> 28
<211> 19
<212> RNA
<213> Homo sapiens

<400> 28
cuuaccuuga gaacucgaa 19

<210> 29
<211> 19
<212> RNA
<213> Homo sapiens

<400> 29
cuguggucua aauacaaug 19

<210> 30
<211> 19
<212> RNA
<213> Homo sapiens

<400> 30
ucagacugug gucuaaaua 19

<210> 31
<211> 19
<212> RNA
<213> Homo sapiens

<400> 31
uugagaacuc gaaaggugc 19

<210> 32
<211> 19
<212> RNA
<213> Homo sapiens

<400> 32
agcuuaguuc aaggacagc 19

<210> 33
<211> 19
<212> RNA
<213> Homo sapiens

<400> 33
cagcuuaguu caaggacag 19

<210> 34
<211> 19
<212> RNA
<213> Homo sapiens

<400> 34
gacuuacuua acagaagag 19

<210> 35
<211> 19
<212> RNA
<213> Homo sapiens

<400> 35
uucauauggu gaccaauga 19

<210> 36
<211> 19
<212> RNA
<213> Homo sapiens

<400> 36
cauaugguga ccaaugaau 19

<210> 37

<211> 19
<212> RNA
<213> Homo sapiens

<400> 37
auagaaaucu ccacagucc 19

<210> 38
<211> 19
<212> RNA
<213> Homo sapiens

<400> 38
auucugacua cauucucaa 19

<210> 39
<211> 19
<212> RNA
<213> Homo sapiens

<400> 39
gcacgaauga guuuugugc 19

<210> 40
<211> 19
<212> RNA
<213> Homo sapiens

<400> 40
gcauugguac agagcuucc 19

<210> 41
<211> 19
<212> RNA
<213> Homo sapiens

<400> 41
caccaguuaa cugagggcu 19

<210> 42
<211> 19
<212> RNA
<213> Homo sapiens

<400> 42
gcuuaccuug agaacucga 19

<210> 43
<211> 19
<212> RNA
<213> Homo sapiens

<400> 43
uuaccuugag aacucgaaa 19

<210> 44
<211> 19
<212> RNA
<213> Homo sapiens

<400> 44

gauuuggcau gcuguaaaa 19

<210> 45
<211> 19
<212> RNA
<213> Homo sapiens

<400> 45
ugcuugaaaa ugguuuacu 19

<210> 46
<211> 19
<212> RNA
<213> Homo sapiens

<400> 46
ugcacgaaug aguuuugug 19

<210> 47
<211> 19
<212> RNA
<213> Homo sapiens

<400> 47
uuucgugacu ugggagacu 19

<210> 48
<211> 19
<212> RNA
<213> Homo sapiens

<400> 48
ugaucagacu guggucuaa 19

<210> 49
<211> 19
<212> RNA
<213> Homo sapiens

<400> 49
uucugacuac auucucaau 19

<210> 50
<211> 19
<212> RNA
<213> Homo sapiens

<400> 50
gugcuugaaa augguuuac 19

<210> 51
<211> 19
<212> RNA
<213> Homo sapiens

<400> 51
uuaucugaau uuguucauu 19

<210> 52
<211> 19

<212> RNA
<213> Homo sapiens

<400> 52
aauagaaauc uccacaguc 19

<210> 53
<211> 19
<212> RNA
<213> Homo sapiens

<400> 53
gacugugguc uaaauacaa 19

<210> 54
<211> 19
<212> RNA
<213> Homo sapiens

<400> 54
cuacauucuc aauaaauca 19

<210> 55
<211> 19
<212> RNA
<213> Homo sapiens

<400> 55
agaugugacu uacuuaaca 19

<210> 56
<211> 19
<212> RNA
<213> Homo sapiens

<400> 56
auuuggcaug cuguaaaac 19

<210> 57
<211> 19
<212> RNA
<213> Homo sapiens

<400> 57
guacauuuuc ccaugguug 19

<210> 58
<211> 19
<212> RNA
<213> Homo sapiens

<400> 58
ucguaaugga gaauagaaa 19

<210> 59
<211> 19
<212> RNA
<213> Homo sapiens

<400> 59
cuuaguucaa ggacagcag 19

<210> 60
<211> 19
<212> RNA
<213> Homo sapiens

<400> 60
gucuaaauac aaugcagac 19

<210> 61
<211> 19
<212> RNA
<213> Homo sapiens

<400> 61
uguacauuuu cccaugguu 19

<210> 62
<211> 19
<212> RNA
<213> Homo sapiens

<400> 62
uugguguuaa aacccugga 19

<210> 63
<211> 19
<212> RNA
<213> Homo sapiens

<400> 63
uuuucccaug guugucuac 19

<210> 64
<211> 19
<212> RNA
<213> Homo sapiens

<400> 64
uuuauuaaug aaccacuga 19

<210> 65
<211> 19
<212> RNA
<213> Homo sapiens

<400> 65
gggucuacac cagauugcu 19

<210> 66
<211> 19
<212> RNA
<213> Homo sapiens

<400> 66
cugacuacau ucucaauaa 19

<210> 67
<211> 19
<212> RNA

<213> Homo sapiens

<400> 67
gcccaaggaa aacugauca 19

<210> 68
<211> 19
<212> RNA
<213> Homo sapiens

<400> 68
aucagacugu ggucuaaau 19

<210> 69
<211> 19
<212> RNA
<213> Homo sapiens

<400> 69
gaauucgcau guacaguga 19

<210> 70
<211> 19
<212> RNA
<213> Homo sapiens

<400> 70
gaucgcuaug gaaaauccu 19

<210> 71
<211> 19
<212> RNA
<213> Homo sapiens

<400> 71
aucgcuaugg aaaauccug 19

<210> 72
<211> 19
<212> RNA
<213> Homo sapiens

<400> 72
uuagggaguu cugggaaau 19

<210> 73
<211> 19
<212> RNA
<213> Homo sapiens

<400> 73
gauuauagcc aaaaauggc 19

<210> 74
<211> 19
<212> RNA
<213> Homo sapiens

<400> 74
agaugaucgc uauggaaaa 19

<210> 75
<211> 19
<212> RNA
<213> Homo sapiens

<400> 75
agauaucaca gacagaucu 19

<210> 76
<211> 19
<212> RNA
<213> Homo sapiens

<400> 76
gguauguuca cauacgaug 19

<210> 77
<211> 19
<212> RNA
<213> Homo sapiens

<400> 77
acaauagaau ucgcaugua 19

<210> 78
<211> 19
<212> RNA
<213> Homo sapiens

<400> 78
ggcuagagau gaucgcuau 19

<210> 79
<211> 19
<212> RNA
<213> Homo sapiens

<400> 79
cuguccaacu uuucuucgu 19

<210> 80
<211> 19
<212> RNA
<213> Homo sapiens

<400> 80
augcacuugu ccggcuaga 19

<210> 81
<211> 19
<212> RNA
<213> Homo sapiens

<400> 81
aauccagaua uugguaugu 19

<210> 82
<211> 19
<212> RNA
<213> Homo sapiens

<400> 82
uccggcuaga gaugaucgc 19

<210> 83
<211> 19
<212> RNA
<213> Homo sapiens

<400> 83
gauugucgaa aaccacuua 19

<210> 84
<211> 19
<212> RNA
<213> Homo sapiens

<400> 84
ugaucgcuau ggaaaaucc 19

<210> 85
<211> 19
<212> RNA
<213> Homo sapiens

<400> 85
augaucgcua uggaaaauc 19

<210> 86
<211> 19
<212> RNA
<213> Homo sapiens

<400> 86
cuuauaugug gaagccgga 19

<210> 87
<211> 19
<212> RNA
<213> Homo sapiens

<400> 87
gauaucacag acagaucuu 19

<210> 88
<211> 19
<212> RNA
<213> Homo sapiens

<400> 88
acuuauucag accagaaga 19

<210> 89
<211> 19
<212> RNA
<213> Homo sapiens

<400> 89
uguccaacuu uucuucgua 19

<210> 90
<211> 19
<212> RNA
<213> Homo sapiens

<400> 90
uuauaugugg aagccggaa 19

<210> 91
<211> 19
<212> RNA
<213> Homo sapiens

<400> 91
agcaguugaa uccauauuu 19

<210> 92
<211> 19
<212> RNA
<213> Homo sapiens

<400> 92
ugcaaaugua gugggaggg 19

<210> 93
<211> 19
<212> RNA
<213> Homo sapiens

<400> 93
gaugcacuug uccggcuag 19

<210> 94
<211> 19
<212> RNA
<213> Homo sapiens

<400> 94
gaugaugcac uuguccggc 19

<210> 95
<211> 19
<212> RNA
<213> Homo sapiens

<400> 95
acuuguccgg cuagagaug 19

<210> 96
<211> 19
<212> RNA
<213> Homo sapiens

<400> 96
aagguuaccu acaucucau 19

<210> 97
<211> 19
<212> RNA
<213> Homo sapiens

<400> 97
agaugauuau agccaaaaa 19

<210> 98
<211> 19
<212> RNA
<213> Homo sapiens

<400> 98
gacuugggag acucucacc 19

<210> 99
<211> 19
<212> RNA
<213> Homo sapiens

<400> 99
acuuuucuuc guauggaua 19

<210> 100
<211> 19
<212> RNA
<213> Homo sapiens

<400> 100
aaagguuacc uacaucuca 19

<210> 101
<211> 19
<212> RNA
<213> Homo sapiens

<400> 101
gacaauagaa uucgcaugu 19

<210> 102
<211> 19
<212> RNA
<213> Homo sapiens

<400> 102
uuuacaauaa cuguauacu 19

<210> 103
<211> 19
<212> RNA
<213> Homo sapiens

<400> 103
uuacucugau uggcauagu 19

<210> 104
<211> 19
<212> RNA
<213> Homo sapiens

<400> 104
cuggcuauuu acaauaacu 19

<210> 105

<211> 19
<212> RNA
<213> Homo sapiens

<400> 105
guauguucac auacgauga 19

<210> 106
<211> 19
<212> RNA
<213> Homo sapiens

<400> 106
agucauaagc aaugaauuu 19

<210> 107
<211> 19
<212> RNA
<213> Homo sapiens

<400> 107
guugaaucca uauuugaga 19

<210> 108
<211> 19
<212> RNA
<213> Homo sapiens

<400> 108
uuaaugcaaa acucuguga 19

<210> 109
<211> 19
<212> RNA
<213> Homo sapiens

<400> 109
ugcacuuguc cggcuagag 19

<210> 110
<211> 19
<212> RNA
<213> Homo sapiens

<400> 110
cccaaugaug uaugaucua 19

<210> 111
<211> 19
<212> RNA
<213> Homo sapiens

<400> 111
uacuuauuca gaccagaag 19

<210> 112
<211> 19
<212> RNA
<213> Homo sapiens

<400> 112

auauugguau guucacaua 19

<210> 113
<211> 19
<212> RNA
<213> Homo sapiens

<400> 113
aaacucugug auccucauc 19

<210> 114
<211> 19
<212> RNA
<213> Homo sapiens

<400> 114
uuccagauau cacagacag 19

<210> 115
<211> 19
<212> RNA
<213> Homo sapiens

<400> 115
gaucacuuuc cagauauca 19

<210> 116
<211> 19
<212> RNA
<213> Homo sapiens

<400> 116
aucacuuucc agauaucac 19

<210> 117
<211> 19
<212> RNA
<213> Homo sapiens

<400> 117
cacuuuccag auaucacag 19

<210> 118
<211> 19
<212> RNA
<213> Homo sapiens

<400> 118
ccagacacag aaagguuac 19

<210> 119
<211> 19
<212> RNA
<213> Homo sapiens

<400> 119
aauaacugua uacuggaug 19

<210> 120
<211> 19

<212> RNA
<213> Homo sapiens

<400> 120
ucacuuucca gauaucaca 19

<210> 121
<211> 19
<212> RNA
<213> Homo sapiens

<400> 121
uuuaugacau gucccuuua 19

<210> 122
<211> 19
<212> RNA
<213> Homo sapiens

<400> 122
agacacagaa agguuaccu 19

<210> 123
<211> 19
<212> RNA
<213> Homo sapiens

<400> 123
ccaaaggauu uggcaugcu 19

<210> 124
<211> 19
<212> RNA
<213> Homo sapiens

<400> 124
ccuacaucuc auacuugcu 19

<210> 125
<211> 19
<212> RNA
<213> Homo sapiens

<400> 125
cccagacaca gaaagguua 19

<210> 126
<211> 19
<212> RNA
<213> Homo sapiens

<400> 126
auaaaauucc aauuacaaa 19

<210> 127
<211> 19
<212> RNA
<213> Homo sapiens

<400> 127
auuaucguaa uggagaaua 19

<210> 128
<211> 19
<212> RNA
<213> Homo sapiens

<400> 128
aguuagacgu gaccauauc 19

<210> 129
<211> 19
<212> RNA
<213> Homo sapiens

<400> 129
aucguaaugg agaauagaa 19

<210> 130
<211> 19
<212> RNA
<213> Homo sapiens

<400> 130
uucguaugga uaauaaugc 19

<210> 131
<211> 19
<212> RNA
<213> Homo sapiens

<400> 131
acguaucaca auguauacu 19

<210> 132
<211> 19
<212> RNA
<213> Homo sapiens

<400> 132
caucacguau gccaaagga 19

<210> 133
<211> 19
<212> RNA
<213> Homo sapiens

<400> 133
ugucgaaaac cacuuaucc 19

<210> 134
<211> 19
<212> RNA
<213> Homo sapiens

<400> 134
acgaugaauc uacaaaauu 19

<210> 135
<211> 19
<212> RNA

<213> Homo sapiens

<400> 135
uuacaacggg cacagacag 19

<210> 136
<211> 19
<212> RNA
<213> Homo sapiens

<400> 136
gaaaucguuc auucauuua 19

<210> 137
<211> 19
<212> RNA
<213> Homo sapiens

<400> 137
ucuucguaug gauaauaau 19

<210> 138
<211> 19
<212> RNA
<213> Homo sapiens

<400> 138
cacauacgau gaaucuaca 19

<210> 139
<211> 19
<212> RNA
<213> Homo sapiens

<400> 139
ucgaagugcu ugaaaaugg 19

<210> 140
<211> 19
<212> RNA
<213> Homo sapiens

<400> 140
gucgaaaacc acuuauccc 19

<210> 141
<211> 19
<212> RNA
<213> Homo sapiens

<400> 141
ucacauacga ugaaucuac 19

<210> 142
<211> 19
<212> RNA
<213> Homo sapiens

<400> 142
acgaagaauc acuguucuc 19

<210> 143
<211> 19
<212> RNA
<213> Homo sapiens

<400> 143
acucucgaga uccuaauua 19

<210> 144
<211> 19
<212> RNA
<213> Homo sapiens

<400> 144
uguauacugg auguacauu 19

<210> 145
<211> 19
<212> RNA
<213> Homo sapiens

<400> 145
ugaccauauc auagaugau 19

<210> 146
<211> 19
<212> RNA
<213> Homo sapiens

<400> 146
ugcuuauaug uggaagccg 19

<210> 147
<211> 19
<212> RNA
<213> Homo sapiens

<400> 147
uauaccaggg acucuguuc 19

<210> 148
<211> 19
<212> RNA
<213> Homo sapiens

<400> 148
guacuuauuc agaccagaa 19

<210> 149
<211> 19
<212> RNA
<213> Homo sapiens

<400> 149
auuggcauag uacuggguc 19

<210> 150
<211> 19
<212> RNA
<213> Homo sapiens

<400> 150
cuguauacug gauguacau 19

<210> 151
<211> 19
<212> RNA
<213> Homo sapiens

<400> 151
uuuucuucgu auggauaau 19

<210> 152
<211> 19
<212> RNA
<213> Homo sapiens

<400> 152
ucggagaggu uuucauaug 19

<210> 153
<211> 19
<212> RNA
<213> Homo sapiens

<400> 153
gggucaguuu acucugauu 19

<210> 154
<211> 19
<212> RNA
<213> Homo sapiens

<400> 154
ggucaguuua cucugauug 19

<210> 155
<211> 19
<212> RNA
<213> Homo sapiens

<400> 155
gaaucucccu uaaaguacu 19

<210> 156
<211> 19
<212> RNA
<213> Homo sapiens

<400> 156
ccaaugaauc ucccuuaaa 19

<210> 157
<211> 19
<212> RNA
<213> Homo sapiens

<400> 157
gacgugacca uaucauaga 19

<210> 158
<211> 19
<212> RNA
<213> Homo sapiens

<400> 158
acgugaccau aucauagau 19

<210> 159
<211> 19
<212> RNA
<213> Homo sapiens

<400> 159
guucacauac gaugaaucu 19

<210> 160
<211> 19
<212> RNA
<213> Homo sapiens

<400> 160
uuuaacaguc gaaaucuag 19

<210> 161
<211> 19
<212> RNA
<213> Homo sapiens

<400> 161
gacgguggcu auaccaggg 19

<210> 162
<211> 19
<212> RNA
<213> Homo sapiens

<400> 162
ucacaaugua uacucucga 19

<210> 163
<211> 19
<212> RNA
<213> Homo sapiens

<400> 163
auuuaacagu cgaaaucua 19

<210> 164
<211> 19
<212> RNA
<213> Homo sapiens

<400> 164
ccuggauugu cgaaaacca 19

<210> 165
<211> 19
<212> RNA
<213> Homo sapiens

<400> 165
aagaugugac uuacuuaac 19

<210> 166
<211> 19
<212> RNA
<213> Homo sapiens

<400> 166
cagaccagau ucggagaau 19

<210> 167
<211> 19
<212> RNA
<213> Homo sapiens

<400> 167
cuucguaugg auaauaaug 19

<210> 168
<211> 19
<212> RNA
<213> Homo sapiens

<400> 168
gacguaucac aauguauac 19

<210> 169
<211> 19
<212> RNA
<213> Homo sapiens

<400> 169
ucguauggau aauaaugca 19

<210> 170
<211> 19
<212> RNA
<213> Homo sapiens

<400> 170
cauacgauga aucuacaaa 19

<210> 171
<211> 19
<212> RNA
<213> Homo sapiens

<400> 171
gacuugacgu aucacaaug 19

<210> 172
<211> 19
<212> RNA
<213> Homo sapiens

<400> 172
gcuuuucgga gagguuuuc 19

<210> 173

<211> 19
<212> RNA
<213> Homo sapiens

<400> 173
ggaaaucguu cauucauuu 19

<210> 174
<211> 19
<212> RNA
<213> Homo sapiens

<400> 174
ucgaaaacca cuuaucccu 19

<210> 175
<211> 19
<212> RNA
<213> Homo sapiens

<400> 175
ugucaaucuu uauucugac 19

<210> 176
<211> 19
<212> RNA
<213> Homo sapiens

<400> 176
accacuuauc ccuuuugaa 19

<210> 177
<211> 19
<212> RNA
<213> Homo sapiens

<400> 177
gcuucgaagu gcuugaaaa 19

<210> 178
<211> 19
<212> RNA
<213> Homo sapiens

<400> 178
uugucaaucu uuauucuga 19

<210> 179
<211> 19
<212> RNA
<213> Homo sapiens

<400> 179
uagacgugac cauaucaua 19

<210> 180
<211> 19
<212> RNA
<213> Homo sapiens

<400> 180

cugcuucgaa gugcuugaa 19

<210> 181
<211> 19
<212> RNA
<213> Homo sapiens

<400> 181
caaggauagg ugauagcuc 19

<210> 182
<211> 19
<212> RNA
<213> Homo sapiens

<400> 182
ugcacuugua uauuuguca 19

<210> 183
<211> 19
<212> RNA
<213> Homo sapiens

<400> 183
cguaucacaa uguauacuc 19

<210> 184
<211> 19
<212> RNA
<213> Homo sapiens

<400> 184
cgcauguaca gugaacgaa 19

<210> 185
<211> 19
<212> RNA
<213> Homo sapiens

<400> 185
auuagaaggg ucuacacca 19

<210> 186
<211> 19
<212> RNA
<213> Homo sapiens

<400> 186
uuuggcaugc uguaaaaca 19

<210> 187
<211> 19
<212> RNA
<213> Homo sapiens

<400> 187
cccuuaaagu acuuauuca 19

<210> 188
<211> 19

<212> RNA
<213> Homo sapiens

<400> 188
acaauguaua cucucgaga 19

<210> 189
<211> 19
<212> RNA
<213> Homo sapiens

<400> 189
ugacgguggc uauaccagg 19

<210> 190
<211> 19
<212> RNA
<213> Homo sapiens

<400> 190
ucgagauccu aauuaucug 19

<210> 191
<211> 19
<212> RNA
<213> Homo sapiens

<400> 191
ugagggucag uuuacucug 19

<210> 192
<211> 19
<212> RNA
<213> Homo sapiens

<400> 192
aucucccuua aaguacuua 19

<210> 193
<211> 19
<212> RNA
<213> Homo sapiens

<400> 193
gaauugcuua uauguggaa 19

<210> 194
<211> 19
<212> RNA
<213> Homo sapiens

<400> 194
cucaaggaua ggugauagc 19

<210> 195
<211> 19
<212> RNA
<213> Homo sapiens

<400> 195
aaaacaguuc aaggcuuuu 19

<210> 196
<211> 19
<212> RNA
<213> Homo sapiens

<400> 196
agggagacaa caauuugca 19

<210> 197
<211> 19
<212> RNA
<213> Homo sapiens

<400> 197
ugugacuuga cguaucaca 19

<210> 198
<211> 19
<212> RNA
<213> Homo sapiens

<400> 198
aaugugacuu gacguauca 19

<210> 199
<211> 19
<212> RNA
<213> Homo sapiens

<400> 199
caaggcuuuu cggagaggu 19

<210> 200
<211> 19
<212> RNA
<213> Homo sapiens

<400> 200
ucgcauguac agugaacga 19

<210> 201
<211> 19
<212> RNA
<213> Homo sapiens

<400> 201
cuaacgugga augugacuu 19

<210> 202
<211> 19
<212> RNA
<213> Homo sapiens

<400> 202
cacuuauccc uuuugaaga 19

<210> 203
<211> 19
<212> RNA

<213> Homo sapiens

<400> 203
aggacuagga aaauuaaag 19

<210> 204
<211> 19
<212> RNA
<213> Homo sapiens

<400> 204
agugaacgaa gaaucacug 19

<210> 205
<211> 19
<212> RNA
<213> Homo sapiens

<400> 205
ccauaucaua gaugaugca 19

<210> 206
<211> 19
<212> RNA
<213> Homo sapiens

<400> 206
caacgggcac agacagagc 19

<210> 207
<211> 19
<212> RNA
<213> Homo sapiens

<400> 207
acuuaucccu uuugaagag 19

<210> 208
<211> 19
<212> RNA
<213> Homo sapiens

<400> 208
uggauaauaa ugcagcagc 19

<210> 209
<211> 19
<212> RNA
<213> Homo sapiens

<400> 209
gaauuuaaca gucgaaauc 19

<210> 210
<211> 19
<212> RNA
<213> Homo sapiens

<400> 210
agauuuauug gaguaugaa 19

<210> 211
<211> 19
<212> RNA
<213> Homo sapiens

<400> 211
cgaagaauca cuguucucu 19

<210> 212
<211> 19
<212> RNA
<213> Homo sapiens

<400> 212
acugaggguc aguuuacuc 19

<210> 213
<211> 19
<212> RNA
<213> Homo sapiens

<400> 213
caguuuacaa cgggcacag 19

<210> 214
<211> 19
<212> RNA
<213> Homo sapiens

<400> 214
ugaauugcuu auaugugga 19

<210> 215
<211> 19
<212> RNA
<213> Homo sapiens

<400> 215
acucaaaguu agacgugac 19

<210> 216
<211> 19
<212> RNA
<213> Homo sapiens

<400> 216
gugacuugac guaucacaa 19

<210> 217
<211> 19
<212> RNA
<213> Homo sapiens

<400> 217
augaauuuaa cagucgaaa 19

<210> 218
<211> 19
<212> RNA
<213> Homo sapiens

<400> 218
auaugacggu ggcuauacc 19

<210> 219
<211> 19
<212> RNA
<213> Homo sapiens

<400> 219
aacgaagaau cacuguucu 19

<210> 220
<211> 19
<212> RNA
<213> Homo sapiens

<400> 220
gccauuagaa gggucuaca 19

<210> 221
<211> 19
<212> RNA
<213> Homo sapiens

<400> 221
uuuauaagau uaaugcaaa 19

<210> 222
<211> 19
<212> RNA
<213> Homo sapiens

<400> 222
gaauccauau uugagacuc 19

<210> 223
<211> 19
<212> RNA
<213> Homo sapiens

<400> 223
uuauuggagu augaaggga 19

<210> 224
<211> 19
<212> RNA
<213> Homo sapiens

<400> 224
agcuuccgga aaguuaaac 19

<210> 225
<211> 19
<212> RNA
<213> Homo sapiens

<400> 225
uacucugauu ggcauagua 19

<210> 226
<211> 19
<212> RNA
<213> Homo sapiens

<400> 226
aguuuuuucu agugcugag 19

<210> 227
<211> 19
<212> RNA
<213> Homo sapiens

<400> 227
uguucacaua cgaugaauc 19

<210> 228
<211> 19
<212> RNA
<213> Homo sapiens

<400> 228
aaucucccuu aaaguacuu 19

<210> 229
<211> 19
<212> RNA
<213> Homo sapiens

<400> 229
gacucaaagu uagacguga 19

<210> 230
<211> 19
<212> RNA
<213> Homo sapiens

<400> 230
aauuuaacag ucgaaaucu 19

<210> 231
<211> 19
<212> RNA
<213> Homo sapiens

<400> 231
augugcuuuu acuuccgga 19

<210> 232
<211> 19
<212> RNA
<213> Homo sapiens

<400> 232
uugucaccua acguggaau 19

<210> 233
<211> 19
<212> RNA
<213> Homo sapiens

<400> 233
augugacuug acguaucac 19

<210> 234
<211> 19
<212> RNA
<213> Homo sapiens

<400> 234
gugaacgaag aaucacugu 19

<210> 235
<211> 19
<212> RNA
<213> Homo sapiens

<400> 235
gaaucuagau uuccaagca 19

<210> 236
<211> 19
<212> RNA
<213> Homo sapiens

<400> 236
gucaaucuuu auucugacu 19

<210> 237
<211> 19
<212> RNA
<213> Homo sapiens

<400> 237
acacaaauca caaugaaga 19

<210> 238
<211> 19
<212> RNA
<213> Homo sapiens

<400> 238
gugaccaaug aaucucccu 19

<210> 239
<211> 19
<212> RNA
<213> Homo sapiens

<400> 239
ugaaucuccc uuaaaguac 19

<210> 240
<211> 19
<212> RNA
<213> Homo sapiens

<400> 240
augaaccacu gaaugaggu 19

<210> 241

<211> 19
<212> RNA
<213> Homo sapiens

<400> 241
gcuuccggaa aguuaaaca 19

<210> 242
<211> 19
<212> RNA
<213> Homo sapiens

<400> 242
gagggucagu uuacucuga 19

<210> 243
<211> 19
<212> RNA
<213> Homo sapiens

<400> 243
cauguacagu gaacgaaga 19

<210> 244
<211> 19
<212> RNA
<213> Homo sapiens

<400> 244
aagaguuuuu ucuagugcu 19

<210> 245
<211> 19
<212> RNA
<213> Homo sapiens

<400> 245
caaugcagac cagauucgg 19

<210> 246
<211> 19
<212> RNA
<213> Homo sapiens

<400> 246
auuucagcaa cuuauuacu 19

<210> 247
<211> 19
<212> RNA
<213> Homo sapiens

<400> 247
uauauuuguc accuaacgu 19

<210> 248
<211> 19
<212> RNA
<213> Homo sapiens

<400> 248

cugagauaaa aaugaacaa 19

<210> 249
<211> 19
<212> RNA
<213> Homo sapiens

<400> 249
ccuuaaagua cuuauucag 19

<210> 250
<211> 19
<212> RNA
<213> Homo sapiens

<400> 250
gugaccauau cauagauga 19

<210> 251
<211> 19
<212> RNA
<213> Homo sapiens

<400> 251
gcuuuaaugu gcuuuuacu 19

<210> 252
<211> 19
<212> RNA
<213> Homo sapiens

<400> 252
gaaucuacaa aauuguuuu 19

<210> 253
<211> 19
<212> RNA
<213> Homo sapiens

<400> 253
uuuucuagug cugaggcau 19

<210> 254
<211> 19
<212> RNA
<213> Homo sapiens

<400> 254
ugaccaauga aucucccuu 19

<210> 255
<211> 19
<212> RNA
<213> Homo sapiens

<400> 255
gggaaaucgu ucauucauu 19

<210> 256
<211> 19

<212> RNA
<213> Homo sapiens

<400> 256
auggauaaua augcagcag 19

<210> 257
<211> 19
<212> RNA
<213> Homo sapiens

<400> 257
gaagggucua caccagauu 19

<210> 258
<211> 19
<212> RNA
<213> Homo sapiens

<400> 258
caauguauac ucucgagau 19

<210> 259
<211> 19
<212> RNA
<213> Homo sapiens

<400> 259
agugcugagg cauugguac 19

<210> 260
<211> 19
<212> RNA
<213> Homo sapiens

<400> 260
uguauacucu cgagauccu 19

<210> 261
<211> 19
<212> RNA
<213> Homo sapiens

<400> 261
cgagcuuuau aagauuaau 19

<210> 262
<211> 19
<212> RNA
<213> Homo sapiens

<400> 262
uaucacaaug uauacucuc 19

<210> 263
<211> 19
<212> RNA
<213> Homo sapiens

<400> 263
cgaagugcuu gaaaauggu 19

<210> 264
<211> 19
<212> RNA
<213> Homo sapiens

<400> 264
gcugucacaa agaauuugg 19

<210> 265
<211> 19
<212> RNA
<213> Homo sapiens

<400> 265
aucacaaugu auacucucg 19

<210> 266
<211> 19
<212> RNA
<213> Homo sapiens

<400> 266
auguacagug aacgaagaa 19

<210> 267
<211> 19
<212> RNA
<213> Homo sapiens

<400> 267
cuacagaaua ugacggugg 19

<210> 268
<211> 19
<212> RNA
<213> Homo sapiens

<400> 268
uucagcaacu uauuacuua 19

<210> 269
<211> 19
<212> RNA
<213> Homo sapiens

<400> 269
aaagauguga cuuacuuaa 19

<210> 270
<211> 19
<212> RNA
<213> Homo sapiens

<400> 270
gugcugaggc auugguaca 19

<210> 271
<211> 19
<212> RNA

<213> Homo sapiens

<400> 271
auauuuguca ccuaacgug 19

<210> 272
<211> 19
<212> RNA
<213> Homo sapiens

<400> 272
uggaagccgg aaucuagau 19

<210> 273
<211> 19
<212> RNA
<213> Homo sapiens

<400> 273
agagauuguu gaaggccau 19

<210> 274
<211> 19
<212> RNA
<213> Homo sapiens

<400> 274
uacagaauau gacgguggc 19

<210> 275
<211> 19
<212> RNA
<213> Homo sapiens

<400> 275
gggagacaac aauuugcaa 19

<210> 276
<211> 19
<212> RNA
<213> Homo sapiens

<400> 276
aauuuguuca uuaucguaa 19

<210> 277
<211> 19
<212> RNA
<213> Homo sapiens

<400> 277
ugaacagaaa agacucuuc 19

<210> 278
<211> 19
<212> RNA
<213> Homo sapiens

<400> 278
aaucaguaga aaaacaguu 19

<210> 279
<211> 19
<212> RNA
<213> Homo sapiens

<400> 279
gacucuucuu gcaguuuac 19

<210> 280
<211> 19
<212> RNA
<213> Homo sapiens

<400> 280
uaaugugcuu uuacuuccg 19

<210> 281
<211> 19
<212> RNA
<213> Homo sapiens

<400> 281
uuucagcaac uuauuacuu 19

<210> 282
<211> 19
<212> RNA
<213> Homo sapiens

<400> 282
ggagacaaca auuugcaaa 19

<210> 283
<211> 19
<212> RNA
<213> Homo sapiens

<400> 283
cauauuugag acucaaagu 19

<210> 284
<211> 19
<212> RNA
<213> Homo sapiens

<400> 284
ucauagauga ugcacuugu 19

<210> 285
<211> 19
<212> RNA
<213> Homo sapiens

<400> 285
cauagaugau gcacuuguc 19

<210> 286
<211> 19
<212> RNA
<213> Homo sapiens

<400> 286
aaacuacaga auaugacgg 19

<210> 287
<211> 19
<212> RNA
<213> Homo sapiens

<400> 287
ugaaccacug aaugagguu 19

<210> 288
<211> 19
<212> RNA
<213> Homo sapiens

<400> 288
gaaaucuagu gaaugauga 19

<210> 289
<211> 19
<212> RNA
<213> Homo sapiens

<400> 289
gaagcgagca gcugcaaag 19

<210> 290
<211> 19
<212> RNA
<213> Homo sapiens

<400> 290
auauugaaug cugucacaa 19

<210> 291
<211> 19
<212> RNA
<213> Homo sapiens

<400> 291
uuaauccauc uucuuuuga 19

<210> 292
<211> 19
<212> RNA
<213> Homo sapiens

<400> 292
ugguuuaauc caucuucuu 19

<210> 293
<211> 19
<212> RNA
<213> Homo sapiens

<400> 293
cgaaaucuag ugaaugaug 19

<210> 294
<211> 19
<212> RNA
<213> Homo sapiens

<400> 294
uauugaaugc ugucacaaa 19

<210> 295
<211> 19
<212> RNA
<213> Homo sapiens

<400> 295
uuugguuuaa uccaucuuc 19

<210> 296
<211> 19
<212> RNA
<213> Homo sapiens

<400> 296
cccuuuauau ugaaugcug 19

<210> 297
<211> 19
<212> RNA
<213> Homo sapiens

<400> 297
uuuugguuua auccaucuu 19

<210> 298
<211> 19
<212> RNA
<213> Homo sapiens

<400> 298
agccgaauga agcgagcag 19

<210> 299
<211> 19
<212> RNA
<213> Homo sapiens

<400> 299
ugcgugaaag uguuacaua 19

<210> 300
<211> 19
<212> RNA
<213> Homo sapiens

<400> 300
guuacauauu cuuucacuu 19

<210> 301
<211> 19
<212> RNA
<213> Homo sapiens

<400> 301
uuacugcuug agguugagc 19

<210> 302
<211> 19
<212> RNA
<213> Homo sapiens

<400> 302
ugaagcuagc cgaaugaag 19

<210> 303
<211> 19
<212> RNA
<213> Homo sapiens

<400> 303
ggguuaaauc acuuuugcu 19

<210> 304
<211> 19
<212> RNA
<213> Homo sapiens

<400> 304
aggguuaaau cacuuuugc 19

<210> 305
<211> 19
<212> RNA
<213> Homo sapiens

<400> 305
uuaguaacag cacaacaaa 19

<210> 306
<211> 19
<212> RNA
<213> Homo sapiens

<400> 306
uauuacugcu ugagguuga 19

<210> 307
<211> 19
<212> RNA
<213> Homo sapiens

<400> 307
uguuucagua gccaauccu 19

<210> 308
<211> 19
<212> RNA
<213> Homo sapiens

<400> 308
cauugaagcu agccgaaug 19

<210> 309

<211> 19
<212> RNA
<213> Homo sapiens

<400> 309
gacauugaag cuagccgaa 19

<210> 310
<211> 19
<212> RNA
<213> Homo sapiens

<400> 310
acauugaagc uagccgaau 19

<210> 311
<211> 19
<212> RNA
<213> Homo sapiens

<400> 311
aaugaagcga gcagcugca 19

<210> 312
<211> 19
<212> RNA
<213> Homo sapiens

<400> 312
aguguuacau auucuuuca 19

<210> 313
<211> 19
<212> RNA
<213> Homo sapiens

<400> 313
gcuugagguu gagccuuuu 19

<210> 314
<211> 19
<212> RNA
<213> Homo sapiens

<400> 314
gucuugcaau gaacuguuu 19

<210> 315
<211> 19
<212> RNA
<213> Homo sapiens

<400> 315
gccgaaugaa gcgagcagc 19

<210> 316
<211> 19
<212> RNA
<213> Homo sapiens

<400> 316

uugcgugaaa guguuacau 19

<210> 317
<211> 19
<212> RNA
<213> Homo sapiens

<400> 317
ucuugcaaug aacuguuuc 19

<210> 318
<211> 19
<212> RNA
<213> Homo sapiens

<400> 318
aguauaugaa aggacaggg 19

<210> 319
<211> 19
<212> RNA
<213> Homo sapiens

<400> 319
uugaagcuag ccgaaugaa 19

<210> 320
<211> 19
<212> RNA
<213> Homo sapiens

<400> 320
agaacuuuag uaacagcac 19

<210> 321
<211> 19
<212> RNA
<213> Homo sapiens

<400> 321
uuaaggguua aaucacuuu 19

<210> 322
<211> 19
<212> RNA
<213> Homo sapiens

<400> 322
uuguauguac agagagguu 19

<210> 323
<211> 19
<212> RNA
<213> Homo sapiens

<400> 323
aaucacuuuu gcuuguguu 19

<210> 324
<211> 19

<212> RNA
<213> Homo sapiens

<400> 324
uacauauucu uucacuugu 19

<210> 325
<211> 19
<212> RNA
<213> Homo sapiens

<400> 325
cagagagguu uuucugaau 19

<210> 326
<211> 19
<212> RNA
<213> Homo sapiens

<400> 326
uuugcuugug uuuauuacu 19

<210> 327
<211> 19
<212> RNA
<213> Homo sapiens

<400> 327
gcaaugaacu guuucagua 19

<210> 328
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 328
gugauagcuc acagggagat t 21

<210> 329
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 329
cucagcuuac cuugagaact t 21

<210> 330
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 330
gagguuuuca uauggugact t 21

<210> 331
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 331
aauccugcag acuugaagat t 21

<210> 332
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 332
ggugauagcu cacagggagt t 21

<210> 333
<211> 21
<212> DNA
<213> Artificial Sequence

<220>
<221> source

<223> /note="Description of Artificial Sequence: Synthetic oligonucleotide"

<220>
<221> source
<223> /note="Description of Combined DNA/RNA Molecule: Synthetic oligonucleotide"

<400> 333
aaaugguuua cuaugcaaat t 21

<210> 334
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 334
caguuaacug agggcugugt t 21

<210> 335
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 335
acuacauucu caauaaauct t 21

<210> 336
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 336
caucccucca agaaaggagt t 21

<210> 337
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 337
gauuagggag uucugggaat t 21

<210> 338
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 338
ugauagcuca cagggagact t 21

<210> 339
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 339
uccacagucc ugaauaucut t 21

<210> 340
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 340
cuccacaguc cugaauauct t 21

<210> 341
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 341
ugggucuggc uauuuacaat t 21

<210> 342
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 342
aaaauccugc agacuugaat t 21

<210> 343
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 343
aaaaugguuu acuaugcaat t 21

<210> 344
<211> 21

<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 344
caaaggauuu ggcaugcugt t 21

<210> 345
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 345
cacgaaugag uuuugugcut t 21

<210> 346
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 346
cuuuucguga cuugggagat t 21

<210> 347
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule:

Synthetic oligonucleotide"

<400> 347
uggucuaaau acaaugcagt t 21

<210> 348
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 348
guggucuaaa uacaaugcat t 21

<210> 349
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 349
aggugauagc ucacagggat t 21

<210> 350
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 350
ccucgagcuu uauaagauut t 21

<210> 351
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 351
gagaacucga aaggugccct t 21

<210> 352
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 352
accaguuaac ugagggcugt t 21

<210> 353
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 353
ccugcacgaa ugaguuuugt t 21

<210> 354
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 354

cccucgagcu uuauaagaut t 21

<210> 355
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 355
cuuaccuuga gaacucgaat t 21

<210> 356
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 356
cuguggucua aauacaaugt t 21

<210> 357
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 357
ucagacugug gucuaaauat t 21

<210> 358
<211> 21
<212> DNA
<213> Artificial Sequence

<220>
<221> source
<223> /note="Description of Artificial Sequence: Synthetic

oligonucleotide"

<220>
<221> source
<223> /note="Description of Combined DNA/RNA Molecule: Synthetic oligonucleotide"

<400> 358
uugagaacuc gaaaggugct t 21

<210> 359
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 359
agcuuaguuc aaggacagct t 21

<210> 360
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 360
cagcuuaguu caaggacagt t 21

<210> 361
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 361
gacuuacuua acagaagagt t 21

<210> 362
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 362
uucauauggu gaccaaugat t 21

<210> 363
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 363
cauaugguga ccaaugaaut t 21

<210> 364
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 364
auagaaaucu ccacagucct t 21

<210> 365
<211> 21
<212> DNA
<213> Artificial Sequence

<220>
<221> source
<223> /note="Description of Artificial Sequence: Synthetic oligonucleotide"

<220>

<221> source
<223> /note="Description of Combined DNA/RNA Molecule: Synthetic oligonucleotide"

<400> 365
auucugacua cauucucaat t 21

<210> 366
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 366
gcacgaauga guuuugugct t 21

<210> 367
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 367
gcauugguac agagcuucct t 21

<210> 368
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 368
caccaguuaa cugagggcut t 21

<210> 369
<211> 21
<212> DNA

<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 369
gcuuaccuug agaacucgat t 21

<210> 370
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 370
uuaccuugag aacucgaaat t 21

<210> 371
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 371
gauuuggcau gcuguaaaat t 21

<210> 372
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 372
ugcuugaaaa ugguuuacut t 21

<210> 373
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 373
ugcacgaaug aguuuugugt t 21

<210> 374
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 374
uuucgugacu ugggagacut t 21

<210> 375
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 375
ugaucagacu guggucuaat t 21

<210> 376
<211> 21
<212> DNA
<213> Artificial Sequence

<220>

<221> source
<223> /note="Description of Artificial Sequence: Synthetic oligonucleotide"

<220>
<221> source
<223> /note="Description of Combined DNA/RNA Molecule: Synthetic oligonucleotide"

<400> 376
uucugacuac auucucaaut t 21

<210> 377
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 377
gugcuugaaa augguuuact t 21

<210> 378
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 378
uuaucugaau uuguucauut t 21

<210> 379
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 379
aauagaaauc uccacagúct t 21

<210> 380
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 380
gacugugguc uaaauacaat t 21

<210> 381
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 381
cuacauucuc aauaaaucat t 21

<210> 382
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 382
agaugugacu uacuuaacat t 21

<210> 383
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 383
auuuggcaug cuguaaaact t 21

<210> 384
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 384
guacauuuuc ccaugguugt t 21

<210> 385
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 385
ucguaaugga gaauagaaat t 21

<210> 386
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 386
cuuaguucaa ggacagcagt t 21

<210> 387

<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 387
gucuaaauac aaugcagact t 21

<210> 388
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 388
uguacauuuu cccaugguut t 21

<210> 389
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 389
uugguguuaa aacccuggat t 21

<210> 390
<211> 21
<212> DNA
<213> Artificial Sequence

<220>
<221> source
<223> /note="Description of Artificial Sequence: Synthetic oligonucleotide"

<220>
<221> source

<223> /note="Description of Combined DNA/RNA Molecule: Synthetic oligonucleotide"

<400> 390
uuuucccaug guugucuact t 21

<210> 391
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 391
uuuauuaaug aaccacugat t 21

<210> 392
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 392
gggucuacac cagauugcut t 21

<210> 393
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 393
cugacuacau ucucaauaat t 21

<210> 394
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 394
gcccaaggaa aacugaucat t 21

<210> 395
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 395
aucagacugu ggucuaaaut t 21

<210> 396
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 396
gaauucgcau guacagugat t 21

<210> 397
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 397
gaucgcuaug gaaaauccut t 21

<210> 398
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 398
aucgcuaugg aaaauccugt t 21

<210> 399
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 399
uuagggaguu cugggaaaut t 21

<210> 400
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 400
gauuauagcc aaaaauggct t 21

<210> 401
<211> 21
<212> DNA
<213> Artificial Sequence

<220>
<221> source

<223> /note="Description of Artificial Sequence: Synthetic oligonucleotide"

<220>
<221> source
<223> /note="Description of Combined DNA/RNA Molecule: Synthetic oligonucleotide"

<400> 401
agaugaucgc uauggaaaat t 21

<210> 402
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 402
agauaucaca gacagaucut t 21

<210> 403
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 403
gguauguuca cauacgaugt t 21

<210> 404
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 404
acaauagaau ucgcauguat t 21

<210> 405
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 405
ggcuagagau gaucgcuaut t 21

<210> 406
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 406
cuguccaacu uuucuucgut t 21

<210> 407
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 407
augcacuugu ccggcuagat t 21

<210> 408
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 408
aauccagaua uugguaugut t 21

<210> 409
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 409
uccggcuaga gaugaucgct t 21

<210> 410
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 410
gauugucgaa aaccacuuat t 21

<210> 411
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 411
ugaucgcuau ggaaaaucct t 21

<210> 412
<211> 21

<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 412
augaucgcua uggaaaauct t 21

<210> 413
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 413
cuuauaugug gaagccggat t 21

<210> 414
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 414
gauaucacag acagaucuut t 21

<210> 415
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule:

Synthetic oligonucleotide

<400> 415
acuuauucag accagaagat t 21

<210> 416
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 416
uguccaacuu uucuucguat t 21

<210> 417
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 417
uuauaugugg aagccggaat t 21

<210> 418
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 418
agcaguugaa uccauauuut t 21

<210> 419
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 419
ugcaaaugua gugggagggt t 21

<210> 420
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 420
gaugcacuug uccggcuagt t 21

<210> 421
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 421
gaugaugcac uuguccggct t 21

<210> 422
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 422

acuuguccgg cuagagaugt t 21

<210> 423
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 423
aagguuaccu acaucucaut t 21

<210> 424
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 424
agaugauuau agccaaaaat t 21

<210> 425
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 425
gacuugggag acucucacct t 21

<210> 426
<211> 21
<212> DNA
<213> Artificial Sequence

<220>
<221> source
<223> /note="Description of Artificial Sequence: Synthetic

oligonucleotide"

<220>
<221> source
<223> /note="Description of Combined DNA/RNA Molecule: Synthetic oligonucleotide"

<400> 426
acuuuucuuc guauggauat t 21

<210> 427
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 427
aaagguuacc uacaucucat t 21

<210> 428
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 428
gacaauagaa uucgcaugut t 21

<210> 429
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 429
uuuacaauaa cuguauacut t 21

<210> 430
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 430
uuacucugau uggcauagut t 21

<210> 431
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 431
cuggcuauuu acaauaacut t 21

<210> 432
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 432
guauguucac auacgaugat t 21

<210> 433
<211> 21
<212> DNA
<213> Artificial Sequence

<220>
<221> source
<223> /note="Description of Artificial Sequence: Synthetic oligonucleotide"

<220>

<221> source
<223> /note="Description of Combined DNA/RNA Molecule: Synthetic oligonucleotide"

<400> 433
agucauaagc aaugaauuut t 21

<210> 434
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 434
guugaaucca uauuugagat t 21

<210> 435
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 435
uuaaugcaaa acucugugat t 21

<210> 436
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 436
ugcacuuguc cggcuagagt t 21

<210> 437
<211> 21
<212> DNA

<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 437
cccaaugaug uaugaucuat t 21

<210> 438
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 438
uacuuauuca gaccagaagt t 21

<210> 439
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 439
auauugguau guucacauat t 21

<210> 440
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 440
aaacucugug auccucauct t 21

<210> 441
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 441
uuccagauau cacagacagt t 21

<210> 442
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 442
gaucacuuuc cagauaucat t 21

<210> 443
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 443
aucacuuucc agauaucact t 21

<210> 444
<211> 21
<212> DNA
<213> Artificial Sequence

<220>

<221> source
<223> /note="Description of Artificial Sequence: Synthetic oligonucleotide"

<220>
<221> source
<223> /note="Description of Combined DNA/RNA Molecule: Synthetic oligonucleotide"

<400> 444
cacuuuccag auaucacagt t 21

<210> 445
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 445
ccagacacag aaagguuact t 21

<210> 446
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 446
aauaacugua uacuggaugt t 21

<210> 447
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 447
ucacuuucca gauaucacat t 21

<210> 448
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 448
uuuaugacau gucccuuuat t 21

<210> 449
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 449
agacacagaa agguuaccut t 21

<210> 450
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 450
ccaaaggauu uggcaugcut t 21

<210> 451
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule:
Synthetic oligonucleotide"

<400> 451
ccuacaucuc auacuugcut t 21

<210> 452
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 452
cccagacaca gaaagguuat t 21

<210> 453
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 453
auaaaauucc aauuacaaat t 21

<210> 454
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 454
auuaucguaa uggagaauat t 21

<210> 455

<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 455
aguuagacgu gaccauauct t 21

<210> 456
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 456
aucguaaugg agaauagaat t 21

<210> 457
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 457
uucguaugga uaauaaugct t 21

<210> 458
<211> 21
<212> DNA
<213> Artificial Sequence

<220>
<221> source
<223> /note="Description of Artificial Sequence: Synthetic oligonucleotide"

<220>
<221> source

<223> /note="Description of Combined DNA/RNA Molecule: Synthetic oligonucleotide"

<400> 458
acguaucaca auguauacut t 21

<210> 459
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 459
caucacguau gccaaaggat t 21

<210> 460
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 460
ugucgaaaac cacuuaucct t 21

<210> 461
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 461
acgaugaauc uacaaaauut t 21

<210> 462
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 462
uuacaacggg cacagacagt t 21

<210> 463
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 463
gaaaucguuc auucauuuat t 21

<210> 464
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 464
ucuucguaug gauaauaaut t 21

<210> 465
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 465
cacauacgau gaaucuacat t 21

<210> 466
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 466
ucgaagugcu ugaaaauggt t 21

<210> 467
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 467
gucgaaaacc acuuauccct t 21

<210> 468
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 468
ucacauacga ugaaucuact t 21

<210> 469
<211> 21
<212> DNA
<213> Artificial Sequence

<220>
<221> source

<223> /note="Description of Artificial Sequence: Synthetic oligonucleotide"

<220>
<221> source
<223> /note="Description of Combined DNA/RNA Molecule: Synthetic oligonucleotide"

<400> 469
acgaagaauc acuguucuct t 21

<210> 470
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 470
acucucgaga uccuaauuat t 21

<210> 471
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 471
uguauacugg auguacauut t 21

<210> 472
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 472
ugaccauauc auagaugaut t 21

<210> 473
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 473
ugcuuauaug uggaagccgt t 21

<210> 474
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 474
uauaccaggg acucuguuct t 21

<210> 475
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 475
guacuuauuc agaccagaat t 21

<210> 476
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 476
auuggcauag uacuggguct t 21

<210> 477
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 477
cuguauacug gauguacaut t 21

<210> 478
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 478
uuuucuucgu auggauaaut t 21

<210> 479
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 479
ucggagaggu uuucauaugt t 21

<210> 480
<211> 21

<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 480
gggucaguuu acucugauut t 21

<210> 481
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 481
ggucaguuua cucugauugt t 21

<210> 482
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 482
gaaucucccu uaaaguacut t 21

<210> 483
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule:

Synthetic oligonucleotide

<400> 483
ccaaugaauc ucccuuaaat t 21

<210> 484
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 484
gacgugacca uaucauagat t 21

<210> 485
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 485
acgugaccau aucauagaut t 21

<210> 486
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 486
guucacauac gaugaaucut t 21

<210> 487
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 487
uuuaacaguc gaaaucuagt t 21

<210> 488
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 488
gacgguggcu auaccagggt t 21

<210> 489
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 489
ucacaaugua uacucucgat t 21

<210> 490
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 490

auuuaacagu cgaaaucuat t 21

<210> 491
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 491
ccuggauugu cgaaaaccat t 21

<210> 492
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 492
aagaugugac uuacuuaact t 21

<210> 493
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 493
cagaccagau ucggagaaut t 21

<210> 494
<211> 21
<212> DNA
<213> Artificial Sequence

<220>
<221> source
<223> /note="Description of Artificial Sequence: Synthetic

oligonucleotide"

<220>
<221> source
<223> /note="Description of Combined DNA/RNA Molecule: Synthetic oligonucleotide"

<400> 494
cuucguaugg auaauaaugt t 21

<210> 495
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 495
gacguaucac aauguauact t 21

<210> 496
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 496
ucguauggau aauaaugcat t 21

<210> 497
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 497
cauacgauga aucuacaaat t 21

<210> 498
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 498
gacuugacgu aucacaaugt t 21

<210> 499
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 499
gcuuuucgga gagguuuuct t 21

<210> 500
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 500
ggaaaucguu cauucauuut t 21

<210> 501
<211> 21
<212> DNA
<213> Artificial Sequence

<220>
<221> source
<223> /note="Description of Artificial Sequence: Synthetic oligonucleotide"

<220>

<221> source
<223> /note="Description of Combined DNA/RNA Molecule: Synthetic oligonucleotide"

<400> 501
ucgaaaacca cuuaucccut t 21

<210> 502
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 502
ugucaaucuu uauucugact t 21

<210> 503
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 503
accacuuauc ccuuuugaat t 21

<210> 504
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 504
gcuucgaagu gcuugaaaat t 21

<210> 505
<211> 21
<212> DNA

<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 505
uugucaaucu uuauucugat t 21

<210> 506
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 506
uagacgugac cauaucauat t 21

<210> 507
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 507
cugcuucgaa gugcuugaat t 21

<210> 508
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 508
caaggauagg ugauagcuct t 21

<210> 509
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 509
ugcacuugua uauuugucat t 21

<210> 510
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 510
cguaucacaa uguauacuct t 21

<210> 511
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 511
cgcauguaca gugaacgaat t 21

<210> 512
<211> 21
<212> DNA
<213> Artificial Sequence

<220>

<221> source
<223> /note="Description of Artificial Sequence: Synthetic oligonucleotide"

<220>
<221> source
<223> /note="Description of Combined DNA/RNA Molecule: Synthetic oligonucleotide"

<400> 512
auuagaaggg ucuacaccat t 21

<210> 513
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 513
uuuggcaugc uguaaaacat t 21

<210> 514
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 514
cccuuaaagu acuuauucat t 21

<210> 515
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 515
acaauguaua cucucgagat t 21

<210> 516
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 516
ugacgguggc uauaccaggt t 21

<210> 517
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 517
ucgagauccu aauuaucugt t 21

<210> 518
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 518
ugagggucag uuuacucugt t 21

<210> 519
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule:
Synthetic oligonucleotide"

<400> 519
aucucccuua aaguacuuat t 21

<210> 520
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 520
gaauugcuua uauguggaat t 21

<210> 521
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 521
cucaaggaua ggugauagct t 21

<210> 522
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 522
aaaacaguuc aaggcuuuut t 21

<210> 523

<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 523
agggagacaa caauuugcat t 21

<210> 524
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 524
ugugacuuga cguaucacat t 21

<210> 525
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 525
aaugugacuu gacguaucat t 21

<210> 526
<211> 21
<212> DNA
<213> Artificial Sequence

<220>
<221> source
<223> /note="Description of Artificial Sequence: Synthetic oligonucleotide"

<220>
<221> source

<223> /note="Description of Combined DNA/RNA Molecule: Synthetic oligonucleotide"

<400> 526
caaggcuuuu cggagaggut t 21

<210> 527
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 527
ucgcauguac agugaacgat t 21

<210> 528
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 528
cuaacgugga augugacuut t 21

<210> 529
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 529
cacuuauccc uuuugaagat t 21

<210> 530
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 530
aggacuagga aaauuaaagt t 21

<210> 531
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 531
agugaacgaa gaaucacugt t 21

<210> 532
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 532
ccauaucaua gaugaugcat t 21

<210> 533
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 533
caacgggcac agacagagct t 21

<210> 534
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 534
acuuaucccu uuugaagagt t 21

<210> 535
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 535
uggauaauaa ugcagcagct t 21

<210> 536
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 536
gaauuuaaca gucgaaauct t 21

<210> 537
<211> 21
<212> DNA
<213> Artificial Sequence

<220>
<221> source

<223> /note="Description of Artificial Sequence: Synthetic oligonucleotide"

<220>
<221> source
<223> /note="Description of Combined DNA/RNA Molecule: Synthetic oligonucleotide"

<400> 537
agauuuauug gaguaugaat t 21

<210> 538
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 538
cgaagaauca cuguucucut t 21

<210> 539
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 539
acugaggguc aguuuacuct t 21

<210> 540
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 540
caguuuacaa cgggcacagt t 21

<210> 541
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 541
ugaauugcuu auauguggat t 21

<210> 542
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 542
acucaaaguu agacgugact t 21

<210> 543
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 543
gugacuugac guaucacaat t 21

<210> 544
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 544
augaauuuaa cagucgaaat t 21

<210> 545
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 545
auaugacggu ggcuauacct t 21

<210> 546
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 546
aacgaagaau cacuguucut t 21

<210> 547
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 547
gccauuagaa gggucuacat t 21

<210> 548
<211> 21

<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 548
uuuauaagau uaaugcaaat t 21

<210> 549
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 549
gaauccauau uugagacuct t 21

<210> 550
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 550
uuauuggagu augaagggat t 21

<210> 551
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule:

Synthetic oligonucleotide

<400> 551
agcuuccgga aaguuaaact t 21

<210> 552
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 552
uacucugauu ggcauaguat t 21

<210> 553
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 553
aguuuuuucu agugcugagt t 21

<210> 554
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 554
uguucacaua cgaugaauct t 21

<210> 555
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 555
aaucucccuu aaaguacuut t 21

<210> 556
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 556
gacucaaagu uagacgugat t 21

<210> 557
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 557
aauuuaacag ucgaaaucut t 21

<210> 558
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 558

augugcuuuu acuuccggat t 21

<210> 559
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 559
uugucaccua acguggaaut t 21

<210> 560
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 560
augugacuug acguaucact t 21

<210> 561
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 561
gugaacgaag aaucacugut t 21

<210> 562
<211> 21
<212> DNA
<213> Artificial Sequence

<220>
<221> source
<223> /note="Description of Artificial Sequence: Synthetic

oligonucleotide"

<220>
<221> source
<223> /note="Description of Combined DNA/RNA Molecule: Synthetic oligonucleotide"

<400> 562
gaaucuagau uuccaagcat t 21

<210> 563
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 563
gucaaucuuu auucugacut t 21

<210> 564
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 564
acacaaauca caaugaagat t 21

<210> 565
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 565
gugaccaaug aaucucccut t 21

<210> 566
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 566
ugaaucuccc uuaaaguact t 21

<210> 567
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 567
augaaccacu gaaugaggut t 21

<210> 568
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 568
gcuuccggaa aguuaaacat t 21

<210> 569
<211> 21
<212> DNA
<213> Artificial Sequence

<220>
<221> source
<223> /note="Description of Artificial Sequence: Synthetic oligonucleotide"

<220>

<221> source
<223> /note="Description of Combined DNA/RNA Molecule: Synthetic oligonucleotide"

<400> 569
gagggucagu uuacucugat t 21

<210> 570
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 570
cauguacagu gaacgaagat t 21

<210> 571
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 571
aagaguuuuu ucuagugcut t 21

<210> 572
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 572
caaugcagac cagauucggt t 21

<210> 573
<211> 21
<212> DNA

<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 573
auuucagcaa cuuauuacut t 21

<210> 574
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 574
uauauuuguc accuaacgut t 21

<210> 575
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 575
cugagauaaa aaugaacaat t 21

<210> 576
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 576
ccuuaaagua cuuauucagt t 21

<210> 577
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 577
gugaccauau cauagaugat t 21

<210> 578
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 578
gcuuuaaugu gcuuuuacut t 21

<210> 579
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 579
gaaucuacaa aauuguuuut t 21

<210> 580
<211> 21
<212> DNA
<213> Artificial Sequence

<220>

<221> source
<223> /note="Description of Artificial Sequence: Synthetic oligonucleotide"

<220>
<221> source
<223> /note="Description of Combined DNA/RNA Molecule: Synthetic oligonucleotide"

<400> 580
uuuucuagug cugaggcaut t 21

<210> 581
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 581
ugaccaauga aucucccuut t 21

<210> 582
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 582
gggaaaucgu ucauucauut t 21

<210> 583
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 583
auggauaaua augcagcagt t 21

<210> 584
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 584
gaagggucua caccagauut t 21

<210> 585
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 585
caauguauac ucucgagaut t 21

<210> 586
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 586
agugcugagg cauugguact t 21

<210> 587
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 587
uguauacucu cgagauccut t 21

<210> 588
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 588
cgagcuuuau aagauuaaut t 21

<210> 589
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 589
uaucacaaug uauacucuct t 21

<210> 590
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 590
cgaagugcuu gaaaauggut t 21

<210> 591

<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 591
gcugucacaa agaauuuggt t 21

<210> 592
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 592
aucacaaugu auacucucgt t 21

<210> 593
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 593
auguacagug aacgaagaat t 21

<210> 594
<211> 21
<212> DNA
<213> Artificial Sequence

<220>
<221> source
<223> /note="Description of Artificial Sequence: Synthetic oligonucleotide"

<220>
<221> source

<223> /note="Description of Combined DNA/RNA Molecule: Synthetic oligonucleotide"

<400> 594
cuacagaaua ugacgguggt t 21

<210> 595
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 595
uucagcaacu uauuacuuat t 21

<210> 596
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 596
aaagauguga cuuacuuaat t 21

<210> 597
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 597
gugcugaggc auugguacat t 21

<210> 598
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 598
auauuuguca ccuaacgugt t 21

<210> 599
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 599
uggaagccgg aaucuagaut t 21

<210> 600
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 600
agagauuguu gaaggccaut t 21

<210> 601
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 601
uacagaauau gacgguggct t 21

<210> 602
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 602
gggagacaac aauuugcaat t 21

<210> 603
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 603
aauuuguuca uuaucguaat t 21

<210> 604
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 604
ugaacagaaa agacucuuct t 21

<210> 605
<211> 21
<212> DNA
<213> Artificial Sequence

<220>
<221> source

<223> /note="Description of Artificial Sequence: Synthetic oligonucleotide"

<220>
<221> source
<223> /note="Description of Combined DNA/RNA Molecule: Synthetic oligonucleotide"

<400> 605
aaucaguaga aaaacaguut t 21

<210> 606
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 606
gacucuucuu gcaguuuact t 21

<210> 607
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 607
uaaugugcuu uuacuuccgt t 21

<210> 608
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 608
uuucagcaac uuauuacuut t 21

<210> 609
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 609
ggagacaaca auuugcaaat t 21

<210> 610
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 610
cauauuugag acucaaagut t 21

<210> 611
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 611
ucauagauga ugcacuugut t 21

<210> 612
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule:
Synthetic oligonucleotide"

<400> 612
cauagaugau gcacuuguct t 21

<210> 613
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 613
aaacuacaga auaugacggt t 21

<210> 614
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 614
ugaaccacug aaugagguut t 21

<210> 615
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 615
gaaaucuagu gaaugaugat t 21

<210> 616
<211> 21

<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 616
gaagcgagca gcugcaaagt t 21

<210> 617
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 617
auauugaaug cugucacaat t 21

<210> 618
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 618
uuaauccauc uucuuuugat t 21

<210> 619
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule:

Synthetic oligonucleotide"

<400> 619
ugguuuaauc caucuucuut t 21

<210> 620
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 620
cgaaaucuag ugaaugaugt t 21

<210> 621
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 621
uauugaaugc ugucacaaat t 21

<210> 622
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 622
uuugguuuaa uccaucuuct t 21

<210> 623
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 623
cccuuuauau ugaaugcugt t 21

<210> 624
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 624
uuuugguuua auccaucuut t 21

<210> 625
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 625
agccgaauga agcgagcagt t 21

<210> 626
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 626

ugcgugaaag uguuacauat t 21

<210> 627
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 627
guuacauauu cuuucacuut t 21

<210> 628
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 628
uuacugcuug agguugagct t 21

<210> 629
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 629
ugaagcuagc cgaaugaagt t 21

<210> 630
<211> 21
<212> DNA
<213> Artificial Sequence

<220>
<221> source
<223> /note="Description of Artificial Sequence: Synthetic

oligonucleotide"

<220>
<221> source
<223> /note="Description of Combined DNA/RNA Molecule:
Synthetic oligonucleotide"

<400> 630
ggguuaaauc acuuuugcut t 21

<210> 631
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 631
aggguuaaau cacuuuugct t 21

<210> 632
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 632
uuaguaacag cacaacaaat t 21

<210> 633
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 633
uauuacugcu ugagguugat t 21

<210> 634
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 634
uguuucagua gccaauccut t 21

<210> 635
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 635
cauugaagcu agccgaaugt t 21

<210> 636
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 636
gacauugaag cuagccgaat t 21

<210> 637
<211> 21
<212> DNA
<213> Artificial Sequence

<220>
<221> source
<223> /note="Description of Artificial Sequence: Synthetic oligonucleotide"

<220>

<221> source
<223> /note="Description of Combined DNA/RNA Molecule:
Synthetic oligonucleotide"

<400> 637
acauugaagc uagccgaaut t 21

<210> 638
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 638
aaugaagcga gcagcugcat t 21

<210> 639
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 639
aguguuacau auucuuucat t 21

<210> 640
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 640
gcuugagguu gagccuuuut t 21

<210> 641
<211> 21
<212> DNA

<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 641
gucuugcaau gaacuguuut t 21

<210> 642
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 642
gccgaaugaa gcgagcagct t 21

<210> 643
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 643
uugcgugaaa guguuacaut t 21

<210> 644
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 644
ucuugcaaug aacuguuuct t 21

<210> 645
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 645
aguauaugaa aggacagggt t 21

<210> 646
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 646
uugaagcuag ccgaaugaat t 21

<210> 647
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 647
agaacuuuag uaacagcact t 21

<210> 648
<211> 21
<212> DNA
<213> Artificial Sequence

<220>

<221> source
<223> /note="Description of Artificial Sequence: Synthetic oligonucleotide"

<220>
<221> source
<223> /note="Description of Combined DNA/RNA Molecule: Synthetic oligonucleotide"

<400> 648
uuaaggguua aaucacuuut t 21

<210> 649
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 649
uuguauguac agagagguut t 21

<210> 650
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 650
aaucacuuuu gcuuguguut t 21

<210> 651
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 651
uacauauucu uucacuugut t 21

<210> 652
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 652
cagagagguu uuucugaaut t 21

<210> 653
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 653
uuugcuugug uuuauuacut t 21

<210> 654
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 654
gcaaugaacu guuucaguat t 21

<210> 655
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule:
Synthetic oligonucleotide"

<400> 655
ucucccugug agcuaucact t 21

<210> 656
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 656
guucucaagg uaagcugagt t 21

<210> 657
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 657
gucaccauau gaaaaccuct t 21

<210> 658
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 658
ucuucaaguc ugcaggauut t 21

<210> 659

<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 659
cucccuguga gcuaucacct t 21

<210> 660
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 660
uuugcauagu aaaccauuut t 21

<210> 661
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 661
cacagcccuc aguuaacugt t 21

<210> 662
<211> 21
<212> DNA
<213> Artificial Sequence

<220>
<221> source
<223> /note="Description of Artificial Sequence: Synthetic oligonucleotide"

<220>
<221> source

<223> /note="Description of Combined DNA/RNA Molecule: Synthetic oligonucleotide"

<400> 662
gauuuauuga gaauguagut t 21

<210> 663
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 663
cuccuuucuu ggagggaugt t 21

<210> 664
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 664
uucccagaac ucccuaauct t 21

<210> 665
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 665
gucucccugu gagcuaucat t 21

<210> 666
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 666
agauauucag gacuguggat t 21

<210> 667
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 667
gauauucagg acuguggagt t 21

<210> 668
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 668
uuguaaauag ccagacccat t 21

<210> 669
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 669
uucaagucug caggauuuut t 21

<210> 670
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 670
uugcauagua aaccauuuut t 21

<210> 671
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 671
cagcaugcca aauccuuugt t 21

<210> 672
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 672
agcacaaaac ucauucgugt t 21

<210> 673
<211> 21
<212> DNA
<213> Artificial Sequence

<220>
<221> source

<223> /note="Description of Artificial Sequence: Synthetic oligonucleotide"

<220>
<221> source
<223> /note="Description of Combined DNA/RNA Molecule: Synthetic oligonucleotide"

<400> 673
ucucccaagu cacgaaaagt t 21

<210> 674
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 674
cugcauugua uuuagaccat t 21

<210> 675
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 675
ugcauuguau uuagaccact t 21

<210> 676
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 676
ucccugugag cuaucaccut t 21

<210> 677
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 677
aaucuuauaa agcucgaggt t 21

<210> 678
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 678
gggcaccuuu cgaguucuct t 21

<210> 679
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 679
cagcccucag uuaacuggut t 21

<210> 680
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 680
caaaacucau ucgugcaggt t 21

<210> 681
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 681
aucuuauaaa gcucgagggt t 21

<210> 682
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 682
uucgaguucu caagguaagt t 21

<210> 683
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 683
cauuguauuu agaccacagt t 21

<210> 684
<211> 21

<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 684
uauuuagacc acagucugat t 21

<210> 685
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 685
gcaccuuucg aguucucaat t 21

<210> 686
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 686
gcuguccuug aacuaagcut t 21

<210> 687
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule:

Synthetic oligonucleotide

<400> 687
cuguccuuga acuaagcugt t 21

<210> 688
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 688
cucuucuguu aaguaaguct t 21

<210> 689
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 689
ucauugguca ccauaugaat t 21

<210> 690
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 690
auucauuggu caccauaugt t 21

<210> 691
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 691
ggacugugga gauuucuaut t 21

<210> 692
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 692
uugagaaugu agucagaaut t 21

<210> 693
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 693
gcacaaaacu cauucgugct t 21

<210> 694
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 694

ggaagcucug uaccaaugct t 21

<210> 695
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 695
agcccucagu uaacuggugt t 21

<210> 696
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 696
ucgaguucuc aagguaagct t 21

<210> 697
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 697
uuucgaguuc ucaagguaat t 21

<210> 698
<211> 21
<212> DNA
<213> Artificial Sequence

<220>
<221> source
<223> /note="Description of Artificial Sequence: Synthetic

oligonucleotide"

<220>
<221> source
<223> /note="Description of Combined DNA/RNA Molecule: Synthetic oligonucleotide"

<400> 698
uuuuacagca ugccaaauct t 21

<210> 699
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 699
aguaaaccau uuucaagcat t 21

<210> 700
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 700
cacaaaacuc auucgugcat t 21

<210> 701
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 701
agucucccaa gucacgaaat t 21

<210> 702
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 702
uuagaccaca gucugaucat t 21

<210> 703
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 703
auugagaaug uagucagaat t 21

<210> 704
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 704
guaaaccauu uucaagcact t 21

<210> 705
<211> 21
<212> DNA
<213> Artificial Sequence

<220>
<221> source
<223> /note="Description of Artificial Sequence: Synthetic oligonucleotide"

<220>

<221> source
<223> /note="Description of Combined DNA/RNA Molecule: Synthetic oligonucleotide"

<400> 705
aaugaacaaa uucagauaat t 21

<210> 706
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 706
gacuguggag auuucuauut t 21

<210> 707
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 707
uuguauuuag accacaguct t 21

<210> 708
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 708
ugauuuauug agaauguagt t 21

<210> 709
<211> 21
<212> DNA

<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 709
uguuaaguaa gucacaucut t 21

<210> 710
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 710
guuuuacagc augccaaaut t 21

<210> 711
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 711
caaccauggg aaaauguact t 21

<210> 712
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 712
uuucuauucu ccauuacgat t 21

<210> 713
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 713
cugcuguccu ugaacuaagt t 21

<210> 714
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 714
gucugcauug uauuuagact t 21

<210> 715
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 715
aaccauggga aaauguacat t 21

<210> 716
<211> 21
<212> DNA
<213> Artificial Sequence

<220>

<221> source
<223> /note="Description of Artificial Sequence: Synthetic oligonucleotide"

<220>
<221> source
<223> /note="Description of Combined DNA/RNA Molecule: Synthetic oligonucleotide"

<400> 716
uccaggguuu uaacaccaat t 21

<210> 717
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 717
guagacaacc augggaaaat t 21

<210> 718
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 718
ucagugguuc auuaauaaat t 21

<210> 719
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 719
agcaaucugg uguagaccct t 21

<210> 720
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 720
uuauugagaa uguagucagt t 21

<210> 721
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 721
ugaucaguuu uccuugggct t 21

<210> 722
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 722
auuuagacca cagucugaut t 21

<210> 723
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 723
ucacuguaca ugcgaauuct t 21

<210> 724
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 724
aggauuuucc auagcgauct t 21

<210> 725
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 725
caggauuuuc cauagcgaut t 21

<210> 726
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 726
auuucccaga acucccuaat t 21

<210> 727

<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 727
gccauuuuug gcuauaauct t 21

<210> 728
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 728
uuuuccauag cgaucaucut t 21

<210> 729
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 729
agaucugucu gugauaucut t 21

<210> 730
<211> 21
<212> DNA
<213> Artificial Sequence

<220>
<221> source
<223> /note="Description of Artificial Sequence: Synthetic oligonucleotide"

<220>
<221> source

<223> /note="Description of Combined DNA/RNA Molecule: Synthetic oligonucleotide"

<400> 730
caucguaugu gaacauacct t 21

<210> 731
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 731
uacaugcgaa uucuauugut t 21

<210> 732
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 732
auagcgauca ucucuagcct t 21

<210> 733
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 733
acgaagaaaa guuggacagt t 21

<210> 734
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 734
ucuagccgga caagugcaut t 21

<210> 735
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 735
acauaccaau aucuggauut t 21

<210> 736
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 736
gcgaucaucu cuagccggat t 21

<210> 737
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 737
uaagugguuu ucgacaauct t 21

<210> 738
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 738
ggauuuucca uagcgaucat t 21

<210> 739
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 739
gauuuuccau agcgaucaut t 21

<210> 740
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 740
uccggcuucc acauauaagt t 21

<210> 741
<211> 21
<212> DNA
<213> Artificial Sequence

<220>
<221> source

<223> /note="Description of Artificial Sequence: Synthetic oligonucleotide"

<220>
<221> source
<223> /note="Description of Combined DNA/RNA Molecule: Synthetic oligonucleotide"

<400> 741
aagaucuguc ugugauauct t 21

<210> 742
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 742
ucuucugguc ugaauaagut t 21

<210> 743
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 743
uacgaagaaa aguuggacat t 21

<210> 744
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 744
uuccggcuuc cacauauaat t 21

<210> 745
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 745
aaauauggau ucaacugcut t 21

<210> 746
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 746
cccucccacu acauuugcat t 21

<210> 747
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 747
cuagccggac aagugcauct t 21

<210> 748
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 748
gccggacaag ugcaucauct t 21

<210> 749
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 749
caucucuagc cggacaagut t 21

<210> 750
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 750
augagaugua gguaaccuut t 21

<210> 751
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 751
uuuuuggcua uaaucaucut t 21

<210> 752
<211> 21

<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 752
ggugagaguc ucccaagucu t 21

<210> 753
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 753
uauccauacg aagaaaagut t 21

<210> 754
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 754
ugagauguag guaaccuuut t 21

<210> 755
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule:

Synthetic oligonucleotide

<400> 755
acaugcgaau ucuauuguct t 21

<210> 756
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 756
aguauacagu uauuguaaat t 21

<210> 757
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 757
acuaugccaa ucagaguaat t 21

<210> 758
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 758
aguuauugua aauagccagt t 21

<210> 759
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 759
ucaucguaug ugaacauact t 21

<210> 760
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 760
aaauucauug cuuaugacut t 21

<210> 761
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 761
ucucaaauau ggauucaact t 21

<210> 762
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 762

ucacagaguu uugcauuaat t 21

<210> 763
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 763
cucuagccgg acaagugcat t 21

<210> 764
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 764
uagaucauac aucauugggt t 21

<210> 765
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 765
cuucuggucu gaauaaguat t 21

<210> 766
<211> 21
<212> DNA
<213> Artificial Sequence

<220>
<221> source
<223> /note="Description of Artificial Sequence: Synthetic

oligonucleotide"

<220>
<221> source
<223> /note="Description of Combined DNA/RNA Molecule: Synthetic oligonucleotide"

<400> 766
uaugugaaca uaccaauaut t 21

<210> 767
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 767
gaugaggauc acagaguuut t 21

<210> 768
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 768
cugucuguga uaucuggaat t 21

<210> 769
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 769
ugauaucugg aaagugauct t 21

<210> 770
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 770
gugauaucug gaaagugaut t 21

<210> 771
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 771
cugugauauc uggaaagugt t 21

<210> 772
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 772
guaaccuuuc ugugucuggt t 21

<210> 773
<211> 21
<212> DNA
<213> Artificial Sequence

<220>
<221> source
<223> /note="Description of Artificial Sequence: Synthetic oligonucleotide"

<220>

<221> source
<223> /note="Description of Combined DNA/RNA Molecule:
Synthetic oligonucleotide"

<400> 773
cauccaguau acaguuauut t 21

<210> 774
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 774
ugugauaucu ggaaagugat t 21

<210> 775
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 775
uaaagggaca ugucauaaat t 21

<210> 776
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 776
agguaaccuu ucugugucut t 21

<210> 777
<211> 21
<212> DNA

<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 777
agcaugccaa auccuuuggt t 21

<210> 778
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 778
agcaaguaug agauguaggt t 21

<210> 779
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 779
uaaccuuucu gugucugggt t 21

<210> 780
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 780
uuuguaauug gaauuuuaut t 21

<210> 781
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 781
uauucuccau uacgauaaut t 21

<210> 782
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 782
gauaugguca cgucuaacut t 21

<210> 783
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 783
uucuauucuc cauuacgaut t 21

<210> 784
<211> 21
<212> DNA
<213> Artificial Sequence

<220>

<221> source
<223> /note="Description of Artificial Sequence: Synthetic oligonucleotide"

<220>
<221> source
<223> /note="Description of Combined DNA/RNA Molecule: Synthetic oligonucleotide"

<400> 784
gcauuauuau ccauacgaat t 21

<210> 785
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 785
aguauacauu gugauacgut t 21

<210> 786
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 786
uccuuuggca uacgugaugt t 21

<210> 787
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 787
ggauaagugg uuuucgacat t 21

<210> 788
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 788
aauuuuguag auucaucgut t 21

<210> 789
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 789
cugucugugc ccguuguaat t 21

<210> 790
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 790
uaaaugaaug aacgauuuct t 21

<210> 791
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 791
auuauuaucc auacgaagat t 21

<210> 792
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 792
uguagauuca ucguaugugt t 21

<210> 793
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 793
ccauuuucaa gcacuucgat t 21

<210> 794
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 794
gggauaagug guuuucgact t 21

<210> 795

<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 795
guagauucau cguaugugat t 21

<210> 796
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 796
gagaacagug auucuucgut t 21

<210> 797
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 797
uaauuaggau cucgagagut t 21

<210> 798
<211> 21
<212> DNA
<213> Artificial Sequence

<220>
<221> source
<223> /note="Description of Artificial Sequence: Synthetic oligonucleotide"

<220>
<221> source

<223> /note="Description of Combined DNA/RNA Molecule: Synthetic oligonucleotide"

<400> 798
aauguacauc caguauacat t 21

<210> 799
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 799
aucaucuaug auauggucat t 21

<210> 800
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 800
cggcuuccac auauaagcat t 21

<210> 801
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 801
gaacagaguc ccugguauat t 21

<210> 802
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 802
uucuggucug aauaaguact t 21

<210> 803
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 803
gacccaguac uaugccaaut t 21

<210> 804
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 804
auguacaucc aguauacagt t 21

<210> 805
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 805
auuauccaua cgaagaaaat t 21

<210> 806
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 806
cauaugaaaa ccucuccgat t 21

<210> 807
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 807
aaucagagua aacugaccct t 21

<210> 808
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 808
caaucagagu aaacugacct t 21

<210> 809
<211> 21
<212> DNA
<213> Artificial Sequence

<220>
<221> source

<223> /note="Description of Artificial Sequence: Synthetic oligonucleotide"

<220>
<221> source
<223> /note="Description of Combined DNA/RNA Molecule: Synthetic oligonucleotide"

<400> 809
aguacuuuaa gggagauuct t 21

<210> 810
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 810
uuuaagggag auucauuggt t 21

<210> 811
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 811
ucuaugauau ggucacguct t 21

<210> 812
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 812
aucuaugaua uggucacgut t 21

<210> 813
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 813
agauucaucg uaugugaact t 21

<210> 814
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 814
cuagauuucg acuguuaaat t 21

<210> 815
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 815
cccugguaua gccaccguct t 21

<210> 816
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 816
ucgagaguau acauugugat t 21

<210> 817
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 817
uagauuucga cuguuaaaut t 21

<210> 818
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 818
ugguuuucga caauccaggt t 21

<210> 819
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 819
guuaaguaag ucacaucuut t 21

<210> 820
<211> 21

<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 820
auucuccgaa ucuggucugt t 21

<210> 821
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 821
cauuauuauc cauacgaagt t 21

<210> 822
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 822
guauacauug ugauacguct t 21

<210> 823
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule:

Synthetic oligonucleotide

<400> 823
ugcauuauua uccauacgat t 21

<210> 824
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 824
uuuguagauu caucguaugt t 21

<210> 825
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 825
cauugugaua cgucaaguct t 21

<210> 826
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 826
gaaaaccucu ccgaaaagct t 21

<210> 827
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 827
aaaugaauga acgauuucct t 21

<210> 828
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 828
agggauaagu gguuuucgat t 21

<210> 829
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 829
gucagaauaa agauugacat t 21

<210> 830
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 830

uucaaaaggg auaaguggut t 21

<210> 831
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 831
uuuucaagca cuucgaagct t 21

<210> 832
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 832
ucagaauaaa gauugacaat t 21

<210> 833
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 833
uaugauaugg ucacgucuat t 21

<210> 834
<211> 21
<212> DNA
<213> Artificial Sequence

<220>
<221> source
<223> /note="Description of Artificial Sequence: Synthetic

oligonucleotide"

<220>
<221> source
<223> /note="Description of Combined DNA/RNA Molecule: Synthetic oligonucleotide"

<400> 834
uucaagcacu ucgaagcagt t 21

<210> 835
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 835
gagcuaucac cuauccuugt t 21

<210> 836
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 836
ugacaaauau acaagugcat t 21

<210> 837
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 837
gaguauacau ugugauacgt t 21

<210> 838
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 838
uucguucacu guacaugcgt t 21

<210> 839
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 839
ugguguagac ccuucuaaut t 21

<210> 840
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 840
uguuuuacag caugccaaat t 21

<210> 841
<211> 21
<212> DNA
<213> Artificial Sequence

<220>
<221> source
<223> /note="Description of Artificial Sequence: Synthetic oligonucleotide"

<220>

<221> source
<223> /note="Description of Combined DNA/RNA Molecule:
Synthetic oligonucleotide"

<400> 841
ugaauaagua cuuuaagggt t 21

<210> 842
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 842
ucucgagagu auacauugut t 21

<210> 843
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 843
ccugguauag ccaccgucat t 21

<210> 844
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 844
cagauaauua ggaucucgat t 21

<210> 845
<211> 21
<212> DNA

<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 845
cagaguaaac ugacccucat t 21

<210> 846
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 846
uaaguacuuu aagggagaut t 21

<210> 847
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 847
uuccacauau aagcaauuct t 21

<210> 848
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 848
gcuaucaccu auccuugagt t 21

<210> 849
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 849
aaaagccuug aacuguuuut t 21

<210> 850
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 850
ugcaaauugu ugucucccut t 21

<210> 851
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 851
ugugauacgu caagucacat t 21

<210> 852
<211> 21
<212> DNA
<213> Artificial Sequence

<220>

<221> source
<223> /note="Description of Artificial Sequence: Synthetic oligonucleotide"

<220>
<221> source
<223> /note="Description of Combined DNA/RNA Molecule: Synthetic oligonucleotide"

<400> 852
ugauacguca agucacauut t 21

<210> 853
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 853
accucuccga aaagccuugt t 21

<210> 854
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 854
ucguucacug uacaugcgat t 21

<210> 855
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 855
aagucacauu ccacguuagt t 21

<210> 856
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 856
ucuucaaaag ggauaagugt t 21

<210> 857
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 857
cuuuaauuuu ccuaguccut t 21

<210> 858
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 858
cagugauucu ucguucacut t 21

<210> 859
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 859
ugcaucaucu augauauggt t 21

<210> 860
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 860
gcucugucug ugcccguugt t 21

<210> 861
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 861
cucuucaaaa gggauaagut t 21

<210> 862
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 862
gcugcugcau uauuauccat t 21

<210> 863

<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 863
gauuucgacu guuaaauuct t 21

<210> 864
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 864
uucauacucc aauaaaucut t 21

<210> 865
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 865
agagaacagu gauucuucgt t 21

<210> 866
<211> 21
<212> DNA
<213> Artificial Sequence

<220>
<221> source
<223> /note="Description of Artificial Sequence: Synthetic oligonucleotide"

<220>
<221> source

<223> /note="Description of Combined DNA/RNA Molecule: Synthetic oligonucleotide"

<400> 866
gaguaaacug acccucagut t 21

<210> 867
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 867
cugugcccgu uguaaacugt t 21

<210> 868
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 868
uccacauaua agcaauucat t 21

<210> 869
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 869
gucacgucua acuuugagut t 21

<210> 870
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 870
uugugauacg ucaagucact t 21

<210> 871
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 871
uuucgacugu uaaauucaut t 21

<210> 872
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 872
gguauagcca ccgucauaut t 21

<210> 873
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 873
agaacaguga uucuucguut t 21

<210> 874
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 874
uguagacccu ucuaauggct t 21

<210> 875
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 875
uuugcauuaa ucuuauaaat t 21

<210> 876
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 876
gagucucaaa uauggauuct t 21

<210> 877
<211> 21
<212> DNA
<213> Artificial Sequence

<220>
<221> source

<223> /note="Description of Artificial Sequence: Synthetic oligonucleotide"

<220>
<221> source
<223> /note="Description of Combined DNA/RNA Molecule: Synthetic oligonucleotide"

<400> 877
ucccuucaua cuccaauaat t 21

<210> 878
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 878
guuuaacuuu ccggaagcut t 21

<210> 879
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 879
uacuaugcca aucagaguat t 21

<210> 880
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 880
cucagcacua gaaaaaacut t 21

<210> 881
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 881
gauucaucgu augugaacat t 21

<210> 882
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 882
aaguacuuua agggagauut t 21

<210> 883
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 883
ucacgucuaa cuuugaguct t 21

<210> 884
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 884
agauuucgac uguuaaauut t 21

<210> 885
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 885
uccggaagua aaagcacaut t 21

<210> 886
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 886
auuccacguu aggugacaat t 21

<210> 887
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 887
gugauacguc aagucacaut t 21

<210> 888
<211> 21

<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 888
acagugauuc uucguucact t 21

<210> 889
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 889
ugcuuggaaa ucuagauuct t 21

<210> 890
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 890
agucagaaua aagauugact t 21

<210> 891
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule:

Synthetic oligonucleotide

<400> 891
ucuucauugu gauuugugut t 21

<210> 892
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 892
agggagauuc auuggucact t 21

<210> 893
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 893
guacuuuaag ggagauucat t 21

<210> 894
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 894
accucauuca gugguucaut t 21

<210> 895
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 895
uguuuaacuu uccggaagct t 21

<210> 896
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 896
ucagaguaaa cugacccuct t 21

<210> 897
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 897
ucuucguuca cuguacaugt t 21

<210> 898
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 898

agcacuagaa aaaacucuut t 21

<210> 899
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 899
ccgaaucugg ucugcauugt t 21

<210> 900
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 900
aguaauaagu ugcugaaaut t 21

<210> 901
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 901
acguuaggug acaaauauat t 21

<210> 902
<211> 21
<212> DNA
<213> Artificial Sequence

<220>
<221> source
<223> /note="Description of Artificial Sequence: Synthetic

oligonucleotide"

<220>
<221> source
<223> /note="Description of Combined DNA/RNA Molecule: Synthetic oligonucleotide"

<400> 902
uuguucauuu uuaucucagt t 21

<210> 903
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 903
cugaauaagu acuuuaaggt t 21

<210> 904
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 904
ucaucuauga uauggucact t 21

<210> 905
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 905
aguaaaagca cauuaaagct t 21

<210> 906
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 906
aaaacaauuu uguagauuct t 21

<210> 907
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 907
augccucagc acuagaaaat t 21

<210> 908
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 908
aagggagauu cauuggucat t 21

<210> 909
<211> 21
<212> DNA
<213> Artificial Sequence

<220>
<221> source
<223> /note="Description of Artificial Sequence: Synthetic oligonucleotide"

<220>

<221> source
<223> /note="Description of Combined DNA/RNA Molecule:
Synthetic oligonucleotide"

<400> 909
aaugaaugaa cgauuuccct t 21

<210> 910
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 910
cugcugcauu auuauccaut t 21

<210> 911
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 911
aaucuggugu agacccuuct t 21

<210> 912
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 912
aucucgagag uauacauugt t 21

<210> 913
<211> 21
<212> DNA

<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 913
guaccaaugc cucagcacut t 21

<210> 914
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 914
aggaucucga gaguauacat t 21

<210> 915
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 915
auuaaucuua uaaagcucgt t 21

<210> 916
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 916
gagaguauac auugugauat t 21

<210> 917
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 917
accauuuuca agcacuucgt t 21

<210> 918
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 918
ccaaauucuu ugugacagct t 21

<210> 919
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 919
cgagaguaua cauugugaut t 21

<210> 920
<211> 21
<212> DNA
<213> Artificial Sequence

<220>

<221> source
<223> /note="Description of Artificial Sequence: Synthetic oligonucleotide"

<220>
<221> source
<223> /note="Description of Combined DNA/RNA Molecule: Synthetic oligonucleotide"

<400> 920
uucuucguuc acuguacaut t 21

<210> 921
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 921
ccaccgucau auucuguagt t 21

<210> 922
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 922
uaaguaauaa guugcugaat t 21

<210> 923
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 923
uuaaguaagu cacaucuuut t 21

<210> 924
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 924
uguaccaaug ccucagcact t 21

<210> 925
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 925
cacguuaggu gacaaauaut t 21

<210> 926
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 926
aucuagauuc cggcuuccat t 21

<210> 927
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 927
auggccuuca acaaucucut t 21

<210> 928
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 928
gccaccguca uauucuguat t 21

<210> 929
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 929
uugcaaauug uugucuccct t 21

<210> 930
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 930
uuacgauaau gaacaaauut t 21

<210> 931

<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 931
gaagagucuu uucuguucat t 21

<210> 932
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 932
aacuguuuuu cuacugauut t 21

<210> 933
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 933
guaaacugca agaagaguct t 21

<210> 934
<211> 21
<212> DNA
<213> Artificial Sequence

<220>
<221> source
<223> /note="Description of Artificial Sequence: Synthetic oligonucleotide"

<220>
<221> source

<223> /note="Description of Combined DNA/RNA Molecule: Synthetic oligonucleotide"

<400> 934
cggaaguaaa agcacauuat t 21

<210> 935
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 935
aaguaauaag uugcugaaat t 21

<210> 936
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 936
uuugcaaauu guugucucct t 21

<210> 937
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 937
acuuugaguc ucaaauaugt t 21

<210> 938
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 938
acaagugcau caucuaugat t 21

<210> 939
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 939
gacaagugca ucaucuaugt t 21

<210> 940
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 940
ccgucauauu cuguaguuut t 21

<210> 941
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 941
aaccucauuc agugguucat t 21

<210> 942
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 942
ucaucauuca cuagauuuct t 21

<210> 943
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 943
cuuugcagcu gcucgcuuct t 21

<210> 944
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 944
uugugacagc auucaauaut t 21

<210> 945
<211> 21
<212> DNA
<213> Artificial Sequence

<220>
<221> source

<223> /note="Description of Artificial Sequence: Synthetic oligonucleotide"

<220>
<221> source
<223> /note="Description of Combined DNA/RNA Molecule: Synthetic oligonucleotide"

<400> 945
ucaaaagaag auggauuaat t 21

<210> 946
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 946
aagaagaugg auuaaaccat t 21

<210> 947
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 947
caucauucac uagauuucgt t 21

<210> 948
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 948
uuugugacag cauucaauat t 21

<210> 949
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 949
gaagauggau uaaaccaaat t 21

<210> 950
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 950
cagcauucaa uauaaagggt t 21

<210> 951
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 951
aagauggauu aaaccaaaat t 21

<210> 952
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule:
Synthetic oligonucleotide"

<400> 952
cugcucgcuu cauucggcut t 21

<210> 953
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 953
uauguaacac uuucacgcat t 21

<210> 954
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 954
aagugaaaga auauguaact t 21

<210> 955
<211> 21
<212> DNA
<213> Artificial Sequence

<220>
<221> source
<223> /note="Description of Artificial Sequence: Synthetic
oligonucleotide"

<220>
<221> source
<223> /note="Description of Combined DNA/RNA Molecule:
Synthetic oligonucleotide"

<400> 955
gcucaaccuc aagcaguaat t 21

<210> 956
<211> 21

<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 956
cuucauucgg cuagcuucat t 21

<210> 957
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 957
agcaaaagug auuuaaccct t 21

<210> 958
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 958
gcaaaaguga uuuaacccut t 21

<210> 959
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule:

Synthetic oligonucleotide

<400> 959
uuuguugugc uguuacuaat t 21

<210> 960
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 960
ucaaccucaa gcaguaauat t 21

<210> 961
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 961
aggauuggcu acugaaacat t 21

<210> 962
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 962
cauucggcua gcuucaaugt t 21

<210> 963
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 963
uucggcuagc uucaauguct t 21

<210> 964
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 964
auucggcuag cuucaaugut t 21

<210> 965
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 965
ugcagcugcu cgcuucauut t 21

<210> 966
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 966

ugaaagaaua uguaacacut t 21

<210> 967
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 967
aaaaggcuca accucaagct t 21

<210> 968
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 968
aaacaguuca uugcaagact t 21

<210> 969
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 969
gcugcucgcu ucauucggct t 21

<210> 970
<211> 21
<212> DNA
<213> Artificial Sequence

<220>
<221> source
<223> /note="Description of Artificial Sequence: Synthetic

oligonucleotide"

<220>
<221> source
<223> /note="Description of Combined DNA/RNA Molecule: Synthetic oligonucleotide"

<400> 970
auguaacacu uucacgcaat t 21

<210> 971
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 971
gaaacaguuc auugcaagat t 21

<210> 972
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 972
cccuguccuu ucauauacut t 21

<210> 973
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 973
uucauucggc uagcuucaat t 21

<210> 974
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 974
gugcuguuac uaaaguucut t 21

<210> 975
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 975
aaagugauuu aacccuuaat t 21

<210> 976
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 976
aaccucucug uacauacaat t 21

<210> 977
<211> 21
<212> DNA
<213> Artificial Sequence

<220>
<221> source
<223> /note="Description of Artificial Sequence: Synthetic oligonucleotide"

<220>

<221> source
<223> /note="Description of Combined DNA/RNA Molecule: Synthetic oligonucleotide"

<400> 977
aacacaagca aaagugauut t 21

<210> 978
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 978
acaagugaaa gaauauguat t 21

<210> 979
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 979
auucagaaaa accucucugt t 21

<210> 980
<211> 21
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 980
aguaauaaac acaagcaaat t 21

<210> 981
<211> 21
<212> DNA

```
<213> Artificial Sequence

<220>
<221> source
<223> /note="Description of Artificial Sequence: Synthetic
      oligonucleotide"

<220>
<221> source
<223> /note="Description of Combined DNA/RNA Molecule:
      Synthetic oligonucleotide"

<400> 981
uacugaaaca guucauugct t                                              21
```